# EUROPEAN PATENT SPECIFICATION

(11) **EP 2 710 383 B1**
(45) Date of publication and mention of the grant of the patent: **11.01.2017**
(21) Application number: 12735358.9
(22) Date of filing: 16.05.2012
(51) Int. Cl.: G01N 33/68, C12Q 1/68

(54) **Performance of a biomarker panel for irritable bowel syndrome**
Leistungsfähigkeit eines Biomarkerpanels für Reizdarmsyndrom
Efficacité d'un panel de biomarqueurs du syndrome du côlon irritable

(30) Priority: 16.05.2011 US 201161486734 P; 02.12.2011 US 201161566521 P
(43) Date of publication of application: 26.03.2014
(73) Proprietor: The University of Newcastle, Callaghan, New South Wales 2308 (AU)
(72) Inventor: JONES, Michael, P., North Ryde, NSW 2109 (AU); TALLEY, Nicholas, J., Callaghan, NSW 2308 (AU)
(74) Representative: Potter Clarkson LLP
(86) International application number: PCT/US2012/038197
(87) International publication number: WO 2012/158831

(56) References cited:
- WO-A1-2010/151699
- WO-A2-2011/066458
- A. J. LEMBO ET AL: "Use of serum biomarkers in a diagnostic test for irritable bowel syndrome", ALIMENTARY PHARMACOLOGY & THERAPEUTICS, vol. 29, no. 8, 1 April 2009 (2009-04-01), pages 834-842, XP55038614, ISSN: 0269-2813, DOI: 10.1111/j.1365-2036.2009.03975.x
- Michael Jones: "PERFORMANCE OF A BIOMARKER PANEL FOR THE IRRITABLE BOWEL SYNDROME", , 25 October 2012 (2012-10-25), XP002683815, Retrieved from the Internet: URL:http://uegw.congress-online.com/uegw20 11/guest/AbstractView?ABSID=12846 [retrieved on 2012-09-20]

## Description

### BACKGROUND OF THE INVENTION

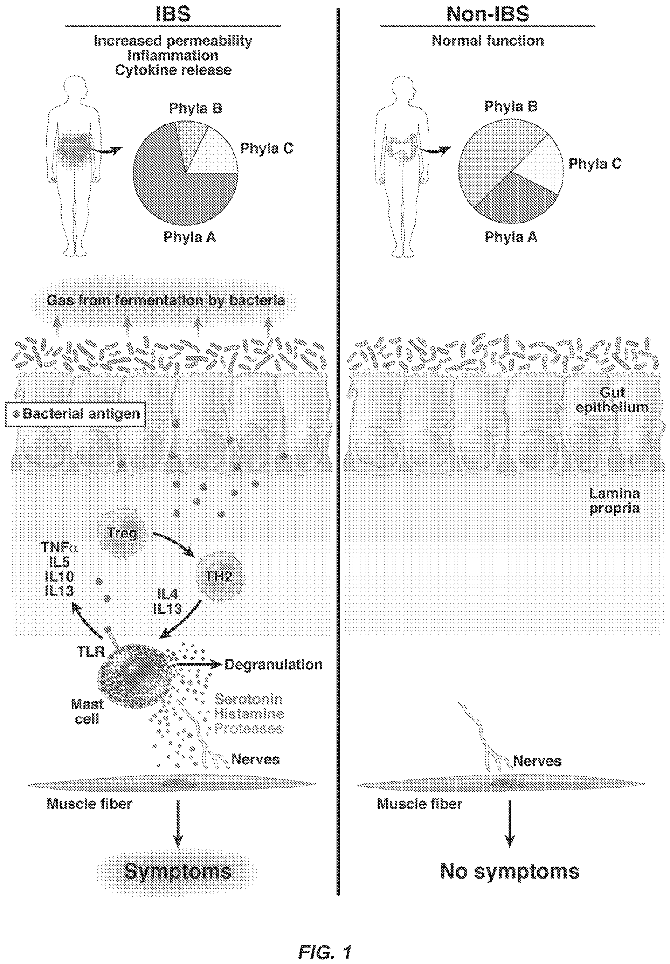

Irritable bowel syndrome (IBS) is a common gastrointestinal disorder characterized by chronic abdominal pain, discomfort, bloating/distension and alteration of bowel habits in the absence of any detectable cause. The pathophysiology of IBS remains unclear, yet studies have shown that numerous factors including alterations in gastrointestinal motility, visceral hypersensitivy, inflammation, cytokine release, alteration in fecal flora, and bacterial overgrowth may play a role (*see*, Figure 1). IBS was originally considered a diagnosis of exclusion, and its diagnosis remains challenging. Lembo et al., Aliment. Pharmacol. Ther., 15: 834-842 (2009) describes a 10 serum biomarker algorithm for differentiating IBS from non-IBS using the Rome I or Rome II criteria of IBS. Unfortunately, the diagnostic method of Lembo *et al.* does not discriminate between IBS subtypes from each other. The present invention provides improved methods of reliable and accurate diagnosis of IBS and/or IBS subtypes. WO2010151699 relates to methods for diagnosing IBS and provides an ELISA assay for the determination of serum mast cell ss-tryptase levels using rabbit anti-tryptase as the capture antibody and alkaline phosphatase conjugated G3 as the detecting antibody. WO2011066458 relates to genomic markers for IBS diagnosis and provides novel algorithms for the diagnosis and prognosis of IBS. Micheal Jones: "performance of a biomarker panel for the IBS", 25 October 2012, XP002686815 describes the use of five markers (histamine, NGAL, znf326, rnf26 and ttg) in discriminating IBS subjects from healthy volunters.

### BRIEF SUMMARY OF THE INVENTION

The present invention is defined by the appended claims. Any subject-matter identified in the application as "invention", "embodiment", "aspect", etc., which exceeds the scope of the invention as represented by the claims does not form part the claimed invention but only serves as background information to better understand the invention.

In certain aspects, the present invention provides an evaluation of an extensive panel of gene expression and serology markers for diagnosing irritable bowel syndrome (IBS) and IBS subtypes. In particular embodiments, the present invention provides a diagnostic model for aiding in the differentiation of IBS subjects from healthy subjects, and for aiding in the discrimination of IBS subtypes from each other (*e*.*g*., differentiating IBS-C from IBS-D).

As such, the present invention is based, in part, upon the surprising discovery that unique combinations of serological and/or genetic markers are advantageous in aiding or assisting in diagnosing IBS (*e*.*g*., compared with healthy subjects) and in discriminating IBS subtypes from each other *(e.g.,* IBS-C from IBS-D). In some instances, the present invention also includes combining serological and/or genetic marker analysis with psychological measures in aiding or assisting in diagnosing IBS and in discriminating IBS subtypes from each other. In one aspect the present invention relates to a method for aiding in the differentiation of IBS-constipation (IBS-C) from IBS-diarrhea (IBS-D) in a subject, the method comprising:
(a) contacting a first sample from the subject with a binding moiety under conditions suitable to transform an IBS serological marker present in the first sample into a complex comprising the IBS serological marker and the binding moiety,
   wherein the IBS serological marker comprises a combination of histamine and neutrophil gelatinase-associated lipocalin (NGAL);
(b) contacting isolated and/or amplified RNA obtained from a second sample from the subject with a detection reagent under conditions suitable to transform an IBS genetic marker present in the second sample into a complex comprising the IBS genetic marker and the detection reagent,
   wherein the IBS genetic marker comprises a combination of MICALL1 and RNF26;
(c) determining the level of the complex in step (a), thereby determining the level of the IBS serological marker present in the first sample; and
(d) determining the level of the complex in step (b), thereby determining the level of the IBS genetic marker present in the second sample.

In another aspect, the present invention provides above method wherein the IBS serological marker further comprises PGE2, tryptase, serotonin, substance P, IL-12, IL-10, IL-6, IL-8, TNF-α, IL-1β, GRO-α, BDNF, ASCA IgA, anti-CBir1 antibody, tTG, TWEAK, ANCA, TIMP-1, or combinations thereof, wherein the level of at least 2, 3, 4, 5, 6, 7, 8, 9, 10, 11, 12, 13, 14, 15, 16, 17, or 18 of the IBS serological markers are determined (see below).

| **Serology Markers** | **Gene Expression Markers** |
|---|---|
| Interleukin-1β (IL-1β) | CBFA2T2 |
| Growth-related oncogene-α (GRO-α) | CCDC147 |
| Brain-derived neurotrophic factor (BDNF) | HSD17B11 |
| Anti*-Saccharomyces cerevisiae* antibody (ASCA IgA) | LDLR |
| Antibody against CBir1 (Anti-CBir1) | MAP6D1 |
| Tumor necrosis factor (TNF)-like weak inducer of apoptosis (TWEAK) | RAB7L1 |
| Tissue inhibitor of metalloproteinase-1 (TIMP-1) | RRP7A |
| Neutrophil gelatinase-associated lipocalin (NGAL) | SUSD4 |
| Histamine | SH3BGRL3 |
| PGE2 | VIPR1 |
| Tryptase | WEE1 |
| Serotonin | ZNF326 |
| Substance P | |
| IL-12 | |
| IL-10 | |
| IL-6 | |
| IL-8 | |
| TNF-α | |

In particular embodiments, the IBS serological marker comprises a combination of histamine, NGAL, PGE2, tryptase, serotonin, substance P, IL-12, IL-10, IL-6, IL-8, TNF-α, IL-1β, GRO-α, BDNF, ASCA IgA, anti-CBir1 antibody, tTG, TWEAK, ANCA, and TIMP-1; and the IBS genetic marker comprises a combination of RNF26, CBFA2T2, CCDC147, HSD17B11, LDLR, MAP6D1, RAB7L1, RRP7A, SUSD4, SH3BGRL3, VIPR1, WEE1, MICALL1, and ZNF326.

In certain embodiments, the present invention provides a method for aiding in the differentiation of IBS-constipation (IBS-C) from IBS-diarrhea (IBS-D) in a subject, wherein the method comprises detecting, determining, measuring, or analyzing at least 1, 2, 3, or all 4 of the following markers: histamine, neutrophil gelatinase-associated lipocalin (NGAL), MICALL1, and RNF26.

Other objects, features, and advantages of the present invention will be apparent to one of skill in the art from the following detailed description and figures.

### BRIEF DESCRIPTION OF THE DRAWINGS

Figure 1 shows a diagram of the pathophysiology of irritable bowel syndrome.
Figure 2 shows the process for the identification of 24 additional markers described in Example 1.
Figure 3 shows the gene array analysis described in Example 1. Figures 3a and 3b show the clustering results. Three groups were completely separated by the gene expression profiles of the DEGs, which are indicated by the panel on the top of the heatmap (Figure 3a). The separation among samples was further visualized based on the gene expression profiles of all unmasked probe sets using a multidimensional scaling plot. (Figure 3b).
Figure 4 shows an ROC curve based on the full panel of markers for IBS v. Health. The full panel of biomarkers provides adequate overall differentiation of IBS cases from healthy volunteers (AUC = 0.81).
Figure 5 shows an ROC curve based on the full panel of markers with psychological measures for IBS v. Health. The full panel of biomarkers in combination with psychological measures provides strong overall differentiation of IBS cases from healthy volunteers (AUC = 0.93).
Figure 6 shows an ROC curve based on the full panel of markers with psychological measures for IBS-C v. IBS-D. The full panel of biomarkers in combination with psychological measures provides strong overall differentiation of IBS-C from IBS-D (AUC = 0.94).
Figure 7 shows an ROC curve based on the full panel of markers with psychological measures for IBS-C v. IBS-M. The full panel of biomarkers in combination with psychological measures provides strong overall differentiation of IBS-C from IBS-M (AUC = 0.88).
Figure 8 shows an ROC curve based on the full panel of markers with psychological measures for IBS-D v. IBS-M. The full panel of biomarkers in combination with psychological measures provides strong overall differentiation of IBS-D from IBS-M (AUC = 0.91).

### DETAILED DESCRIPTION OF THE INVENTION

### I. Introduction

The present invention is based, in part, upon the surprising discovery that unique combinations of serological and/or genetic markers are advantageous in aiding or assisting in diagnosing IBS (*e*.*g*., compared with healthy subjects) and in discriminating IBS subtypes from each other *(e.g.,* IBS-C from IBS-D, IBS-D from IBS-M, and/or IBS-D from IBS-M). In some instances, the present invention also includes combining serological and/or genetic marker analysis with psychological measures in aiding or assisting in diagnosing IBS and in discriminating IBS subtypes from each other.

In certain embodiments, as described in Example 1, a panel of 34 biomarkers can provide clinically useful and relevant discrimination of IBS from healthy subjects. Statistical performance analysis showed that sensitivity is higher than specificity. Interestingly, the full panel displayed even better performance in discriminating IBS subtypes from each other. In all diagnostic classifications considered (*e*.*g*., IBS, IBS-C, IBS-D, IBS-M, and healthy), a subset of the 34 biomarkers can provide useful discrimination of IBS and IBS subtype with relatively minimal loss in diagnostic performance, compared to the full panel. In particular embodiments, the panel of 34 biomarkers or subsets thereof is combined with psychological markers to further improve the differentiation of IBS from healthy subjects and/or to further improve the discrimination of IBS subtypes from each other.

### II. Definitions

As used herein, the following terms have the meanings ascribed to them unless specified otherwise.

The term "irritable bowel syndrome" or "IBS" includes a group of functional bowel disorders characterized by one or more symptoms including, but not limited to, abdominal pain, abdominal discomfort, change in bowel pattern, loose or more frequent bowel movements, diarrhea, and constipation, typically in the absence of any apparent structural abnormality. There are at least three forms of IBS, depending on which symptom predominates: (1) diarrhea-predominant (IBS-D); (2) constipation-predominant (IBS-C); and (3) IBS with alternating stool pattern (IBS-A). IBS can also occur in the form of a mixture of symptoms (IBS-M). There are also various clinical subtypes of IBS, such as post-infectious IBS (IBS-PI).

The terms "transforming a sample" and "transforming a marker" include a physical and/or chemical change of the sample to extract a marker or to change or modify a marker as defined and described herein. In particular embodiments, an extraction, a manipulation, a chemical precipitation, an ELISA, a complexation, an immuno-extraction, a physical or chemical modification of the sample or marker to measure a level or concentration of a marker all constitute a transformation. As long as the sample or marker is not identical before and after the transformation step, the change or modification is a transformation.

The term "sample" includes any biological specimen obtained from an individual. Suitable samples for use in the present invention include, without limitation, whole blood, plasma, serum, saliva, urine, stool *(i.e.,* feces), sputum, tears, and any other bodily fluid, or a tissue sample (*i*.*e*., biopsy) such as a small intestine or colon sample, and cellular extracts thereof (*e*.*g*., red blood cellular extract). In a preferred embodiment, the sample is a blood, plasma, or serum sample. In a more preferred embodiment, the sample is a serum sample. The use of samples such as serum, saliva, and urine is well known in the art (*see, e.g.,* Hashida et al., J. Clin. Lab. Anal., 11:267-86 (1997)). One skilled in the art will appreciate that samples such as whole blood or serum samples can be diluted prior to the analysis of marker levels. One skilled in the art will also appreciate that different aliquots of the same sample *(e.g.,* a whole blood or serum sample) can be used to detect, determine, measure, or analyze different markers (*e*.*g*., one aliquot can be used to measure IBS serological markers while another aliquot can be used to measure IBS genetic markers).

The term "biomarker" or "marker" includes any diagnostic marker such as a biochemical marker, serological marker, genetic marker, or other clinical or echo graphic characteristic that can be used to aid or assist in diagnosing IBS (*e*.*g*., compared with healthy subjects), to aid or assist in discriminating IBS subtypes from each other *(e.g.,* IBS-C from IBS-D, IBS-D from IBS-M, and/or IBS-D from IBS-M), to classify a sample from a subject as an IBS sample, and/or to classify IBS into one of its various forms or clinical subtypes.

The term "classifying" includes "to associate" or "to categorize" a sample with a disease state. In certain instances, "classifying" is based on statistical evidence, empirical evidence, or both. In certain embodiments, the methods and systems of classifying use a so-called training set of samples having known disease states. Once established, the training data set serves as a basis, model, or template against which the features of an unknown sample are compared, in order to classify the unknown disease state of the sample. In certain instances, classifying a sample is akin to diagnosing the disease state of the sample. In other instances, classifying a sample is akin to differentiating the disease state of the sample from another disease state or differentiating forms or subtypes of a disease state from each other.

As used herein, the term "binding moiety" includes any class of molecules capable of specifically recognizing a marker of interest. Non-limiting examples of binding moieties include proteins, such as monoclonal or polyclonal antibodies (*e*.*g*., chimeric, humanized, or human antibodies) and functional fragments thereof (*e*.*g*., minibodies, diabodies, triabodies, single chain Fv, F(ab)', and the like), antigens such as proteins that specifically bind to an antibody or autoantibody and immunoreactive fragments thereof, combinatorially-derived proteins from phage display or ribosome display, peptides, nucleic acids (*e*.*g*., aptamers), other molecules that are capable of specifically recognizing a biomarker of interest, and combinations thereof.

As used herein, the term "detection reagent" includes a nucleic acid molecule such as an oligonucleotide or a polynucleotide that specifically hybridizes to an IBS marker of the invention (*e*.*g*., an IBS genetic marker such as an mRNA or expressed non-coding RNA). In particular embodiments, the detection reagent is an oligonucleotide comprising at least about 10, 11, 12, 13, 14, 15, 16, 17, 18, 19, 20, 21, 22, 23, 24, 25, 26, 27, 28, 29, or 30, or between about 10-30, about 15-30, or about 15-25 nucleotides in length. In particular embodiments, the detection reagent is an oligonucleotide such as a detector probe comprising a reporter moiety *(e.g.,* FAM™, TET™, JOE™, VIC™, or SYBR^{®} Green), a quencher moiety *(e.g.,* Black Hole Quencher™ or TAMRA™), an MGB moiety, and/or a passive reference (*e*.*g*., ROX™). In other embodiments, the detection reagent (*e*.*g*., an oligonucleotide such as a detector probe) can optionally comprise reporter moieties or labels such as radioisotopes, fluorescent compounds, chemiluminescent compounds, enzymes, and enzyme co-factors. In certain embodiments, the detection reagent is a nucleic acid molecule and determining the level of a complex of interest *(e.g.,* a complex between an IBS genetic marker such as an mRNA or expressed non-coding RNA and the detection reagent) can comprise nucleic acid *(e.g.,* oligonucleotide) hybridization *(e.g.,* microarray or bead-based hybridization assays, xMAP assays, northern blot, dot blot, RNase protection assays, *etc.)* and/or nucleic acid amplification *(e.g.,* PCR, qPCR, RT-PCR, qRT-PCR, mass spectrometry, *etc.).* The term "detection reagent" also includes an antibody or antigen-binding fragment thereof optionally comprising a label or reporter moiety and determining the level of a complex of interest in a sample can comprise an immunochemical assay (*e*.*g*., ELISA, immunofluorescence assay, IFA, and the like).

The term "specifically hybridizes" includes the ability of a detection reagent such as an oligonucleotide or a polynucleotide to hybridize to at least a portion of, for example, at least about 6, 10, 12, 15, 20, 25, 30, 40, 50, 75, 100, 150, 200, 300, 350, 400, 500, 750, or 1000 contiguous nucleotides of an IBS marker described herein *(e.g.,* an IBS genetic marker such as an mRNA or expressed non-coding RNA), or a sequence complementary thereto, or naturally occurring mutants thereof, such that it has less than about 20%, 15%, 10%, or 5% background hybridization to a cellular nucleic acid (*e*.*g*., mRNA or genomic DNA) encoding a different protein. In particular embodiments, a detection reagent such as an oligonucleotide probe detects only a specific nucleic acid, *e.g.,* it does not substantially hybridize to similar or related nucleic acids, or complements thereof.

The term "individual," "subject," or "patient" typically refers to humans, but also to other animals including, *e*.*g*., other primates, rodents, canines, felines, equines, bovines, porcines, and the like.

The term "therapeutically effective amount or dose" includes a dose of a drug that is capable of achieving a therapeutic effect in a subject in need thereof. As a non-limiting example, a therapeutically effective amount of a drug useful for treating IBS or an IBS subtype can be the amount that is capable of preventing or relieving one or more symptoms associated with IBS or an IBS subtype. The exact amount can be ascertainable by one skilled in the art using known techniques *(see, e.g.,* Lieberman, Pharmaceutical Dosage Forms, Vols. 1-3 (1992); Lloyd, The Art, Science and Technology of Pharmaceutical Compounding (1999); Pickar, Dosage Calculations (1999); and Remington: The Science and Practice of Pharmacy, 20th Edition, Gennaro, Ed., Lippincott, Williams & Wilkins (2003)).

The terms "Rome I", "Rome II", and "Rome III" include a series of diagnostic criteria developed to classify functional gastrointestinal disorders (FGIDs) based on clinical symptoms. Functional gastrointestinal disorders are a group of disorders of the digestive system in which symptoms can not be explained by the presence of structural or tissue abnormality. Non-limiting examples of FGIDs include irritable bowel syndrome, functional pepsia, functional constipation, and functional heartburn. Detailed descriptions of Rome I, Rome II, and Rome III diagnostic criteria can be found in, *e.g.,* Dossman, Gastroenterology, 130:1377-1390 (2006), Drossman and Dumitrascu, J. Gastrointestin. Liver Dis., 15:237-241 (2006), and Thompson et al., "Functional Bowel Disorders." Rome II: The Functional Gastrointestinal Disorders. Diagnosis, Pathophysiology and Treatment. A Multinational Consensus. Lawrence, JS: Allen Press, 2000.

### III. Description of the Embodiments

In certain aspects, the present invention provides an evaluation of an extensive panel of gene expression and serology markers for diagnosing irritable bowel syndrome (IBS) and IBS subtypes. In particular embodiments, the present invention provides a diagnostic model for aiding in the differentiation of IBS subjects from healthy subjects, and for aiding in the discrimination of IBS subtypes from each other (*e*.*g*., differentiating IBS-C from IBS-D).

In some aspects, the present invention provides a method for measuring, detecting, analyzing, or determining the presence, (concentration) level, and/or gene expression level of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or all 34 of the following serological and/or genetic markers in a sample, *e.g.,* to aid or assist in diagnosing IBS *(e.g.,* compared with healthy subjects) and/or to aid or assist in discriminating between various subtypes of IBS *(e.g.,* IBS-C from IBS-D): (1) serological markers including interleukin-1β (IL-1β), growth-related oncogene-α (GRO-α), brain-derived neurotrophic factor (BDNF), anti-*Saccharomyces cerevisiae* antibody (ASCA IgA), antibody against CBir1 (anti-CBir1), anti-human tissue transglutaminase (tTG), tumor necrosis factor (TNF)-like weak inducer of apoptosis (TWEAK), anti-neutrophil cytoplasmic antibody (ANCA), tissue inhibitor of metalloproteinase-1 (TIMP-1), neutrophil gelatinase-associated lipocalin (NGAL), histamine, prostaglandin E2 (PGE2), tryptase, serotonin, substance P, IL-12, IL-10, IL-6, IL-8, and/or TNF-α; and/or (2) genetic markers including CBFA2T2, CCDC147, HSD17B11, LDLR, MAP6D1, MICALL1, RAB7L1, RNF26, RRP7A, SUSD4, SH3BGRL3, VIPR1, WEE1, and/or ZNF326. In some instances, the methods of the present invention can further comprise additional IBS biomarkers known to one skilled in the art and/or described herein.

In some embodiments, a panel for measuring one or more of the markers described herein can be constructed and used in the methods of the present invention, *e*.*g*., for aiding or assisting in diagnosing IBS or discriminating IBS subtypes from each other. One skilled in the art will appreciate that the presence or level of a plurality of markers can be determined simultaneously or sequentially, using, for example, an aliquot and/or dilution of a subject's sample. In certain instances, the level of a particular marker in a sample is considered to be elevated when it is at least about 10%, 15%, 20%, 25%, 30%, 35%, 40%, 45%, 50%, 75%, 100%, 125%, 150%, 175%, 200%, 250%, 300%, 350%, 400%, 450%, 500%, 600%, 700%, 800%, 900%, or 1000% greater than the level of the same marker in a comparative sample *(e.g.,* a normal (healthy), GI control, IBD, and/or Celiac disease sample) or population of samples (*e*.*g*., greater than a median level of the same marker in a comparative population of normal (healthy), GI control, IBD, and/or Celiac disease samples). In other instances, the level of a particular marker in a sample is considered to be lowered when it is at least about 5%, 10%, 15%, 20%, 25%, 30%, 35%, 40%, 45%, 50%, 55%, 60%, 65%, 70%, 75%, 80%, 85%, 90%, or 95% less than the level of the same marker in a comparative sample *(e.g.,* a normal (healthy), GI control, IBD, and/or Celiac disease sample) or population of samples *(e.g.,* less than a median level of the same marker in a comparative population of normal (healthy), GI control, IBD, and/or Celiac disease samples). In further instances, the level of a particular marker in a sample is considered to be differentially expressed when its magnitude *(e.g.,* log2 fold change) is at least about 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0, 6.0, 7.0, 8.0, 9.0, 10.0, or greater (*e*.*g*., positive or negative value), with respect to the same marker in a comparative population of normal (healthy), GI control, IBD, and/or Celiac disease samples. In a preferred embodiment, the magnitude of a differentially expressed IBS biomarker is at least about 1.0, 1.5, 2.0, or 2.5.

In some aspects, the present invention provides a method for aiding in the diagnosis of IBS in a subject, comprising detecting, determining, measuring, or analyzing at least l, 2, 3, or all 4 of the following markers: histamine, tTG, ZNF326, and RNF26. The levels of these markers can be determined in accordance with the techniques described herein. For example, the level of an IBS serological marker can be determined by contacting a first sample from the subject with a binding moiety under conditions suitable to transform the IBS serological marker present in the first sample into a complex comprising the IBS serological marker and the binding moiety. For example, the level of an IBS genetic marker can be determined by contacting isolated and/or amplified RNA obtained from a second sample from the subject with a detection reagent under conditions suitable to transform the IBS genetic marker present in the second sample into a complex comprising the IBS genetic marker and the detection reagent. The level of each IBS serological and/or genetic marker of interest can then be determined by determining the level of the complex.

In other aspects, the present invention provides a method for aiding in the diagnosis of IBS in a subject, comprising detecting, determining, measuring, or analyzing at least 1, 2, 3, 4, 5, or all 6 of the following markers: histamine, NGAL, ZNF326, substance P, RNF26, and tTG. The levels of these markers can be determined in accordance with the techniques described herein. For example, the level of an IBS serological marker can be determined by contacting a first sample from the subject with a binding moiety under conditions suitable to transform the IBS serological marker present in the first sample into a complex comprising the IBS serological marker and the binding moiety. For example, the level of an IBS genetic marker can be determined by contacting isolated and/or amplified RNA obtained from a second sample from the subject with a detection reagent under conditions suitable to transform the IBS genetic marker present in the second sample into a complex comprising the IBS genetic marker and the detection reagent. The level of each IBS serological and/or genetic marker of interest can then be determined by determining the level of the complex.

In certain embodiments, the present invention provides a method for aiding in the diagnosis of IBS in a subject, the method comprising:
(a) contacting a first sample from the subject with a binding moiety under conditions suitable to transform an IBS serological marker present in the first sample into a complex comprising the IBS serological marker and the binding moiety,
   wherein the IBS serological marker is selected from the group consisting of histamine, anti-human tissue transglutaminase (tTG) IgA, and combinations thereof;
(b) contacting isolated and/or amplified RNA obtained from a second sample from the subject with a detection reagent under conditions suitable to transform an IBS genetic marker present in the second sample into a complex comprising the IBS genetic marker and the detection reagent,
   wherein the IBS genetic marker is selected from the group consisting of ZNF326, RNF26, and combinations thereof;
(c) determining the level of the complex in step (a), thereby determining the level of the IBS serological marker present in the first sample; and
(d) determining the level of the complex in step (b), thereby determining the level of the IBS genetic marker present in the second sample.

In particular embodiments, the IBS serological marker comprises a combination of histamine and tTG and/or the IBS genetic marker comprises a combination of ZNF326 and RNF26. In certain embodiments, the methods of the present invention for discriminating or aiding in the differentiation of subjects with IBS from healthy subjects (*e*.*g*., subjects who are Rome III-negative for IBS) may comprise detecting, determining, measuring, or analyzing the (concentration) level of histamine and tTG in a first sample and the gene expression level of ZNF326 and RNF26 in a second sample.

In some embodiments, the method for aiding or assisting in the diagnosis of IBS further comprises determining the level of at least 1, 2, 3, 4, 5, 6, 7, 8, 9, 10, 11, 12, 13, 14, 15, 16, 17, or all 18 of the following IBS serological markers: PGE2, tryptase, serotonin, substance P, IL-12, IL-10, IL-6, IL-8, TNF-α, IL-1β, GRO-α, BDNF, ASCA IgA, anti-CBir1 antibody, TWEAK, ANCA, TIMP-1, NGAL, or combinations thereof.

In other embodiments, the method for aiding or assisting in the diagnosis of IBS further comprises determining the level of at least 1, 2, 3, 4, 5, 6, 7, 8, 9, 10, 11, or all 12 of the following IBS genetic markers: CBFA2T2, CCDC147, HSD17B11, LDLR, MAP6D1, MICALL1, RAB7L1, RRP7A, SUSD4, SH3BGRL3, VIPR1, WEE1, or combinations thereof.

In some embodiments, the IBS serological marker comprises a combination of histamine, NGAL, PGE2, tryptase, serotonin, substance P, IL-12, IL-10, IL-6, IL-8, TNF-α, IL-1β, GRO-α, BDNF, ASCA IgA, anti-CBir1 antibody, tTG, TWEAK, ANCA, and TIMP-1. In other embodiments, the IBS genetic marker comprises a combination of CBFA2T2, CCDC147, HSD17B11, LDLR, MAP6D1, RAB7L1, RNF26, RRP7A, SUSD4, SH3BGRL3, VIPR1, WEE1, MICALL1, and ZNF326. In particular embodiments, the method for aiding or assisting in the diagnosis of IBS further comprises determining the level of all of these IBS serological and genetic markers.

In certain embodiments, the method for aiding or assisting in the diagnosis of IBS further comprises comparing the determined level of the IBS serological or genetic marker present in a sample to a control level, wherein a similarity or a difference in the determined level of the IBS marker relative to the control level is predictive or indicative of an increased or higher likelihood of the subject either having IBS or not having IBS.

In particular embodiments, the level of the IBS serological marker and/or the level of the IBS genetic marker is compared to a control level of the same marker from a healthy subject. A "healthy subject" in the context of the present invention includes a subject who is Rome III-negative for IBS, does not have chronic gastrointestinal symptoms, does not have any active infections, and/or does not have significant chronic medical conditions. In some instances, an increased or higher level of the IBS serological or genetic marker present in the sample relative to the control level is predictive or indicative of an increased or higher likelihood of the subject having IBS. In other instances, the same, a similar, or a reduced level of the IBS serological or genetic marker present in the sample relative to the control level is predictive or indicative of an increased or higher likelihood of the subject not having IBS.

In other embodiments, the level of the IBS serological marker and/or the level of the IBS genetic marker is compared to a control level of the same marker from a subject having IBS. In some instances, the same, a similar, or an increased level of the IBS serological or genetic marker present in the sample relative to the control level is predictive or indicative of an increased or higher likelihood of the subject having IBS. In other instances, a reduced level of the IBS serological or genetic marker present in the sample relative to the control level is predictive or indicative of an increased or higher likelihood of the subject not having IBS.

In further embodiments, the method for aiding or assisting in the diagnosis of IBS further comprises comparing the determined level of the IBS serological or genetic marker present in a sample to a cutoff value or reference value or threshold value, wherein the level of the IBS serological or genetic marker above or below that value is predictive or indicative of an increased or higher likelihood of the subject either having IBS or not having IBS. One skilled in the art will understand that the cutoff value or reference value or threshold value is in units such as mg/ml, µg/ml, ng/ml, pg/ml, fg/ml, EU/ml, or U/ml depending on the marker of interest that is being measured.

In some embodiments, the method further comprises determining a psychological measure of the subject. The psychological measures of the invention can include, but are not limited to, a Patient Health Questionnaire 15 (PHQ-15), a PHQ-15 wherein gastrointestinal symptoms have been excluded from consideration (PHQ-non GI), a perceived stress scale (PSS), a Hospital Anxiety and/or Depression scale (HADs) *(e.g.,* an anxiety score on the HADs and/or depression score on the HADs), an IBS-Severity Scoring System (IBS-SSS), a Functional Bowel Disease Severity Index (FBDSI), a self-report of overall IBS severity (*e*.*g*., bowel symptom questionnaires such as the Rome III 10-question IBS Module, the Bristol Stool Form Scale, and/or the Rome III 93-question GI questionnaire), a self-rated pain severity, and combinations thereof.

In certain embodiments, the method for aiding or assisting in the diagnosis of IBS comprises determining the level of the IBS serological markers tTG and TNF-α, the level of the IBS genetic markers VIPR1, ZNF326, HSD17B11, and WEE1, and the psychological measures PHQ-non GI and PSS.

In other embodiments, the method further comprises applying an algorithm or a combination thereof to the determined level(s) of the IBS serological marker(s) and/or IBS genetic marker(s) and/or to the psychological measure(s) determined for the subject. In certain instances, the algorithm is a statistical algorithm such as, for example, regression analysis (*e*.*g*., logistic regression, linear regression) and/or a learning statistical classifier system. The learning statistical classifier system can be selected from the group consisting of a random forest (RF), classification and regression tree (C&RT), boosted tree, neural network (NN), support vector machine (SVM), general chi-squared automatic interaction detector model, interactive tree, multiadaptive regression spline, machine learning classifier, and combinations thereof. In some instances, the learning statistical classifier system is a tree-based statistical algorithm *(e.g.,* RF, C&RT, *etc.)* and/or a NN *(e.g.,* artificial NN, *etc.).*

In certain embodiments, the determined levels of one or more *(e.g.,* a plurality or an array or a panel of) IBS serological and/or genetic markers can be used to generate an index comprising a representation of the concentration levels of each of the markers, and the index that is generated can be compared to that of a control *(e.g.,* an index generated from the levels of the same markers in a sample from a healthy subject), to aid or assist in the differentiation of a subject with IBS from healthy subjects. In certain instances, one or more algorithms can be applied to the determined levels of the one or more *(e.g.,* a plurality or an array or a panel of) IBS serological and/or genetic markers to generate the index.

The sample used for detecting or determining the presence or level of at least one IBS marker is typically whole blood, plasma, serum, saliva, urine, stool *(i.e.,* feces), tears, and any other bodily fluid, or a tissue sample (*i*.*e*., biopsy) such as a small intestine or colon sample. In preferred embodiments, the sample is whole blood, serum, plasma, stool, urine, or a tissue biopsy. In certain embodiments, the methods of the present invention may comprise detecting or determining the presence or level of at least one IBS marker in the sample. In other embodiments, the methods of the present invention may further comprise isolating and/or amplifying RNA from a biological sample taken from the subject.

In certain instances, the first and second samples are the same sample (*e*.*g*., whole blood, serum, or plasma sample), and a different aliquot and/or dilution of the sample is used for determining the IBS serological marker levels and for determining the IBS genetic marker levels. In particular embodiments, the first sample and the second sample are independently selected from the group consisting of whole blood, serum, plasma, and stool.

In some embodiments, the method further comprises sending the IBS diagnosis results to a clinician, *e*.*g*., a gastroenterologist or a general practitioner. In certain instances, the method of the present invention provides a diagnosis in the form of a probability that the subject has IBS. For example, the individual can have about a 0%, 5%, 10%, 15%, 20%, 25%, 30%, 35%, 40%, 45%, 50%, 55%, 60%, 65%, 70%, 75%, 80%, 85%, 90%, 95%, or greater probability of having IBS. In certain other instances, the method further provides a prognosis of IBS in the subject. For example, the prognosis can be surgery, development of a category or clinic al subtype of IBS, development of one or more symptoms, and/or recovery from the disease.

In other embodiments, the diagnosis of a subject as having IBS can be followed by determining or selecting an appropriate course of therapy or therapy regimen for the subject and/or administering to the subject a therapeutically effective amount of a drug useful for treating one or more symptoms associated with IBS. Suitable IBS drugs include, but are not limited to, serotonergic agents, antidepressants, chloride channel activators, chloride channel blockers, guanylate cyclase agonists, antibiotics, opioid agonists, neurokinin antagonists, antispasmodic or anticholinergic agents, belladonna alkaloids, barbiturates, GLP-1 analogs, CRF antagonists, probiotics, free bases thereof, pharmaceutically acceptable salts thereof, derivatives thereof, analogs thereof, and combinations thereof. Other IBS drugs include bulking agents, dopamine antagonists, carminatives, tranquilizers, dextofisopam, phenytoin, timolol, and diltiazem. Amino acids such as glutamine and glutamic acid, which regulate intestinal permeability by affecting neuronal or glial cell signaling, can be administered to treat patients with IBS.

In other aspects, the present invention provides a method for aiding or assisting in the differentiation of one or more IBS subtypes from each other (*e*.*g*., discriminating between IBS-constipation (IBS-C), IBS-diarrhea (IBS-D), IBS-mixed (IBS-M), IBS-alternating (IBS-A), and/or post-infectious IBS (IBS-PI).

In certain embodiments, the present invention provides a method for aiding in the differentiation of IBS-constipation (IBS-C) from IBS-diarrhea (IBS-D) in a subject, wherein the method comprises detecting, determining, measuring, or analyzing at least 1, 2, 3, or all 4 of the following markers: histamine, NGAL, MICALL1, and RNF26. The levels of these markers can be determined in accordance with the techniques described herein. For example, the level of an IBS serological marker can be determined by contacting a first sample from the subject with a binding moiety under conditions suitable to transform the IBS serological marker present in the first sample into a complex comprising the IBS serological marker and the binding moiety. For example, the level of an IBS genetic marker can be determined by contacting isolated and/or amplified RNA obtained from a second sample from the subject with a detection reagent under conditions suitable to transform the IBS genetic marker present in the second sample into a complex comprising the IBS genetic marker and the detection reagent. The level of each IBS serological and/or genetic marker of interest can then be determined by determining the level of the complex.

In other embodiments, the present invention provides a method for aiding in the differentiation of IBS-C from IBS-D in a subject, wherein the method comprises detecting, determining, measuring, or analyzing at least 1, 2, 3, 4, 5, 6, 7, 8, 9, 10, 11, 12, 13, 14 or all 15 of the following markers: histamine, tTG, VIPR1, substance P, IL-12, IL-10, IL-6, IL-1β, TNF-α, RRP7A, CCDC147, ASCA IgA, NGAL, MAP6D1, and GRO-α. The levels of these markers can be determined in accordance with the techniques described herein. For example, the level of an IBS serological marker can be determined by contacting a first sample from the subject with a binding moiety under conditions suitable to transform the IBS serological marker present in the first sample into a complex comprising the IBS serological marker and the binding moiety. For example, the level of an IBS genetic marker can be determined by contacting isolated and/or amplified RNA obtained from a second sample from the subject with a detection reagent under conditions suitable to transform the IBS genetic marker present in the second sample into a complex comprising the IBS genetic marker and the detection reagent. The level of each IBS serological and/or genetic marker of interest can then be determined by determining the level of the complex.

In related embodiments, the present invention provides a method for aiding in the differentiation of IBS-C from IBS-D in a subject, the method comprising:
(a) contacting a first sample from the subject with a binding moiety under conditions suitable to transform an IBS serological marker present in the first sample into a complex comprising the IBS serological marker and the binding moiety,
   wherein the IBS serological marker is selected from the group consisting of histamine, neutrophil gelatinase-associated lipocalin (NGAL), and combinations thereof;
(b) contacting isolated and/or amplified RNA obtained from a second sample from the subject with a detection reagent under conditions suitable to transform an IBS genetic marker present in the second sample into a complex comprising the IBS genetic marker and the detection reagent,
   wherein the IBS genetic marker is selected from the group consisting of MICALL1, RNF26, and combinations thereof;
(c) determining the level of the complex in step (a), thereby determining the level of the IBS serological marker present in the first sample; and
(d) determining the level of the complex in step (b), thereby determining the level of the IBS genetic marker present in the second sample.

In particular embodiments, the IBS serological marker comprises a combination of histamine and NGAL and/or the IBS genetic marker comprises a combination of MICALL1 and RNF26. In certain embodiments, the methods of the invention for discriminating or aiding in the differentiation of subjects with IBS-C from subjects with IBS-D may comprise detecting, determining, measuring, or analyzing the (concentration) level of histamine and NGAL in a first sample and the gene expression level of MICALL1 and RNF26 in a second sample.

In some embodiments, the method for aiding or assisting in the differentiation of IBS-C from IBS-D further comprises determining the level of at least 1, 2, 3, 4, 5, 6, 7, 8, 9, 10, 11, 12, 13, 14, 15, 16, 17, or all 18 of the following IBS serological markers: PGE2, tryptase, serotonin, substance P, IL-12, IL-10, IL-6, IL-8, TNF-α, IL-1β, GRO-α, BDNF, ASCA IgA, anti-CBir1 antibody, tTG, TWEAK, ANCA, TIMP-1, or combinations thereof.

In other embodiments, the method for aiding or assisting in the differentiation of IBS-C from IBS-D further comprises determining the level of at least 1, 2, 3, 4, 5, 6, 7, 8, 9, 10, 11, or all 12 of the following IBS genetic markers: CBFA2T2, CCDC147, HSD17B11, LDLR, MAP6D1, RAB7L1, RRP7A, SUSD4, SH3BGRL3, VIPR1, WEE1, ZNF326, or combinations thereof.

In some embodiments, the IBS serological marker comprises a combination of histamine, NGAL, PGE2, tryptase, serotonin, substance P, IL-12, IL-10, IL-6, IL-8, TNF-α, IL-1β, GRO-α, BDNF, ASCA IgA, anti-CBir1 antibody, tTG, TWEAK, ANCA, and TIMP-1. In other embodiments, the IBS genetic marker comprises a combination of RNF26, CBFA2T2, CCDC147, HSD17B11, LDLR, MAP6D1, RAB7L1, RRP7A, SUSD4, SH3BGRL3, VIPR1, WEE1, MICALL1, and ZNF326. In particular embodiments, the method for aiding or assisting in the differentiation of IBS-C from IBS-D further comprises determining the level of all of these IBS serological and genetic markers.

In certain embodiments, the method for aiding or assisting in the differentiation of IBS-C from IBS-D further comprises comparing the determined level of the IBS serological or genetic marker present in a sample to a control level, wherein a similarity or a difference in the level of the IBS serological or genetic marker relative to the control level is predictive or indicative of an increased or higher likelihood of the subject having either IBS-C or IBS-D.

In particular embodiments, the level of the IBS serological marker and/or the level of the IBS genetic marker is compared to a control level of the same marker from a subject having IBS-C. In some instances, the same level or a similar level of the IBS serological or genetic marker present in the sample relative to the control level is predictive or indicative of an increased or higher likelihood of the subject having IBS-C (and not having IBS-D). In other instances, a reduced or an increased level of the IBS serological or genetic marker present in the sample relative to the control level is predictive or indicative of an increased or higher likelihood of the subject not having IBS-C.

In other embodiments, the level of the IBS serological marker and/or the level of the IBS genetic marker is compared to a control level of the same marker from a subject having IBS-D. In some instances, the same level or a similar level of the IBS serological or genetic marker present in the sample relative to the control level is predictive or indicative of an increased or higher likelihood of the subject having IBS-D (and not having IBS-C). In other instances, a reduced or an increased level of the IBS serological or genetic marker present in the sample relative to the control level is predictive or indicative of an increased or higher likelihood of the subject not having IBS-D.

In further embodiments, the method for aiding or assisting in the differentiation of IBS-C from IBS-D further comprises comparing the determined level of the IBS serological or genetic marker present in a sample to a cutoff value or reference value or threshold value, wherein the level of the IBS serological or genetic marker above or below that value is predictive or indicative of an increased or higher likelihood of the subject having either IBS-C or IBS-D. One skilled in the art will understand that the cutoff value or reference value or threshold value is in units such as mg/ml, µg/ml, ng/ml, pg/ml, fg/ml, EU/ml, or U/ml depending on the marker of interest that is being measured.

In some embodiments, the method further comprises determining a psychological measure of the subject. The psychological measures of the invention can include, but are not limited to, a Patient Health Questionnaire 15 (PHQ-15), a PHQ-15 wherein gastrointestinal symptoms have been excluded from consideration (PHQ-non GI), a perceived stress scale (PSS), a Hospital Anxiety and/or Depression scale (HADs) *(e.g.,* an anxiety score on the HADs and/or depression score on the HADs), an IBS-Severity Scoring System (IBS-SSS), a Functional Bowel Disease Severity Index (FBDSI), a self-report of overall IBS severity (*e*.*g*., bowel symptom questionnaires such as the Rome III 10-question IBS Module, the Bristol Stool Form Scale, and/or the Rome III 93-question GI questionnaire), a self-rated pain severity, and combinations thereof.

In certain embodiments, the method for aiding or assisting in the differentiation of IBS-C from IBS-D comprises determining the level of the IBS serological markers histamine, NGAL, and substance P, the level of the IBS genetic markers RNF26, RRP7A, and RAB7L1, and the psychological measures PHQ-non GI and PSS.

In other embodiments, the method further comprises applying an algorithm or a combination thereof to the determined level(s) of the IBS serological marker(s) and/or IBS genetic marker(s) and/or to the psychological measure(s) determined for the subject. In certain instances, the algorithm is a statistical algorithm such as, for example, regression analysis (*e*.*g*., logistic regression, linear regression) and/or a learning statistical classifier system. The learning statistical classifier system can be selected from the group consisting of a random forest (RF), classification and regression tree (C&RT), boosted tree, neural network (NN), support vector machine (SVM), general chi-squared automatic interaction detector model, interactive tree, multiadaptive regression spline, machine learning classifier, and combinations thereof. In some instances, the learning statistical classifier system is a tree-based statistical algorithm *(e.g.,* RF, C&RT, *etc.)* and/or a NN *(e.g.,* artificial NN, *etc.).*

In certain embodiments, the determined levels of one or more *(e.g.,* a plurality or an array or a panel of) IBS serological and/or genetic markers can be used to generate an index comprising a representation of the concentration levels of each of the markers, and the index that is generated can be compared to that of a control *(e.g.,* an index generated from the levels of the same markers in a sample from a subject having IBS-C or IBS-D), to aid or assist in the differentiation of IBS-C from IBS-D. In certain instances, one or more algorithms can be applied to the determined levels of the one or more *(e.g.,* a plurality or an array or a panel of) IBS serological and/or genetic markers to generate the index.

The sample used for detecting or determining the presence or level of at least one IBS marker is typically whole blood, plasma, serum, saliva, urine, stool *(i.e.,* feces), tears, and any other bodily fluid, or a tissue sample (*i.e.,* biopsy) such as a small intestine or colon sample. In preferred embodiments, the sample is whole blood, serum, plasma, stool, urine, or a tissue biopsy. In certain embodiments, the methods of the present invention may further comprise detecting or determining the presence or level of at least one IBS marker in the sample. In other embodiments, the methods of the present invention may further comprise isolating and/or amplifying RNA from a biological sample taken from the subject.

In certain instances, the first and second samples are the same sample (*e*.*g*., whole blood, serum, or plasma sample), and a different aliquot and/or dilution of the sample is used for determining the IBS serological marker levels and for determining the IBS genetic marker levels. In particular embodiments, the first sample and the second sample are independently selected from the group consisting of whole blood, serum, plasma, and stool.

In certain other instances, the subject has previously been diagnosed with IBS, *e.g.,* in accordance with the methods described herein for differentiating IBS subjects from healthy subjects and/or using the Rome III criteria.

In other embodiments, the present invention provides a method for aiding in the differentiation of IBS-C from IBS-M in a subject, wherein the method comprises detecting, determining, measuring, or analyzing at least 1, 2, 3, or all 4 of the following markers: tTG, IL-6, RAB7L1, and VIPR1. The levels of these markers can be determined in accordance with the techniques described herein. For example, the level of an IBS serological marker can be determined by contacting a first sample from the subject with a binding moiety under conditions suitable to transform the IBS serological marker present in the first sample into a complex comprising the IBS serological marker and the binding moiety. For example, the level of an IBS genetic marker can be determined by contacting isolated and/or amplified RNA obtained from a second sample from the subject with a detection reagent under conditions suitable to transform the IBS genetic marker present in the second sample into a complex comprising the IBS genetic marker and the detection reagent. The level of each IBS serological and/or genetic marker of interest can then be determined by determining the level of the complex.

In some embodiments, the present invention provides methods for discriminating IBS-C subjects from IBS-M subjects, by analyzing, determining, or measuring at least 1, 2, 3, 4, 5, 6, 7, 8 or all 9 of the following markers: MAP6D1, RAB7L1, NGAL, serotonin, VIPR1, IL-1β, IL-10, IL-6, and RRP7A. The levels of these markers can be determined in accordance with the techniques described herein. For example, the level of an IBS serological marker can be determined by contacting a first sample from the subject with a binding moiety under conditions suitable to transform the IBS serological marker present in the first sample into a complex comprising the IBS serological marker and the binding moiety. For example, the level of an IBS genetic marker can be determined by contacting isolated and/or amplified RNA obtained from a second sample from the subject with a detection reagent under conditions suitable to transform the IBS genetic marker present in the second sample into a complex comprising the IBS genetic marker and the detection reagent. The level of each IBS serological and/or genetic marker of interest can then be determined by determining the level of the complex.

In related embodiments, the present invention provides a method for aiding in the differentiation of IBS-C from IBS-M in a subject, the method comprising:
(a) contacting a first sample from the subject with a binding moiety under conditions suitable to transform an IBS serological marker present in the first sample into a complex comprising the IBS serological marker and the binding moiety,
   wherein the IBS serological marker is selected from the group consisting of tTG, IL-6, and combinations thereof;
(b) contacting isolated and/or amplified RNA obtained from a second sample from the subject with a detection reagent under conditions suitable to transform an IBS genetic marker present in the second sample into a complex comprising the IBS genetic marker and the detection reagent,
   wherein the IBS genetic marker is selected from the group consisting of RAB7L1, VIPR1, and combinations thereof;
(c) determining the level of the complex in step (a), thereby determining the level of the IBS serological marker present in the first sample; and
(d) determining the level of the complex in step (b), thereby determining the level of the IBS genetic marker present in the second sample.

In particular embodiments, the IBS serological marker comprises a combination of tTG and IL-6 and/or the IBS genetic marker comprises a combination of RAB7L1 and VIPER1. In certain embodiments, the methods of the invention for discriminating or aiding in the differentiation of subjects with IBS-C from subjects with IBS-M may comprise detecting, determining, measuring, or analyzing the (concentration) level of tTG and IL-6 in a first sample and the gene expression level of RAB7L1 and VIPR1 in a second sample.

In some embodiments, the method for aiding or assisting in the differentiation of IBS-C from IBS-M further comprises determining the level of at least 1, 2, 3, 4, 5, 6, 7, 8, 9, 10, 11, 12, 13, 14, 15, 16, 17, or all 18 of the following IBS serological markers: histamine, PGE2, tryptase, serotonin, substance P, IL-12, IL-10, IL-8, TNF-α, IL-1β, GRO-α, BDNF, ASCA IgA, anti-CBir1 antibody, TWEAK, ANCA, TIMP-1, NGAL or combinations thereof.

In other embodiments, the method for aiding or assisting in the differentiation of IBS-C from IBS-M further comprises determining the level of at least 1, 2, 3, 4, 5, 6, 7, 8, 9, 10, 11, or all 12 of the following IBS genetic markers: CBFA2T2, CCDC147, HSD17B11, LDLR, MAP6D1, RRP7A, SUSD4, SH3BGRL3, WEE1, ZNF326, MICALL1, RNF26, or combinations thereof.

In some embodiments, the IBS serological marker comprises a combination of histamine, NGAL, PGE2, tryptase, serotonin, substance P, IL-12, IL-10, IL-6, IL-8, TNF-α, IL-1β, GRO-α, BDNF, ASCA IgA, anti-CBir1 antibody, tTG, TWEAK, ANCA, and TIMP-1. In other embodiments, the IBS genetic marker comprises a combination of RNF26, CBFA2T2, CCDC147, HSD17B11, LDLR, MAP6D1, RAB7L1, RRP7A, SUSD4, SH3BGRL3, VIPR1, WEE1, MICALL1, and ZNF326. In particular embodiments, the method for aiding or assisting in the differentiation of IBS-C from IBS-M further comprises determining the level of all of these IBS serological and genetic markers.

In certain embodiments, the method for aiding or assisting in the differentiation of IBS-C from IBS-M further comprises comparing the determined level of the IBS serological or genetic marker present in a sample to a control level, wherein a similarity or a difference in the level of the IBS serological or genetic marker relative to the control level is predictive or indicative of an increased or higher likelihood of the subject having either IBS-C or IBS-M.

In particular embodiments, the level of the IBS serological marker and/or the level of the IBS genetic marker is compared to a control level of the same marker from a subject having IBS-C. In some instances, the same level or a similar level of the IBS serological or genetic marker present in the sample relative to the control level is predictive or indicative of an increased or higher likelihood of the subject having IBS-C (and not having IBS-M). In other instances, a reduced or an increased level of the IBS serological or genetic marker present in the sample relative to the control level is predictive or indicative of an increased or higher likelihood of the subject not having IBS-C.

In other embodiments, the level of the IBS serological marker and/or the level of the IBS genetic marker is compared to a control level of the same marker from a subject having IBS-M. In some instances, the same level or a similar level of the IBS serological or genetic marker present in the sample relative to the control level is predictive or indicative of an increased or higher likelihood of the subject having IBS-M (and not having IBS-C). In other instances, a reduced or an increased level of the IBS serological or genetic marker present in the sample relative to the control level is predictive or indicative of an increased or higher likelihood of the subject not having IBS-M.

In further embodiments, the method for aiding or assisting in the differentiation of IBS-C from IBS-M further comprises comparing the determined level of the IBS serological or genetic marker present in a sample to a cutoff value or reference value or threshold value, wherein the level of the IBS serological or genetic marker above or below that value is predictive or indicative of an increased or higher likelihood of the subject having either IBS-C or IBS-M. One skilled in the art will understand that the cutoff value or reference value or threshold value is in units such as mg/ml, µg/ml, ng/ml, pg/ml, fg/ml, EU/ml, or U/ml depending on the marker of interest that is being measured.

In some embodiments, the method further comprises determining a psychological measure of the subject. The psychological measures of the invention can include, but are not limited to, a Patient Health Questionnaire 15 (PHQ-15), a PHQ-15 wherein gastrointestinal symptoms have been excluded from consideration (PHQ-non GI), a perceived stress scale (PSS), a Hospital Anxiety and/or Depression scale (HADs) *(e.g.,* an anxiety score on the HADs and/or depression score on the HADs), an IBS-Severity Scoring System (IBS-SSS), a Functional Bowel Disease Severity Index (FBDSI), a self-report of overall IBS severity (*e*.*g*., bowel symptom questionnaires such as the Rome III 10-question IBS Module, the Bristol Stool Form Scale, and/or the Rome III 93-question GI questionnaire), a self-rated pain severity, and combinations thereof.

In certain embodiments, the method for aiding or assisting in the differentiation of IBS-C from IBS-M comprises determining the level of the IBS serological marker IL-6, the level of the IBS genetic markers MAP6D1, VIPR1, and RAB7L1, and the psychological measures PHQ-non GI and HAD depression.

In other embodiments, the method further comprises applying an algorithm or a combination thereof to the determined level(s) of the IBS serological marker(s) and/or IBS genetic marker(s) and/or to the psychological measure(s) determined for the subject. In certain instances, the algorithm is a statistical algorithm such as, for example, regression analysis (*e*.*g*., logistic regression, linear regression) and/or a learning statistical classifier system.

In certain embodiments, the determined levels of one or more *(e.g.,* a plurality or an array or a panel of) IBS serological and/or genetic markers can be used to generate an index comprising a representation of the concentration levels of each of the markers, and the index that is generated can be compared to that of a control *(e.g.,* an index generated from the levels of the same markers in a sample from a subject having IBS-C or IBS-M), to aid or assist in the differentiation of IBS-C from IBS-M. In certain instances, one or more algorithms can be applied to the determined levels of the one or more *(e.g.,* a plurality or an array or a panel of) IBS serological and/or genetic markers to generate the index.

The sample used for detecting or determining the presence or level of at least one IBS marker is typically whole blood, plasma, serum, saliva, urine, stool *(i.e.,* feces), tears, and any other bodily fluid, or a tissue sample (*i*.*e*., biopsy) such as a small intestine or colon sample. In preferred embodiments, the sample is whole blood, serum, plasma, stool, urine, or a tissue biopsy. In certain embodiments, the methods of the present invention may further comprise detecting or determining the presence or level of at least one IBS marker in the sample. In other embodiments, the methods of the present invention may further comprise isolating and/or amplifying RNA from a biological sample taken from the subject.

In certain instances, the first and second samples are the same sample (*e*.*g*., whole blood, serum, or plasma sample), and a different aliquot and/or dilution of the sample is used for determining the IBS serological marker levels and for determining the IBS genetic marker levels. In particular embodiments, the first sample and the second sample are independently selected from the group consisting of whole blood, serum, plasma, and stool.

In certain other instances, the subject has previously been diagnosed with IBS, *e.g.,* in accordance with the methods described herein for differentiating IBS subjects from healthy subjects and/or using the Rome III criteria.

In yet other embodiments, the present invention provides a method for aiding in the differentiation of IBS-D from IBS-M in a subject, wherein the method comprises detecting, determining, measuring, or analyzing at least 1, 2, 3, 4, or all 5 of the following markers: histamine, tTG, TWEAK, VIPR1, and RNF26. The levels of these markers can be determined in accordance with the techniques described herein. For example, the level of an IBS serological marker can be determined by contacting a first sample from the subject with a binding moiety under conditions suitable to transform the IBS serological marker present in the first sample into a complex comprising the IBS serological marker and the binding moiety. For example, the level of an IBS genetic marker can be determined by contacting isolated and/or amplified RNA obtained from a second sample from the subject with a detection reagent under conditions suitable to transform the IBS genetic marker present in the second sample into a complex comprising the IBS genetic marker and the detection reagent. The level of each IBS serological and/or genetic marker of interest can then be determined by determining the level of the complex.

In further embodiments, the present invention provides a method for aiding in the differentiation of IBS-D from IBS-M in a subject, wherein the method comprises detecting, determining, measuring, or analyzing at least 1, 2, 3, 4, 5, 6, or all 7 of the following markers: histamine, PGE2, GRO-α, tTG, TWEAK, RNF26, and VIPR1. The levels of these markers can be determined in accordance with the techniques described herein. For example, the level of an IBS serological marker can be determined by contacting a first sample from the subject with a binding moiety under conditions suitable to transform the IBS serological marker present in the first sample into a complex comprising the IBS serological marker and the binding moiety. For example, the level of an IBS genetic marker can be determined by contacting isolated and/or amplified RNA obtained from a second sample from the subject with a detection reagent under conditions suitable to transform the IBS genetic marker present in the second sample into a complex comprising the IBS genetic marker and the detection reagent. The level of each IBS serological and/or genetic marker of interest can then be determined by determining the level of the complex.

In related embodiments, the present invention provides a method for aiding in the differentiation of IBS-D from IBS-M in a subject, the method comprising:
(a) contacting a first sample from the subject with a binding moiety under conditions suitable to transform an IBS serological marker present in the first sample into a complex comprising the IBS serological marker and the binding moiety,
   wherein the IBS serological marker is selected from the group consisting of histamine, tTG, TWEAK, and combinations thereof;
(b) contacting isolated and/or amplified RNA obtained from a second sample from the subject with a detection reagent under conditions suitable to transform an IBS genetic marker present in the second sample into a complex comprising the IBS genetic marker and the detection reagent,
   wherein the IBS genetic marker is selected from the group consisting of VIPR1, RNF26, and combinations thereof;
(c) determining the level of the complex in step (a), thereby determining the level of the IBS serological marker present in the first sample; and
(d) determining the level of the complex in step (b), thereby determining the level of the IBS genetic marker present in the second sample.

In particular embodiments, the IBS serological marker comprises a combination of histamine, tTG, and TWEAK and/or the IBS genetic marker comprises a combination of VIPR1 and RNF26. In certain embodiments, the methods of the invention for discriminating or aiding in the differentiation of subjects with IBS-D from subjects with IBS-M may comprise detecting, determining, measuring, or analyzing the (concentration) level of histamine, tTG, and TWEAK in a first sample and the gene expression level of VIPR1 and RNF26 in a second sample.

In some embodiments, the method for aiding or assisting in the differentiation of IBS-D from IBS-M further comprises determining the level of at least 1, 2, 3, 4, 5, 6, 7, 8, 9, 10, 11, 12, 13, 14, 15, 16, or all 17 of the following IBS serological markers: NGAL, PGE2, tryptase, serotonin, substance P, IL-12, IL-10, IL-6, IL-8, TNF-α, IL-1β, GRO-α, BDNF, ASCA IgA, anti-CBir1 antibody, ANCA, TIMP-1, or combinations thereof.

In other embodiments, the method for aiding or assisting in the differentiation of IBS-D from IBS-M further comprises determining the level of at least 1, 2, 3, 4, 5, 6, 7, 8, 9, 10, 11, or all 12 of the following IBS genetic markers: CBFA2T2, CCDC147, HSD17B11, LDLR, MAP6D1, RAB7L1, RRP7A, SUSD4, SH3BGRL3, WEE1, MICALL1, ZNF326, or combinations thereof.

In some embodiments, the IBS serological marker comprises a combination of histamine, NGAL, PGE2, tryptase, serotonin, substance P, IL-12, IL-10, IL-6, IL-8, TNF-α, IL-1β, GRO-α, BDNF, ASCA IgA, anti-CBir1 antibody, tTG, TWEAK, ANCA, and TIMP-1. In other embodiments, the IBS genetic marker comprises a combination of RNF26, CBFA2T2, CCDC147, HSD17B11, LDLR, MAP6D1, RAB7L1, RRP7A, SUSD4, SH3BGRL3, VIPR1, WEE1, MICALL1, and ZNF326. In particular embodiments, the method for aiding or assisting in the differentiation of IBS-D from IBS-M further comprises determining the level of all of these IBS serological and genetic markers.

In certain embodiments, the method for aiding or assisting in the differentiation of IBS-D from IBS-M further comprises comparing the determined level of the IBS serological or genetic marker present in a sample to a control level, wherein a similarity or a difference in the level of the IBS serological or genetic marker relative to the control level is predictive or indicative of an increased or higher likelihood of the subject having either IBS-D or IBS-M.

In particular embodiments, the level of the IBS serological marker and/or the level of the IBS genetic marker is compared to a control level of the same marker from a subject having IBS-D. In some instances, the same level or a similar level of the IBS serological or genetic marker present in the sample relative to the control level is predictive or indicative of an increased or higher likelihood of the subject having IBS-D (and not having IBS-M). In other instances, a reduced or an increased level of the IBS serological or genetic marker present in the sample relative to the control level is predictive or indicative of an increased or higher likelihood of the subject not having IBS-D.

In other embodiments, the level of the IBS serological marker and/or the level of the IBS genetic marker is compared to a control level of the same marker from a subject having IBS-M. In some instances, the same level or a similar level of the IBS serological or genetic marker present in the sample relative to the control level is predictive or indicative of an increased or higher likelihood of the subject having IBS-M (and not having IBS-D). In other instances, a reduced or an increased level of the IBS serological or genetic marker present in the sample relative to the control level is predictive or indicative of an increased or higher likelihood of the subject not having IBS-M.

In further embodiments, the method for aiding or assisting in the differentiation of IBS-D from IBS-M further comprises comparing the determined level of the IBS serological or genetic marker present in a sample to a cutoff value or reference value or threshold value, wherein the level of the IBS serological or genetic marker above or below that value is predictive or indicative of an increased or higher likelihood of the subject having either IBS-D or IBS-M. One skilled in the art will understand that the cutoff value or reference value or threshold value is in units such as mg/ml, µg/ml, ng/ml, pg/ml, fg/ml, EU/ml, or U/ml depending on the marker of interest that is being measured.

In some embodiments, the method further comprises determining a psychological measure of the subject. The psychological measures of the invention can include, but are not limited to, a Patient Health Questionnaire 15 (PHQ-15), a PHQ-15 wherein gastrointestinal symptoms have been excluded from consideration (PHQ-non GI), a perceived stress scale (PSS), a Hospital Anxiety and/or Depression scale (HADs) *(e.g.,* an anxiety score on the HADs and/or depression score on the HADs), an IBS-Severity Scoring System (IBS-SSS), a Functional Bowel Disease Severity Index (FBDSI), a self-report of overall IBS severity (*e*.*g*., bowel symptom questionnaires such as the Rome III 10-question IBS Module, the Bristol Stool Form Scale, and/or the Rome III 93-question GI questionnaire), a self-rated pain severity, and combinations thereof.

In certain embodiments, the method for aiding or assisting in the differentiation of IBS-D from IBS-M comprises determining the level of the IBS serological markers GRO-α, PGE2, and TWEAK, the level of the IBS genetic markers RNF26 and VIPR1, and the psychological measures HAD anxiety and HAD depression.

In other embodiments, the method further comprises applying an algorithm or a combination thereof to the determined level(s) of the IBS serological marker(s) and/or IBS genetic marker(s) and/or to the psychological measure(s) determined for the subject. In certain instances, the algorithm is a statistical algorithm such as, for example, regression analysis (*e*.*g*., logistic regression, linear regression) and/or a learning statistical classifier system.

In certain embodiments, the determined levels of one or more *(e.g.,* a plurality or an array or a panel of) IBS serological and/or genetic markers can be used to generate an index comprising a representation of the concentration levels of each of the markers, and the index that is generated can be compared to that of a control *(e.g.,* an index generated from the levels of the same markers in a sample from a subject having IBS-D or IBS-M), to aid or assist in the differentiation of IBS-D from IBS-M. In certain instances, one or more algorithms can be applied to the determined levels of the one or more *(e.g.,* a plurality or an array or a panel of) IBS serological and/or genetic markers to generate the index.

The sample used for detecting or determining the presence or level of at least one IBS marker is typically whole blood, plasma, serum, saliva, urine, stool *(i.e.,* feces), tears, and any other bodily fluid, or a tissue sample *(i.e.,* biopsy) such as a small intestine or colon sample. In preferred embodiments, the sample is whole blood, serum, plasma, stool, urine, or a tissue biopsy. In certain embodiments, the methods of the present invention may further comprise detecting or determining the presence or level of at least one IBS marker in the sample. In other embodiments, the methods of the present invention may further comprise isolating and/or amplifying RNA from a biological sample taken from the subject.

In certain instances, the first and second samples are the same sample (*e*.*g*., whole blood, serum, or plasma sample), and a different aliquot and/or dilution of the sample is used for determining the IBS serological marker levels and for determining the IBS genetic marker levels. In particular embodiments, the first sample and the second sample are independently selected from the group consisting of whole blood, serum, plasma, and stool.

In certain other instances, the subject has previously been diagnosed with IBS, *e.g.,* in accordance with the methods described herein for differentiating IBS subjects from healthy subjects and/or using the Rome III criteria.

In certain embodiments, the methods further comprise sending the results from the IBS differentiation to a clinician. In certain other embodiments, the methods further provide a diagnosis in the form of a probability that the subject has IBS-C, IBS-D, or IBS-M.

In some embodiments, the methods can further comprise determining or selecting an appropriate course of therapy or therapy regimen for the subject and/or administering to the subject a therapeutically effective amount of a drug useful for treating IBS-C, IBS-D, or IBS-M. Suitable drugs include, but are not limited to, tegaserod (Zelnorm), alosetron (Lotronex®), lubiprostone (Amitiza), rifamixin (Xifaxan), MD-1100, probiotics, and a combination thereof. In instances where a subject is determined to have IBS-C *(e.g.,* based on differentiation from IBS-D and/or IBS-M), a therapeutically effective amount of tegaserod and/or other 5-HT₄ agonists *(e.g.,* mosapride, renzapride, AG1-001, *etc.),* lubiprostone and/or other chloride channel activators, rifamixin and/or other antibiotics capable of controlling intestinal bacterial overgrowth, MD-1100 and/or other guanylate cyclase agonists, asimadoline and/or other opioid agonists, and/or talnetant and/or other neurokinin antagonists can be administered to the subject. In other instances where a subject is determined to have IBS-D (*e*.*g*., based on differentiation from IBS-C and/or IBS-M), a therapeutically effective amount of alosetron and/or other 5-HT₃ antagonists *(e.g.,* ramosetron, DDP-225, *etc.),* crofelemer and/or other chloride channel blockers, talnetant and/or other neurokinin antagonists *(e.g.,* saredutant, *etc.),* and/or an antidepressant such as a tricyclic antidepressant can be administered to the subject.

In certain other aspects, the present invention provides a method for monitoring the progression or regression of IBS or an IBS subtype in a subject, the method comprising: (a) contacting a first sample (*e*.*g*., blood or serum) from the subject at a first time with a binding moiety under conditions suitable to transform an IBS serological marker present in the first sample into a complex comprising the IBS serological marker and the binding moiety, and wherein the IBS serological marker comprises at least 1, 2, 3, 4, 5, 6, 7, 8, 9, 10, 11, 12, 13, 14, 15, 16, 17, 18, 19, or all 20 of the following serological markers in the sample: IL-1β, GRO-α, BDNF, ASCAIgA, anti-CBir1, tTG, TWEAK, ANCA, TIMP-1, NGAL, histamine, prostaglandin E2 (PGE2), tryptase, serotonin, substance P, IL-12, IL-10, IL-6, IL-8, and TNF-α; (b) contacting isolated and/or amplified RNA obtained from a second sample (*e*.*g*., blood or serum) from the subject at a first time with a detection reagent under conditions suitable to transform an IBS genetic marker present in the second sample into a complex comprising the IBS genetic marker and the detection reagent, and wherein the IBS genetic marker comprises at least 1, 2, 3, 4, 5, 6, 7, 8, 9, 10, 11, 12, 13, or all 14 of the following genetic markers in the second sample: CBFA2T2, CCDC147, HSD17B11, LDLR, MAP6D1, MICALL1, RAB7L1, RNF26, RRP7A, SUSD4, SH3BGRL3, VIPR1, WEE1, and ZNF326; (c) determining the level of the complex in step (a), thereby determining the level of the IBS serological marker present in the first sample; (d) determining the level of the complex in step (b), thereby determining the level of the IBS genetic marker present in the second sample; (e) contacting a third sample (*e*.*g*., blood or serum) from the subject at a second time with a binding moiety under conditions suitable to transform the IBS serological marker present in the third sample into a complex comprising the IBS serological marker and the binding moiety; (f) contacting isolated and/or amplified RNA obtained from a fourth sample (*e*.*g*., blood or serum) from the subject at a second time with a detection reagent under conditions suitable to transform the IBS genetic marker present in the fourth sample into a complex comprising the IBS genetic marker and the detection reagent; (g) determining the level of the complex in step (e), thereby determining the level of the IBS serological marker present in the third sample; (h) determining the level of the complex in step (f), thereby determining the level of the IBS genetic marker present in the fourth sample; (i) comparing the level of the IBS serological marker present in the first and third samples; and (j) comparing the level of the IBS genetic marker present in the second and fourth samples.

In some embodiments, a similarity or a difference in the level of the IBS serological and/or genetic marker over time is predictive or indicative of the progression or regression of IBS or an IBS subtype in the subject. As a non-limiting example, a higher level of the IBS serological and/or genetic marker over time can be predictive or indicative of the progression of IBS or an IBS subtype in the subject, while a lower level of the IBS serological and/or genetic marker over time can be predictive or indicative of the regression of IBS or an IBS subtype in the subject.

In yet other aspects, the present invention provides a computer-readable medium comprising code for controlling one or more processors to aid in the differentiation of IBS subjects from healthy subjects or to aid in the discrimination of one or more IBS subtypes from each other (*e*.*g*., differentiating IBS-C from IBS-D, IBS-D from IBS-M, and/or IBS-D from IBS-M), the code comprising instructions to apply a statistical process to a data set comprising the presence, (concentration) level, and/or gene expression level of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or all 34 of the following serological and/or genetic markers in a sample: IL-1β, GRO-α, BDNF, ASCAIgA, anti-CBir1, tTG, TWEAK, ANCA, TIMP-1, NGAL, histamine, prostaglandin E2 (PGE2), tryptase, serotonin, substance P, IL-12, IL-10, IL-6, IL-8, TNF-α CBFA2T2, CCDC147, HSD17B11, LDLR, MAP6D1, MICALL1, RAB7L1, RNF26, RRP7A, SUSD4, SH3BGRL3, VIPR1, WEE1, and/or ZNF326; to produce a statistically derived decision differentiating IBS subjects from healthy subjects or discriminating one or more IBS subtypes from each other based upon the presence, (concentration) level, and/or gene expression level of the IBS markers.

In certain other aspects, the present invention provides a system for aiding in the differentiation of IBS subjects from healthy subjects or aiding in the discrimination of one or more IBS subtypes from each other (*e*.*g*., differentiating IBS-C from IBS-D, IBS-D from IBS-M, and/or IBS-D from IBS-M), the system comprising: (a) a data acquisition module configured to produce a data set comprising the presence, (concentration) level, and/or gene expression level of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or all 34 of the following serological and/or genetic markers in a sample: IL-1β, GRO-α, BDNF, ASCA IgA, anti-CBir1, tTG, TWEAK, ANCA, TIMP-1, NGAL, histamine, prostaglandin E2 (PGE2), tryptase, serotonin, substance P, IL-12, IL-10, IL-6, IL-8, TNF-α CBFA2T2, CCDC147, HSD17B11, LDLR, MAP6D1, MICALL1, RAB7L1, RNF26, RRP7A, SUSD4, SH3BGRL3, VIPR1, WEE1, and/or ZNF326; (b) a data processing module configured to process the data set by applying a statistical process to the data set to produce a statistically derived decision differentiating IBS subjects from healthy subjects or discriminating one or more IBS subtypes from each other based upon the presence, (concentration) level, and/or gene expression level of the IBS markers; and (c) a display module configured to display the statistically derived decision.

### IV. IBS Markers

In some aspects, the present invention provides unique IBS biomarkers and panels thereof to aid or assist in diagnosing IBS (*e*.*g*., compared with healthy subjects) and/or to aid or assist in discriminating between various subtypes of IBS from each other. In particular embodiments, the presence, (concentration) level, and/or gene expression level of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or all 34 of the following serological and/or genetic markers are measured in a sample: interleukin-1β (IL-1β), growth-related oncogene-α (GRO-α), brain-derived neurotrophic factor (BDNF), anti*-Saccharomyces cerevisiae* antibody (ASCA IgA), antibody against CBir1 (anti-CBir1), anti-human tissue transglutaminase (tTG), tumor necrosis factor (TNF)-like weak inducer of apoptosis (TWEAK), anti-neutrophil cytoplasmic antibody (ANCA), tissue inhibitor of metalloproteinase-1 (TIMP-1), neutrophil gelatinase-associated lipocalin (NGAL), histamine, prostaglandin E2 (PGE2), tryptase, serotonin, substance P, IL-12, IL-10, IL-6, IL-8, TNF-α, CBFA2T2, CCDC147, HSD17B11, LDLR, MAP6D1, MICALL1, RAB7L1, RNF26, RRP7A, SUSD4, SH3BGRL3, VIPR1, WEE1, and/or ZNF326. In some instances, additional IBS biomarkers known to one skilled in the art and/or described herein can be included in the methods, codes, and systems of the present invention. Non-limiting examples of additional IBS biomarkers suitable for use in the methods, codes, and systems of present invention include those described in, *e.g.,* US Patent Publication Nos. US 2008/0085524, US 2008/0166719, US 2010/0094560, and US 2011/0159521; and PCT Patent Publication Nos. WO 2011/066458 and WO2011/053831.

### A. Cytokines

In some embodiments, the determination of the presence or level of one or more cytokines in a sample is useful in the present invention. As used herein, the term "cytokine" includes any of a variety of polypeptides or proteins secreted by immune cells that regulate a range of immune system functions and encompasses small cytokines such as chemokines. The term "cytokine" also includes adipocytokines, which comprise a group of cytokines secreted by adipocytes that function, for example, in the regulation of body weight, hematopoiesis, angiogenesis, wound healing, insulin resistance, the immune response, and the inflammatory response.

In certain aspects, the presence, (concentration) level, and/or gene expression level of at least one of the following cytokines is determined in a sample: TNF-α, TNF-related weak inducer of apoptosis (TWEAK), osteoprotegerin (OPG), IFN-α, IFN-β, IFN-γ, IL-1α, IL-1β, IL-1 receptor antagonist (IL-1ra), IL-2, IL-4, IL-5, IL-6, soluble IL-6 receptor (sIL-6R), IL-7, IL-8, IL-9, IL-10, IL-12 *(e.g.,* IL-12A and/or IL-12B), IL-13, IL-15, IL-17, IL-23, IL-27, CXCL1/GRO1/GROα, CXCL2/GRO2, CXCL3/GRO3, CXCL4/PF-4, CXCL5/ENA-78, CXCL6/GCP-2, CXCL7/NAP-2, CXCL9/MIG, CXCL10/IP-10, CXCL11/I-TAC, CXCL12/SDF-1, CXCL13/BCA-1, CXCL14/BRAK, CXCL15, CXCL16, CXCL17/DMC, CCL1, CCL2/MCP-1, CCL3/MIP-1α, CCL4/MIP-1β, CCL5/RANTES, CCL6/C10, CCL7/MCP-3, CCL8/MCP-2, CCL9/CCL10, CCL11/Eotaxin, CCL12/MCP-5, CCL13/MCP-4, CCL14/HCC-1, CCL15/MIP-5, CCL16/LEC, CCL17/TARC, CCL18/MIP-4, CCL19/MIP-3β, CCL20/MIP-3α, CCL21/SLC, CCL22/MDC, CCL23/MPIF1, CCL24/Eotaxin-2, CCL25/TECK, CCL26/Eotaxin-3, CCL27/CTACK, CCL28/MEC, CL1, CL2, CX₃CL1, leptin, adiponectin, resistin, active or total plasminogen activator inhibitor-1 (PAI-1), visfatin, retinol binding protein 4 (RBP4), and combinations thereof. In some embodiments, a ratio of cytokine levels is determined in a sample.

In particular embodiments, the presence or level of at least 1, 2, 3, 4, 5, 6, 7, or all 8 of the following cytokines is determined in a sample: TNF-α, TWEAK, IL-1β, IL-6, IL-8, IL-10, IL-12 *(e.g.,* IL-12A and/or IL-12B), and/or GRO-α. Exemplary protein and mRNA sequences for TNF-α are set forth in GenBank Accession Nos. NP_000585 and NM_000594, respectively. Exemplary protein and mRNA sequences for TWEAK are set forth in GenBank Accession Nos. NP_003800 and NM_003809, respectively. Exemplary protein and mRNA sequences for IL-1β are set forth in GenBank Accession Nos. NP_000567 and NM_000576, respectively. Exemplary protein and mRNA sequences for IL-6 are set forth in GenBank Accession Nos. NP_000591 and NM_000600, respectively. Exemplary protein and mRNA sequences for IL-8 are set forth in GenBank Accession Nos. NP_000575 and NM_000584, respectively. Exemplary protein and mRNA sequences for IL-10 are set forth in GenBank Accession Nos. NP_000563 and NM_000572, respectively. Exemplary protein and mRNA sequences for IL-12A are set forth in GenBank Accession Nos. NP_000873 and NM_000882, respectively. Exemplary protein and mRNA sequences for IL-12B are set forth in GenBank Accession Nos. NP_002178 and NM_002187, respectively. Exemplary protein and mRNA sequences for GRO-α are set forth in GenBank Accession Nos. NP_001502 and NM_001511, respectively.

In particular embodiments, the cytokine binding moiety is an anti-cytokine antibody or a functional fragment thereof. Suitable anti-cytokine antibodies for determining the presence or level of a cytokine such as TNF-α, TWEAK, IL-1β, IL-6, IL-8, IL-10, IL-12 (*e.g.,* IL-12A and/or IL-12B), or GRO-α are available from, *e.g.,* Thermo Fisher Scientific Inc. (Rockford, IL) and eBioscience, Inc. (San Diego, CA). In other embodiments, the cytokine binding moiety is a cytokine binding protein such as, for example, an extracellular binding protein such as a receptor or fragment thereof (*e*.*g*., receptor for TNF-α, TWEAK, IL-1β, IL-6, IL-8, IL-10, IL-12 *(e.g.,* IL-12A and/or IL-12B), or GRO-α or cytokine-binding fragments thereof) that specifically binds to a cytokine of interest.

In certain instances, the presence or level of a particular cytokine is detected at the level of mRNA expression with an assay such as, for example, a hybridization assay or an amplification-based assay. In certain other instances, the presence or level of a particular cytokine is detected at the level of protein expression using, for example, an immunoassay *(e.g.,* ELISA) or an immunohistochemical assay. Suitable ELISA kits for determining the presence or level of a cytokine of interest in a serum, plasma, saliva, or urine sample are available from, *e.g.,* R&D Systems, Inc. (Minneapolis, MN), Neogen Corp. (Lexington, KY), Alpco Diagnostics (Salem, NH), Assay Designs, Inc. (Ann Arbor, MI), BD Biosciences Pharmingen (San Diego, CA), Invitrogen (Camarillo, CA), Calbiochem (San Diego, CA), CHEMICON International, Inc. (Temecula, CA), Antigenix America Inc. (Huntington Station, NY), QIAGEN Inc. (Valencia, CA), Bio-Rad Laboratories, Inc. (Hercules, CA), Bender MedSystems Inc. (Burlingame, CA), Agdia Inc. (Elkhart, IN), American Research Products Inc. (Belmont, MA), Biomeda Corp. (Foster City, CA), BioVision, Inc. (Mountain View, CA), and/or Kamiya Biomedical Co. (Seattle, WA).

### B. Serine Proteases

In some embodiments, the determination of the presence or level of one or more serine proteases in a sample is useful in the present invention. As used herein, the term "serine protease" includes any member of a family of proteases in which one of the amino acids at the active site is serine. Non-limiting examples of serine proteases include tryptase (*e.g.,* α-tryptase, β-tryptase, γ-tryptase, and/or Δ-tryptase), elastase, chymotrypsin, trypsin, subtilisin, and combinations thereof. Tryptase is an abundant specific neutral protease of human mast cells that can be measured in various biological fluids and can serve as a useful marker for mast cell activation.

Exemplary protein and mRNA sequences for β-tryptase are set forth in GenBank Accession Nos. NP_003285 *(i.e.,* a 275 amino acid tryptase beta-1 precursor protein) and NM_003294, respectively. In certain instances, the tryptase beta-1 precursor protein is then processed by the removal of a signal peptide (amino acids 1-18) and activation peptide propeptide (amino acids 19-30), resulting in the mature β-tryptase polypeptide (amino acids 31-275; UniProt: Q15661).

In certain instances, the presence or level of a particular serine protease such as tryptase is detected at the level of mRNA expression with an assay such as, for example, a hybridization assay or an amplification-based assay. In certain other instances, the presence or level of a particular serine protease such as tryptase is detected at the level of protein expression using, for example, an immunoassay (*e*.*g*., ELISA) or an immunohistochemical assay. Suitable ELISA techniques for determining the presence or level of tryptase in a serum sample are described in, e.g., US Patent No. 8,114,616.

### C. Prostaglandins

In some embodiments, the determination of the presence or level of one or more prostaglandins in a sample is useful in the present invention. As used herein, the term "prostaglandin" includes any member of a group of lipid compounds that are derived enzymatically from fatty acids and have important functions in the animal body. Every prostaglandin contains 20 carbon atoms, including a 5-carbon ring. Prostaglandins, together with the thromboxanes and prostacyclins, form the prostanoid class of fatty acid derivatives. The prostanoid class is a subclass of the eicosanoids. Non-limiting examples of prostaglandins include prostaglandin h (PGI₂), prostaglandin E₂ (PGE₂), prostaglandin F_{2α} (PGF_{2α}), and combinations thereof.

In particular embodiments, the PGE₂ binding moiety is an anti-PGE₂ antibody or a functional fragment thereof. Suitable anti-PGE₂ antibodies for determining the presence or level of PGE₂ in a sample are available from, *e*.*g*., Abcam plc (Cambridge, MA) and Novus Biologicals (Littleton, CO). In some other embodiments, the PGE₂ binding moiety is a PGE₂ binding protein such as, for example, the PGE₂ receptor EP₂. In certain instances, PGE₂ may be detected by an ELISA or chemiluminescent assay. Suitable ELISA kits for determining the presence or level of PGE₂ in a serum sample are available from, *e*.*g*., Cayman Chemical Co. (Ann Arbor, MI).

### D. Histamine

As used herein, the term "histamine" includes a biogenic amine involved in local immune responses as well as regulating physiological function in the gut and acting as a neurotransmitter. Histamine triggers the inflammatory response. As part of an immune response to foreign pathogens, histamine is produced by basophils and by mast cells found in nearby connective tissues. Histamine increases the permeability of the capillaries to white blood cells and other proteins, in order to allow them to engage foreign invaders in the affected tissues. It is found in virtually all animal body cells.

In particular embodiments, the histamine binding moiety is an anti-histamine antibody or a functional fragment thereof. Suitable anti-histamine antibodies for determining the presence or level of histamine in a sample are available from, *e*.*g*., MyBioSource, LLC (San Diego, CA), Thermo Fisher Scientific Inc. (Rockford, IL), and Novus Biologicals (Littleton, CO). In other embodiments, the histamine binding moiety is a histamine binding protein such as, for example, a histamine binding protein derived from ticks such as EV131 and/or one of the histamine binding proteins disclosed in US Patent No. 6,617,312 and US Patent Publication No. 2011/0152171. In certain embodiments, histamine may be detected by an ELISA or chemiluminescent assay. Suitable ELISA kits for determining the presence or level of histamine in a blood, serum, plasma, or urine sample are available from, *e.g.,* GenWay Biotech, Inc. (San Diego, CA), ALPCO Diagnostics (Salem, NH), Immunotech (Czech Republic) and Cayman Chemical Co. (Ann Arbor, MI).

### E. Lipocalins

In some embodiments, the determination of the presence or level of one or more lipocalins in a sample is useful in the present invention. As used herein, the term "lipocalin" includes any of a variety of small extracellular proteins that are characterized by several common molecular recognition properties: the ability to bind a range of small hydrophobic molecules; binding to specific cell-surface receptors; and the formation of complexes with soluble macromolecules (*see, e.g.,* Flowers, Biochem. J., 318:1-14 (1996)). The varied biological functions of lipocalins are mediated by one or more of these properties. The lipocalin protein family exhibits great functional diversity, with roles in retinol transport, invertebrate cryptic coloration, olfaction and pheromone transport, and prostaglandin synthesis. Lipocalins have also been implicated in the regulation of cell homoeostasis and the modulation of the immune response, and, as carrier proteins, to act in the general clearance of endogenous and exogenous compounds. Although lipocalins have great diversity at the sequence level, their three-dimensional structure is a unifying characteristic. Lipocalin crystal structures are highly conserved and comprise a single eight-stranded continuously hydrogen-bonded antiparallel beta-barrel, which encloses an internal ligand-binding site.

In certain embodiments, the presence or level of at least one lipocalin including, but not limited to, neutrophil gelatinase-associated lipocalin (NGAL; also known as lipocalin-2 or human neutrophil lipocalin (HNL)), von Ebner's gland protein (VEGP; also known as lipocalin-1), retinol-binding protein (RBP), purpurin (PURP), retinoic acid-binding protein (RABP), α₂ᵤ-globulin (A2U), major urinary protein (MUP), bilin-binding protein (BBP), α-crustacyanin, pregnancy protein 14 (PP14), β-lactoglobulin (Blg), α₁-microglobulin (AIM), the gamma chain of C8 (C8γ), Apolipoprotein D (ApoD), lazarillo (LAZ), prostaglandin D2 synthase (PGDS), quiescence-specific protein (QSP), choroid plexus protein, odorant-binding protein (OBP), α₁-acid glycoprotein (AGP), probasin (PBAS), aphrodisin, orosomucoid, and progestagen-associated endometrial protein (PAEP) is determined in a sample. In certain other embodiments, the presence or level of at least one lipocalin complex including, for example, a complex of NGAL and a matrix metalloproteinase (*e*.*g*., NGAL/MMP-9 complex) is determined. Exemplary protein and mRNA sequences for NGAL are set forth in GenBank Accession Nos. NP_005555 and NM_005564, respectively.

In particular embodiments, the NGAL binding moiety is an anti-NGAL antibody or a functional fragment thereof. Suitable anti-NGAL antibodies for determining the presence or level of NGAL in a sample are available from, *e*.*g*., Santa Cruz Biotechnology, Inc. (Santa Cruz, CA), Thermo Fisher Scientific Inc. (Rockford, IL), and Novus Biologicals (Littleton, CO). In other embodiments, the NGAL binding moiety is an NGAL binding protein such as, for example, MMP-9, megalin, and catecholate-type siderophores.

In certain instances, the presence or level of a particular lipocalin is detected at the level of mRNA expression with an assay such as, for example, a hybridization assay or an amplification-based assay. In certain other instances, the presence or level of a particular lipocalin is detected at the level of protein expression using, for example, an immunoassay *(e.g.,* ELISA) or an immunohistochemical assay. Suitable ELISA kits for determining the presence or level of a lipocalin such as NGAL in a serum, plasma, or urine sample are available from, *e*.*g*., AntibodyShop A/S (Gentofte, Denmark), LabClinics SA (Barcelona, Spain), Lucerna-Chem AG (Luzern, Switzerland), R&D Systems, Inc. (Minneapolis, MN), and Assay Designs, Inc. (Ann Arbor, MI). Suitable ELISA kits for determining the presence or level of the NGAL/MMP-9 complex are available from, *e.g.,* R&D Systems, Inc. Other NGAL and NGAL/MMP-9 complex ELISA techniques are described in, *e.g.,* Kjeldsen et al., Blood, 83:799-807 (1994); and Kjeldsen et al., J. Immunol. Methods, 198:155-164 (1996).

### F. Tissue Inhibitor of Metalloproteinases

In some embodiments, the determination of the presence or level of one or more tissue inhibitor of metalloproteinases in a sample is useful in the present invention. As used herein, the term "tissue inhibitor of metalloproteinase" or "TIMP" includes proteins capable of inhibiting MMPs. In some embodiments, the presence or level of at least one at least one TIMP including, but not limited to, TIMP-1, TIMP-2, TIMP-3,and TIMP-4 is determined in a sample. Exemplary protein and mRNA sequences for TIMP-1 are set forth in GenBank Accession Nos. NP_003245 and NM_003254, respectively.

In particular embodiments, the TIMP-1 binding moiety is an anti-TIMP-1 antibody or a functional fragment thereof. Suitable anti-TIMP-1 antibodies for determining the presence or level of TIMP-1 in a sample are available from, *e*.*g*., Abcam plc (Cambridge, MA), Thermo Fisher Scientific Inc. (Rockford, IL), and Novus Biologicals (Littleton, CO). In other embodiments, the TIMP-1 binding moiety is a TIMP-1 binding protein such as, for example, a matrix metalloproteinase (MMP).

In certain instances, the presence or level of a particular TIMP is detected at the level of mRNA expression with an assay such as, for example, a hybridization assay or an amplification-based assay. In certain other instances, the presence or level of a particular TIMP is detected at the level of protein expression using, for example, an immunoassay (*e*.*g*., ELISA) or an immunohistochemical assay. Suitable ELISA kits for determining the presence or level of a TIMP such as TIMP-1 in a serum or plasma sample are available from, *e.g.,* Alpco Diagnostics (Salem, NH), Calbiochem (San Diego, CA), Invitrogen (Camarillo, CA), CHEMICON International, Inc. (Temecula, CA), and R&D Systems, Inc. (Minneapolis, MN).

### G. Substance P

Substance P is a peptide of 11 amino acids in length (RPKPQQFFGLM-NH₂) that is released by nerve endings in both the central and peripheral nervous systems. Among the numerous biological sites innervated by substance P-releasing neurons are the skin, intestines, stomach, bladder, and cardiovascular system. Substance P is derived from a polypeptide precursor after differential splicing of the preprotachyknin A gene (TAC1; GenBank Accession No. NM_003182). In certain embodiments, substance P or a substance P precursor protein may be useful as a marker of IBS, for example, a TAC1 polypeptide (GenBank Accession Nos. NP_003173; NP_054704; NP_054702; and NP_054703) or a transcript thereof.

In particular embodiments, the substance P binding moiety is an anti-substance P antibody or a functional fragment thereof. Examples of suitable anti-substance P antibodies for determining the presence or level of substance P in a sample are available from, *e.g.,* Abcam plc (Cambridge, MA) and Novus Biologicals (Littleton, CO). In other embodiments, the substance P binding moiety is a substance P binding protein such as, for example, NK1-receptor (neurokinin 1 receptor) or (extracellular) fragments or domains of NK-1 receptor that are capable of specifically binding to substance P.

In certain instances, the presence or level of substance P or precursor thereof is detected at the level of mRNA expression with an assay such as, *e.g.,* a hybridization assay, an amplification-based assay, e.g. qPCR assay, RT-PCR assay, or a mass spectrometry based assay. In certain other instances, the presence or level of substance P or precursor thereof is detected at the level of protein expression using, *e.g.,* an immunoassay *(e.g.,* ELISA), an immunohistochemical assay, or a mass spectrometry based assay. Suitable ELISA kits for determining the presence or level of substance P in a serum, plasma, saliva, or urine sample are available from, *e*.*g*., Cayman Chemical Co. (Ann Arbor, MI), Bachem Holding AG/ Peninsula Laboratories, LLC (San Carlos, CA), and MD Biosciences Inc. (St. Paul, MN).

### H. Serotonin Metabolites

In some embodiments, the determination of the presence or level of one or more serotonin metabolites in a sample is useful in the present invention. As used herein, the term "serotonin metabolite" includes serotonin, serotonin biosynthesis intermediates, and serotonin metabolites. Serotonin is primarily found in the gastrointestinal tract, where it functions to regulate intestinal movements, and to a lesser extent in the central nervous systems, where it participates in the regulation of mood, appetite, sleep, muscle contraction, and various cognitive functions. Non-limiting examples of serotonin metabolites suitable for use as IBS markers include serotonin, tryptophan, 5-HT-o-sulfate, 5-hydroxyindoleacetic acid (5-HIAA), 5-HT glucuronide (5-HT-GA), and/or 5-hydroxytrytophol (5-HTOL).

In particular embodiments, the serotonin metabolite binding moiety is an anti-serotonin metabolite antibody or a functional fragment thereof. Suitable anti-serotonin antibodies for determining the presence or level of serotonin in a sample are available from, *e*.*g*., Abcam plc (Cambridge, MA) and Novus Biologicals (Littleton, CO). In some other embodiments, the serotonin metabolite binding moiety is a serotonin metabolite binding protein or a functional fragment thereof. In certain instances, the serotonin binding moiety is a serotonin binding protein such as, for example, SBP (serotonin binding protein), a 5-HT receptor *(e.g.,* a 5-HT₁, 5-HT₂, 5-HT₃, 5-HT₄, 5-HT₅, 5-HT₆, and/or 5-HT₇ receptor and/or subtypes thereof), and (extracellular) fragments or domains of 5-HT receptors capable of specifically binding to serotonin.

In certain instances, the presence or level of a serotonin metabolite such as serotonin is detected with a mass spectrometry based assay, a proton magnetic resonance spectroscopy based assay, a chromatographic assay (*e*.*g*., liquid chromatographic assay such as HPLC), an immunoassay (*e*.*g*., ELISA), and the like.

### I. Growth Factors

In some embodiments, the determination of the presence or level of one or more growth factors in a sample is useful in the present invention. As used herein, the term "growth factor" includes any of a variety of peptides, polypeptides, or proteins that are capable of stimulating cellular proliferation and/or cellular differentiation.

In certain aspects, the presence or level of at least one growth factor including, but not limited to, epidermal growth factor (EGF), heparin-binding epidermal growth factor (HB-EGF), vascular endothelial growth factor (VEGF), pigment epithelium-derived factor (PEDF; also known as SERPINF1), amphiregulin (AREG; also known as schwannoma-derived growth factor (SDGF)), basic fibroblast growth factor (bFGF), hepatocyte growth factor (HGF), transforming growth factor-α (TGF-α), transforming growth factor-β (TGF-β), bone morphogenetic proteins (*e*.*g*., BMP1-BMP15), platelet-derived growth factor (PDGF), nerve growth factor (NGF), β-nerve growth factor (β-NGF), neurotrophic factors (*e*.*g*., brain-derived neurotrophic factor (BDNF), neurotrophin 3 (NT3), neurotrophin 4 (NT4), *etc.),* growth differentiation factor-9 (GDF-9), granulocyte-colony stimulating factor (G-CSF), granulocyte-macrophage colony stimulating factor (GM-CSF), myostatin (GDF-8), erythropoietin (EPO), and thrombopoietin (TPO) is determined in a sample. Exemplary protein sequences for BDNF are set forth in GenBank Accession Nos. NP_733931, NP_733930, NP_733927, NP_001137282, and NP_001137281. Exemplary mRNA sequences for BDNF are set forth in GenBank Accession Nos. NM_170735, NM_170734, NM_170731, NM_001143810, and NM_001143809.

In particular embodiments, the growth factor binding moiety is an anti-growth factor antibody or a functional fragment thereof. Suitable anti-growth factor antibodies for determining the presence or level of a growth factor such as BDNF are available from, *e.g.,* Novus Biologicals (Littleton, CO) and Abcam plc (Cambridge, MA). In other embodiments, the growth factor binding moiety is a growth factor binding protein such as, for example, an extracellular binding protein such as a receptor or fragment thereof (*e*.*g*., BDNF receptor or growth factor-binding fragments thereof) that specifically binds to a growth factor of interest.

In certain instances, the presence or level of a particular growth factor is detected at the level of mRNA expression with an assay such as, for example, a hybridization assay or an amplification-based assay. In certain other instances, the presence or level of a particular growth factor is detected at the level of protein expression using, for example, an immunoassay (*e*.*g*., ELISA) or an immunohistochemical assay. Suitable ELISA kits for determining the presence or level of a growth factor such as EGF, VEGF, PEDF, SDGF, or BDNF in a serum, plasma, saliva, or urine sample are available from, *e*.*g*., Antigenix America Inc. (Huntington Station, NY), Promega (Madison, WI), R&D Systems, Inc. (Minneapolis, MN), Invitrogen (Camarillo, CA), CHEMICON International, Inc. (Temecula, CA), Neogen Corp. (Lexington, KY), PeproTech (Rocky Hill, NJ), Alpco Diagnostics (Salem, NH), Pierce Biotechnology, Inc. (Rockford, IL), and/or Abazyme (Needham, MA).

### J. Anti-Human Tissue Transglutaminase (tTG)

In some embodiments, the determination of the presence or level of an anti-tissue transglutaminase (tTG) antibody in a sample is useful in the present invention. As used herein, the term "tTG" or "anti-tTG antibody" includes any antibody that recognizes tissue transglutaminase or a fragment thereof. Transglutaminases are a diverse family of Ca²⁺-dependent enzymes that are ubiquitous and highly conserved across species. Of all the transglutaminases, tissue transglutaminase is the most widely distributed. In certain instances, the anti-tTG antibody is an anti-tTG IgA antibody, anti-tTG IgG antibody, or mixtures thereof. In particular embodiments, the binding moiety for anti-tTG is a tissue transglutaminase or an immunoreactive fragment thereof. An ELISA kit available from ScheBo Biotech USA Inc. (Marietta, GA) can be used to detect the presence or level of human anti-tTG IgA antibodies in a sample such as a blood sample.

### K. Anti-Neutrophil Antibodies

In some embodiments, the determination of ANCA levels and/or the presence or absence of pANCA in a sample is useful in the present invention. As used herein, the term "anti-neutrophil cytoplasmic antibody" or "ANCA" includes antibodies directed to cytoplasmic and/or nuclear components of neutrophils. ANCA activity can be divided into several broad categories based upon the ANCA staining pattern in neutrophils: (1) cytoplasmic neutrophil staining without perinuclear highlighting (cANCA); (2) perinuclear staining around the outside edge of the nucleus (pANCA); (3) perinuclear staining around the inside edge of the nucleus (NSNA); and (4) diffuse staining with speckling across the entire neutrophil (SAPPA). In certain instances, pANCA staining is sensitive to DNase treatment. The term ANCA encompasses all varieties of anti-neutrophil reactivity, including, but not limited to, cANCA, pANCA, NSNA, and SAPPA. Similarly, the term ANCA encompasses all immunoglobulin isotypes including, without limitation, immunoglobulin A and G.

ANCA levels in a sample from an individual can be determined, for example, using an immunoassay such as an enzyme-linked immunosorbent assay (ELISA) with alcohol-fixed neutrophils. The presence or absence of a particular category of ANCA such as pANCA can be determined, for example, using an immunohistochemical assay such as an indirect fluorescent antibody (IFA) assay. Preferably, the presence or absence of pANCA in a sample is determined using an immunofluorescence assay with DNase-treated, fixed neutrophils. In addition to fixed neutrophils, antigens specific for ANCA that are suitable for determining ANCA levels include, without limitation, unpurified or partially purified neutrophil extracts; purified proteins, protein fragments, or synthetic peptides such as histone H1 or ANCA-reactive fragments thereof *(see, e.g.,* U.S. Patent No. 6,074,835); histone H1-like antigens, porin antigens, Bacteroides antigens, or ANCA-reactive fragments thereof *(see, e.g.,* U.S. Patent No. 6,033,864); secretory vesicle antigens or ANCA-reactive fragments thereof (*see, e.g.,* U.S. Patent Application No. 08/804,106); and anti-ANCA idiotypic antibodies. One skilled in the art will appreciate that the use of additional antigens specific for ANCA is within the scope of the present invention.

### L. Anti-Saccharomyces cerevisiae Antibodies

In some embodiments, the determination of the presence or level of ASCA *(e.g.,* ASCA-IgA and/or ASCA-IgG) in a sample is useful in the present invention. As used herein, the term "anti-*Saccharomyces cerevisiae* immunoglobulin A" or "ASCA-IgA" includes antibodies of the immunoglobulin A isotype that react specifically with *S. cerevisiae.* Similarly, the term "anti-*Saccharomyces cerevisiae* immunoglobulin G" or "ASCA-IgG" includes antibodies of the immunoglobulin G isotype that react specifically with *S*. *cerevisiae.*

The determination of the presence or level of ASCA-IgA or ASCA-IgG is made using an antigen specific for ASCA. Such an antigen can be any antigen or mixture of antigens that is bound specifically by ASCA-IgA and/or ASCA-IgG. Although ASCA antibodies were initially characterized by their ability to bind *S. cerevisiae,* those of skill in the art will understand that an antigen that is bound specifically by ASCA can be obtained from *S. cerevisiae* or from a variety of other sources so long as the antigen is capable of binding specifically to ASCA antibodies. Accordingly, exemplary sources of an antigen specific for ASCA, which can be used to determine the levels of ASCA-IgA and/or ASCA-IgG in a sample, include, without limitation, whole killed yeast cells such as *Saccharomyces* or *Candida* cells; yeast cell wall mannan such as phosphopeptidomannan (PPM); oligosachharides such as oligomannosides; neoglycolipids; anti-ASCA idiotypic antibodies; and the like. Different species and strains of yeast, such as *S. cerevisiae* strain Su1, Su2, CBS 1315, or BM 156, or *Candida albicans* strain VW32, are suitable for use as an antigen specific for ASCA-IgA and/or ASCA-IgG. Purified and synthetic antigens specific for ASCA are also suitable for use in determining the levels of ASCA-IgA and/or ASCA-IgG in a sample. Examples of purified antigens include, without limitation, purified oligosaccharide antigens such as oligomannosides. Examples of synthetic antigens include, without limitation, synthetic oligomannosides such as those described in U.S. Patent Publication No. 20030105060, *e.g.,* D-Man β(1-2) D-Man β(1-2) D-Man β(1-2) D-Man-OR, D-Man α(1-2) D-Man α(1-2) D-Man α(1-2) D-Man-OR, and D-Man α(1-3) D-Man α(1-2) D-Man α(1-2) D-Man-OR, wherein R is a hydrogen atom, a C₁ to C₂₀ alkyl, or an optionally labeled connector group.

Preparations of yeast cell wall mannans, *e*.*g*., PPM, can be used in determining the levels of ASCA-IgA and/or ASCA-IgG in a sample. Such water-soluble surface antigens can be prepared by any appropriate extraction technique known in the art, including, for example, by autoclaving, or can be obtained commercially *(see, e.g.,* Lindberg et al., Gut, 33:909-913 (1992)). The acid-stable fraction of PPM is also useful in the statistical algorithms of the present invention (Sendid et al., Clin. Diag. Lab. Immunol., 3:219-226 (1996)). An exemplary PPM that is useful in determining ASCA levels in a sample is derived from *S*. *uvarum* strain ATCC #38926.

Purified oligosaccharide antigens such as oligomannosides can also be useful in determining the levels of ASCA-IgA and/or ASCA-IgG in a sample. The purified oligomannoside antigens are preferably converted into neoglycolipids as described in, for example, Faille et al., Eur. J. Microbiol. Infect. Dis., 11:438-446 (1992). One skilled in the art understands that the reactivity of such an oligomannoside antigen with ASCA can be optimized by varying the mannosyl chain length (Frosh et al., Proc Natl. Acad. Sci. USA, 82:1194-1198 (1985)); the anomeric configuration (Fukazawa et al., In "Immunology of Fungal Disease," E. Kurstak (ed.), Marcel Dekker Inc., New York, pp. 37-62 (1989); Nishikawa et al., Microbial. Immunol., 34:825-840 (1990); Poulain et al., Eur. J. Clin. Microbiol., 23:46-52 (1993); Shibata et al., Arch. Biochem. Biophys., 243:338-348 (1985); Trinel et al., Infect. Immun., 60:3845-3851 (1992)); or the position of the linkage (Kikuchi et al., Planta, 190:525-535 (1993)).

Suitable oligomannosides for use in the methods of the present invention include, without limitation, an oligomannoside having the mannotetraose Man(1-3) Man(1-2) Man(1-2) Man. Such an oligomannoside can be purified from PPM as described in, *e.g.,* Faille *et al*., *supra.* An exemplary neoglycolipid specific for ASCA can be constructed by releasing the oligomannoside from its respective PPM and subsequently coupling the released oligomannoside to 4-hexadecylaniline or the like.

### M. Anti-Microbial Antibodies

In some embodiments, the determination of anti-OmpC antibody levels in a sample is useful in the present invention. As used herein, the term "anti-outer membrane protein C antibody" or "anti-OmpC antibody" includes antibodies directed to a bacterial outer membrane porin as described in, *e.g.,* PCT Patent Publication No. WO 01/89361. The term "outer membrane protein C" or "OmpC" refers to a bacterial porin that is immunoreactive with an anti-OmpC antibody.

The level of anti-OmpC antibody present in a sample from an individual can be determined using an OmpC protein or a fragment thereof such as an immunoreactive fragment thereof. Suitable OmpC antigens useful in determining anti-OmpC antibody levels in a sample include, without limitation, an OmpC protein, an OmpC polypeptide having substantially the same amino acid sequence as the OmpC protein, or a fragment thereof such as an immunoreactive fragment thereof. As used herein, an OmpC polypeptide generally describes polypeptides having an amino acid sequence with greater than about 50% identity, preferably greater than about 60% identity, more preferably greater than about 70% identity, still more preferably greater than about 80%, 85%, 90%, 95%, 96%, 97%, 98%, or 99% amino acid sequence identity with an OmpC protein, with the amino acid identity determined using a sequence alignment program such as CLUSTALW. Such antigens can be prepared, for example, by purification from enteric bacteria such as *E. coli*, by recombinant expression of a nucleic acid such as Genbank Accession No. K00541, by synthetic means such as solution or solid phase peptide synthesis, or by using phage display.

In some embodiments, the determination of anti-I2 antibody levels in a sample is useful in the present invention. As used herein, the term "anti-I2 antibody" includes antibodies directed to a microbial antigen sharing homology to bacterial transcriptional regulators as described in, *e.g.,* U.S. Patent No. 6,309,643. The term "12" refers to a microbial antigen that is immunoreactive with an anti-I2 antibody. The microbial 12 protein is a polypeptide of 100 amino acids sharing some similarity weak homology with the predicted protein 4 from C. *pasteurianum*, Rv3557c from *Mycobacterium tuberculosis*, and a transcriptional regulator from *Aquifex aeolicus.* The nucleic acid and protein sequences for the I2 protein are described in, *e.g.,* U.S. Patent No. 6,309,643.

The level of anti-I2 antibody present in a sample from an individual can be determined using an 12 protein or a fragment thereof such as an immunoreactive fragment thereof. Suitable 12 antigens useful in determining anti-I2 antibody levels in a sample include, without limitation, an I2 protein, an I2 polypeptide having substantially the same amino acid sequence as the I2 protein, or a fragment thereof such as an immunoreactive fragment thereof. Such I2 polypeptides exhibit greater sequence similarity to the I2 protein than to the *C*. *pasteurianum* protein 4 and include isotype variants and homologs thereof. As used herein, an I2 polypeptide generally describes polypeptides having an amino acid sequence with greater than about 50% identity, preferably greater than about 60% identity, more preferably greater than about 70% identity, still more preferably greater than about 80%, 85%, 90%, 95%, 96%, 97%, 98%, or 99% amino acid sequence identity with a naturally-occurring I2 protein, with the amino acid identity determined using a sequence alignment program such as CLUSTALW. Such I2 antigens can be prepared, for example, by purification from microbes, by recombinant expression of a nucleic acid encoding an I2 antigen, by synthetic means such as solution or solid phase peptide synthesis, or by using phage display.

In some embodiments, the determination of anti-flagellin antibody levels in a sample is also useful in the present invention. As used herein, the term "anti-flagellin antibody" includes antibodies directed to a protein component of bacterial flagella as described in, *e.g.,* PCT Patent Publication No. WO 03/053220 and U.S. Patent Publication No. 20040043931. The term "flagellin" refers to a bacterial flagellum protein that is immunoreactive with an anti-flagellin antibody. Microbial flagellins are proteins found in bacterial flagellum that arrange themselves in a hollow cylinder to form the filament.

The level of anti-flagellin antibody present in a sample from an individual can be determined using a flagellin protein or a fragment thereof such as an immunoreactive fragment thereof. Suitable flagellin antigens useful in determining anti-flagellin antibody levels in a sample include, without limitation, a flagellin protein such as CBir-1 flagellin, flagellin X, flagellin A, flagellin B, fragments thereof, and combinations thereof, a flagellin polypeptide having substantially the same amino acid sequence as the flagellin protein, or a fragment thereof such as an immunoreactive fragment thereof. As used herein, a flagellin polypeptide generally describes polypeptides having an amino acid sequence with greater than about 50% identity, preferably greater than about 60% identity, more preferably greater than about 70% identity, still more preferably greater than about 80%, 85%, 90%, 95%, 96%, 97%, 98%, or 99% amino acid sequence identity with a naturally-occurring flagellin protein, with the amino acid identity determined using a sequence alignment program such as CLUSTALW. Such flagellin antigens can be prepared, *e.g.,* by purification from bacterium such as *Helicobacter Bilis*, *Helicobacter mustelae*, *Helicobacter pylori*, *Butyrivibrio fibrisolvens*, and bacterium found in the cecum, by recombinant expression of a nucleic acid encoding a flagellin antigen, by synthetic means such as solution or solid phase peptide synthesis, or by using phage display. In particular embodiments, the presence or level of anti-CBir-1 antibodies are determined in a sample.

### N. CCDC147 coiled-coil domain containing 147 (CCDC147)

CCDC147 is a 104 kDa protein (GenBank Accession No. NP_001008723) encoded by the CCDC147 gene (GenBank Accession No. NM_001008723). In certain embodiments, CCDC147 and/or an mRNA encoding CCDC147 are useful biomarkers for IBS.

In particular embodiments, the CCDC147 detection reagent is a nucleic acid such as an oligonucleotide or a polynucleotide that specifically hybridizes to an mRNA that encodes CCDC147, and can optionally comprise reporter moieties or labels. In some instances, the CCDC147 detection reagent is an oligonucleotide probe.

In certain instances, the presence or level of CCDC147, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of CCDC147, a precursor thereof, or an isoform thereof is detected at the level of protein expression using, *e.g.,* an immunoassay *(e.g.,* ELISA), an immunohistochemical assay, or a mass spectrometry based assay.

### O. Vasoactive Intestinal Peptide Receptor 1 (VIPR1)

VIPR1 is a 7-transmembrane domain neuropeptide receptor that interacts with the vasoative intestinal peptide (VIP). VIPR1 is a 48.5 kDa transmembrane protein encoded by the vasoactive intestinal peptide receptor 1 gene (GenBank Accession No. NM_004624) and is produced after processing of the VIPR1 precursor polypeptide (GenBank Accession No. NP_004615). In certain embodiments, VIPR1, a VIPR1 precursor protein, and/or an mRNA encoding VIPR1 are useful biomarkers for IBS.

In particular embodiments, the VIPR1 detection reagent is a nucleic acid such as an oligonucleotide or a polynucleotide that specifically hybridizes to an mRNA that encodes VIPR1, and can optionally comprise reporter moieties or labels. In some instances, the VIPR1 detection reagent is an oligonucleotide probe.

In certain embodiments, the presence or level of VIPR1, a precursor thereof, or a variant thereof is detected at the level of mRNA expression with an assay such as, *e.g.,* a hybridization assay, an amplification-based assay, e.g. qPCR assay, RT-PCR assay, or a mass spectrometry based assay. In certain other instances, the presence or level of VIPR1, a precursor thereof, or an isoform thereof is detected at the level of protein expression using, *e.g.,* an immunoassay *(e.g.,* ELISA), an immunohistochemical assay, or a mass spectrometry based assay. Suitable ELISA kits for determining the presence or level of VIPR1 in a serum, plasma, saliva, or urine sample are available from, *e*.*g*., Sigma-Aldrich (St. Louis, MO), US Biological (Swampscott, MA), and Novus Biologicals (Littleton, CO).

### P. CBFA2T2

CBFA2T2 (core-binding factor, runt domain, alpha subunit 2; translocated to, 2) is a protein (GenBank Accession Nos. NP_001028171, NP_001034798, and NP_005084 for various isoforms) encoded by the CBFA2T2 gene (GenBank Accession Nos. NM_005093, NM_001032999, and NM_001039709 for transcript variants). In certain embodiments, CBFA2T2 and/or an mRNA encoding CBFA2T2 are useful biomarkers for IBS.

In particular embodiments, the CBFA2T2 detection reagent is a nucleic acid such as an oligonucleotide or a polynucleotide that specifically hybridizes to an mRNA that encodes CBFA2T2, and can optionally comprise reporter moieties or labels. In some instances, the CBFA2T2 detection reagent is an oligonucleotide probe.

In certain instances, the presence or level of CBFA2T2, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of CBFA2T2, a precursor thereof, or an isoform thereof is detected at the level of protein expression using, *e.g.,* an immunoassay *(e.g.,* ELISA), an immunohistochemical assay, or a mass spectrometry based assay.

### Q. HSD17B11

HSD17B11 (hydroxysteroid (17-beta) dehydrogenase 11) is a protein (GenBank Accession No. NP_057329.2) encoded by the HSD17B11 gene (GenBank Accession No. NM_016245). In certain embodiments, HSD17B11 and/or an mRNA encoding HSD17B11 are useful biomarkers for IBS.

In particular embodiments, the HSD17B11 detection reagent is a nucleic acid such as an oligonucleotide or a polynucleotide that specifically hybridizes to an mRNA encoding HSD17B11, and can optionally comprise reporter moieties or labels. In some instances, the HSD17B11 detection reagent is an oligonucleotide probe.

In certain instances, the presence or level of HSD17B11, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of HSD17B11, a precursor thereof, or an isoform thereof is detected at the level of protein expression using, *e.g.,* an immunoassay *(e.g.,* ELISA), an immunohistochemical assay, or a mass spectrometry based assay.

### R. LDLR

LDLR (low density lipoprotein receptor) is a protein (GenBank Accession Nos. NP_001182732, NP_001182731, NP_001182729, NP_001182728, NP_001182727, NP_000518 for various isoforms) encoded by the LDLR gene (GenBank Accession Nos. NM_000527, M_001195798, NM_001195799, NM_001195800, M_001195802, and NM_001195803 for transcript variants). In certain embodiments, LDLR and/or an mRNA encoding LDLR are useful biomarkers for IBS.

In particular embodiments, the LDLR detection reagent is a nucleic acid such as an oligonucleotide or a polynucleotide that specifically hybridizes to an mRNA that encodes LDLR, and can optionally comprise reporter moieties or labels. In some embodiments, the LDLR detection reagent is an oligonucleotide probe.

In certain embodiments, the presence or level of LDLR, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of LDLR, a precursor thereof, or an isoform thereof is detected at the level of protein expression using, *e.g.,* an immunoassay *(e.g.,* ELISA), an immunohistochemical assay, or a mass spectrometry based assay.

### S. MAP6D1

MAP6D1 (MAP6 domain containing 1) is a protein (GenBank Accession No. NP_079147) encoded by the MAP6D1 gene (GenBank Accession No. NM_024871). In certain embodiments, MAP6D1 and/or an mRNA encoding MAP6D1 are useful biomarkers for IBS.

In particular embodiments, the MAP6D1 detection reagent is a nucleic acid such as an oligonucleotide or a polynucleotide that specifically hybridizes to an mRNA that encodes MAP6D1, and can optionally comprise reporter moieties or labels. In some embodiments, the MAP6D1 detection reagent is an oligonucleotide probe.

In certain embodiments, the presence or level of MAP6D1, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of MAP6D1, a precursor thereof, or an isoform thereof is detected at the level of protein expression using, *e.g.,* an immunoassay *(e.g.,* ELISA), an immunohistochemical assay, or a mass spectrometry based assay.

### T. MICALL1

MICALL1 (MICAL-like 1) is a protein (GenBank Accession No. NP_203744) encoded by the MICALL1 gene (GenBank Accession No. NM_033386). In some instances, MICALL1 and/or an mRNA encoding MICALL1 are useful biomarkers for IBS.

In particular embodiments, the MICALL1 detection reagent is a nucleic acid such as an oligonucleotide or a polynucleotide that specifically hybridizes to an mRNA that encodes MICALL1, and can optionally comprise reporter moieties or labels. In some embodiments, the MICALL1 detection reagent is an oligonucleotide probe.

In certain embodiments, the presence or level of MICALL1, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of MICALL1, a precursor thereof, or an isoform thereof is detected at the level of protein expression using, *e.g.,* an immunoassay *(e.g.,* ELISA), an immunohistochemical assay, or a mass spectrometry based assay.

### U. RAB7L1

RAB7L1 (RAB7, member RAS oncogene family-like 1) is a protein (GenBank Accession Nos. NP_001129134, NP_001129135, NP_001129136, and NP_003920 for various isoforms) encoded by the RAB7L1 gene (GenBank Accession Nos. NM_001135662, NM_001135663, NM_001135664, and NM_003929 for transcript variants). In certain embodiments, RAB7L1 and/or an mRNA encoding RAB7L1 are useful biomarkers for IBS.

In particular embodiments, the RAB7L1 detection reagent is a nucleic acid such as an oligonucleotide or a polynucleotide that specifically hybridizes to an mRNA that encodes RAB7L1, and can optionally comprise reporter moieties or labels. In some embodiments, the RAB7L1 detection reagent is an oligonucleotide probe.

In certain embodiments, the presence or level of RAB7L1, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of RAB7L1, a precursor thereof, or an isoform thereof is detected at the level of protein expression using, *e.g.,* an immunoassay *(e.g.,* ELISA), an immunohistochemical assay, or a mass spectrometry based assay.

### V. RNF26

RNF26 (ring finger protein 26) is a protein (GenBank Accession No. NP_114404) encoded by the RNF26 gene (GenBank Accession No. NM_032015). In some embodiments, RNF26 and/or an mRNA encoding RNF26 are useful biomarkers for IBS.

In particular embodiments, the RNF26 detection reagent is a nucleic acid such as an oligonucleotide or a polynucleotide that specifically hybridizes to an mRNA that encodes RNF26, and can optionally comprise reporter moieties or labels. In some embodiments, the RNF26 detection reagent is an oligonucleotide probe.

In certain embodiments, the presence or level of RNF26, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of RNF26, a precursor thereof, or an isoform thereof is detected at the level of protein expression using, *e.g.,* an immunoassay *(e.g.,* ELISA), an immunohistochemical assay, or a mass spectrometry based assay.

### W. RRP7A

RRP7A (ribosomal RNA processing 7 homo log A) is a protein (GenBank Accession No. NP_056518) encoded by the RRP7Agene (GenBank Accession No. NM_015703). In some embodiments, RRP7A and/or an mRNA encoding RRP7A are useful biomarkers for IBS.

In particular embodiments, the RRP7A detection reagent is a nucleic acid such as an oligonucleotide or a polynucleotide that specifically hybridizes to an mRNA that encodes RRP7A, and can optionally comprise reporter moieties or labels. In some embodiments, the RRP7A detection reagent is an oligonucleotide probe.

In certain embodiments, the presence or level of RRP7A, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of RRP7A, a precursor thereof, or an isoform thereof is detected at the level of protein expression using, *e.g.,* an immunoassay *(e.g.,* ELISA), an immunohistochemical assay, or a mass spectrometry based assay.

### X. SUSD4

SUSD4 (sushi domain containing 4) is a protein (GenBank Accession Nos. NP_001032252 and NP_060452 for various isoforms) encoded by the SUSD4 gene (GenBank Accession Nos. NM_001037175 and NM_017982 for transcript variants). In certain embodiments, SUSD4 and/or an mRNA encoding SUSD4 are useful biomarkers for IBS.

In particular embodiments, the SUSD4 detection reagent is a nucleic acid such as an oligonucleotide or a polynucleotide that specifically hybridizes to an mRNA that encodes SUSD4, and can optionally comprise reporter moieties or labels. In some embodiments, the SUSD4 detection reagent is an oligonucleotide probe.

In certain embodiments, the presence or level of SUSD4, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of SUSD4, a precursor thereof, or an isoform thereof is detected at the level of protein expression using, *e.g.,* an immunoassay *(e.g.,* ELISA), an immunohistochemical assay, or a mass spectrometry based assay.

### Y. SH3BGRL3

SH3BGRL3 (SH3 domain binding glutamic acid-rich protein like 3) is a protein (GenBank Accession No. NP_112576) encoded by the SH3BGRL3 gene (GenBank Accession No. NM_031286). In some embodiments, SH3BGRL3 and/or an mRNA encoding SH3BGRL3 are useful biomarkers for IBS.

In particular embodiments, the SH3BGRL3 detection reagent is a nucleic acid such as an oligonucleotide or a polynucleotide that specifically hybridizes to an mRNA encoding SH3BGRL3, and can optionally comprise reporter moieties or labels. In some embodiments, the SH3BGRL3 detection reagent is an oligonucleotide probe.

In certain embodiments, the presence or level of SH3BGRL3, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other instances, the presence or level of SH3BGRL3, a precursor thereof, or an isoform thereof is detected at the level of protein expression using, *e.g.,* an immunoassay *(e.g.,* ELISA), an immunohistochemical assay, or a mass spectrometry based assay.

### Z. WEE1

WEE1 (WEE1 homolog) is a protein (GenBank Accession Nos. NP_001137448 and NP_003381 for various isoforms) encoded by the WEE1 gene (GenBank Accession Nos. NM_001143976 and NM_003390 for transcript variants). In certain instances, WEE1 and/or an mRNA encoding WEE1 are useful biomarkers for IBS.

In particular embodiments, the WEE1 detection reagent is a nucleic acid such as an oligonucleotide or a polynucleotide that specifically hybridizes to an mRNA that encodes WEE1, and can optionally comprise reporter moieties or labels. In some embodiments, the WEE1 detection reagent is an oligonucleotide probe.

In certain embodiments, the presence or level of WEE1, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other embodiments, the presence or level of WEE1, a precursor thereof, or an isoform thereof is detected at the level of protein expression using, *e.g.,* an immunoassay *(e.g.,* ELISA), an immunohistochemical assay, or a mass spectrometry based assay.

### AA. ZNF326

ZNF326 (zinc finger protein 326) is a protein (GenBank Accession Nos. NP_892020 and NP_892021 for various isoforms) encoded by the ZNF326 gene (GenBank Accession Nos. NM_182975 and NM_182976 for transcript variants). In certain instances, ZNF326 and/or an mRNA encoding ZNF326 are useful biomarkers for IBS.

In particular embodiments, the ZNF326 detection reagent is a nucleic acid such as an oligonucleotide or a polynucleotide that specifically hybridizes to an mRNA that encodes ZNF326, and can optionally comprise reporter moieties or labels. In some embodiments, the ZNF326 detection reagent is an oligonucleotide probe.

In certain embodiments, the presence or level of ZNF326, a precursor thereof, or a variant thereof is detected at the level of mRNA expression with an assay such as, *e.g.,* a hybridization assay, an amplification-based assay, *e*.*g*. qPCR assay, RT-PCR assay, or a mass spectrometry based assay. In certain other embodiments, the presence or level of ZNF326, a precursor thereof, or an isoform thereof is detected at the level of protein expression using, *e.g.,* an immunoassay *(e.g.,* ELISA), an immunohistochemical assay, or a mass spectrometry based assay.

### V. Assays

Any of a variety of assays, techniques, and kits known in the art can be used to determine the presence, (concentration) level, and/or gene expression level of one or more IBS biomarkers in a sample.

The present invention relies, in part, on determining the presence or level of at least one marker in a sample obtained from a subject. As used herein, the term "determining the presence of at least one marker" includes determining the presence of each marker of interest by using any quantitative or qualitative assay known to one of skill in the art. In certain instances, qualitative assays that determine the presence or absence of a particular trait, variable, or biochemical or serological substance (*e*.*g*., RNA, mRNA, miRNA, protein, or antibody) are suitable for detecting each marker of interest. In certain other instances, quantitative assays that determine the presence or absence of RNA, protein, antibody, or activity are suitable for detecting each marker of interest. The term "determining the level of at least one marker" includes determining the level of each marker of interest by using any direct or indirect quantitative assay known to one of skill in the art. In certain instances, quantitative assays that determine, for example, the relative or absolute amount of RNA, mRNA, miRNA, protein, antibody, or activity are suitable for determining the level of each marker of interest. One skilled in the art will appreciate that any assay useful for determining the level of a marker is also useful for determining the presence or absence of the marker.

Analysis of marker mRNA levels using routine techniques such as Northern analysis, reverse-transcriptase polymerase chain reaction (*e*.*g*., qRT-PCR, RT-PCR), microarray analysis, Luminex MultiAnalyte Profiling (xMAP) technology or any other methods based on hybridization to a nucleic acid sequence that is complementary to a portion of the marker coding sequence (*e*.*g*., slot blot hybridization) are within the scope of the present invention. Applicable PCR amplification techniques are described in, *e*.*g*., Ausubel et al., Current Protocols in Molecular Biology, John Wiley & Sons, Inc. New York (1999), Chapter 7 and Supplement 47; Theophilus et al., "PCR Mutation Detection Protocols," Humana Press, (2002); and Innis et al., PCR Protocols, San Diego, Academic Press, Inc. (1990). General nucleic acid hybridization methods are described in Anderson, "Nucleic Acid Hybridization," BIOS Scientific Publishers, 1999. Amplification or hybridization of a plurality of transcribed nucleic acid sequences (*e*.*g*., mRNA or cDNA) can also be performed from mRNA or cDNA sequences arranged in a microarray. Microarray methods are generally described in Hardiman, "Microarrays Methods and Applications: Nuts & Bolts," DNA Press, 2003; and Baldi et al., "DNA Microarrays and Gene Expression: From Experiments to Data Analysis and Modeling," Cambridge University Press, 2002.

Analysis of the genotype of a marker such as a genetic marker can be performed using techniques known in the art including, without limitation, polymerase chain reaction (PCR)-based analysis, sequence analysis, and electrophoretic analysis. A non-limiting example of a PCR-based analysis includes a Taqman^{®} allelic discrimination assay available from Applied Biosystems. Non-limiting examples of sequence analysis include Maxam-Gilbert sequencing, Sanger sequencing, capillary array DNA sequencing, thermal cycle sequencing (Sears et al., Biotechniques, 13:626-633 (1992)), solid-phase sequencing (Zimmerman et al., Methods Mol. Cell Biol., 3:39-42 (1992)), sequencing with mass spectrometry such as matrix-assisted laser desorption/ionization time-of-flight mass spectrometry (MALDI-TOF/MS; Fu et al., Nature Biotech., 16:381-384 (1998)), and sequencing by hybridization (Chee et al., Science, 274:610-614 (1996); Drmanac et al., Science, 260:1649-1652 (1993); Drmanac et al., Nature Biotech., 16:54-58 (1998)). Non-limiting examples of electrophoretic analysis include slab gel electrophoresis such as agarose or polyacrylamide gel electrophoresis, capillary electrophoresis, and denaturing gradient gel electrophoresis. Other methods for genotyping an individual at a polymorphic site in a marker include, *e.g.,* the INVADER^{®} assay from Third Wave Technologies, Inc., restriction fragment length polymorphism (RFLP) analysis, allele-specific oligonucleotide hybridization, a heteroduplex mobility assay, and single strand conformational polymorphism (SSCP) analysis.

As used herein, the term "antibody" includes a population of immunoglobulin molecules, which can be polyclonal or monoclonal and of any isotype, or an immunologically active fragment of an immunoglobulin molecule. Such an immunologically active fragment contains the heavy and light chain variable regions, which make up the portion of the antibody molecule that specifically binds an antigen. For example, an immunologically active fragment of an immunoglobulin molecule known in the art as Fab, Fab' or F(ab')₂ is included within the meaning of the term antibody.

Flow cytometry can be used to determine the presence or level of one or more markers in a sample. Such flow cytometric assays, including bead based immunoassays, can be used to determine, *e*.*g*., antibody marker levels in the same manner as described for detecting serum antibodies to *Candida albicans* and HIV proteins *(see, e.g.,* Bishop and Davis, J. Immunol. Methods, 210:79-87 (1997); McHugh et al., J. Immunol. Methods, 116:213 (1989); Scillian et al., Blood, 73:2041 (1989)).

Phage display technology for expressing a recombinant antigen specific for a marker can be used to determine the presence or level of one or more markers in a sample. Phage particles expressing an antigen specific for, *e.g.,* an antibody marker can be anchored, if desired, to a multi-well plate using an antibody such as an anti-phage monoclonal antibody (Felici et al., "Phage-Displayed Peptides as Tools for Characterization of Human Sera" in Abelson (Ed.), Methods in Enzymol., 267, San Diego: Academic Press, Inc. (1996)).

A variety of immunoassay techniques, including competitive and non-competitive immunoassays, can be used to determine the presence or level of one or more markers in a sample *(see, e.g.,* Self and Cook, Curr. Opin. Biotechnol., 7:60-65 (1996)). The term immunoassay encompasses techniques including, without limitation, enzyme immunoassays (EIA) such as enzyme multiplied immunoassay technique (EMIT), enzyme-linked immunosorbent assay (ELISA), antigen capture ELISA, sandwich ELISA, IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); and chemiluminescence assays (CL). If desired, such immunoassays can be automated. Immunoassays can also be used in conjunction with laser induced fluorescence *(see, e.g.,* Schmalzing and Nashabeh, Electrophoresis, 18:2184-2193 (1997); Bao, J. Chromatogr. B. Biomed. Sci., 699:463-480 (1997)). Liposome immunoassays, such as flow-injection liposome immunoassays and liposome immunosensors, are also suitable for use in the present invention *(see, e.g.,* Rongen et al., J. Immunol. Methods, 204:105-133 (1997)). In addition, nephelometry assays, in which the formation of protein/antibody complexes results in increased light scatter that is converted to a peak rate signal as a function of the marker concentration, are suitable for use in the present invention. Nephelometry assays are commercially available from Beckman Coulter (Brea, CA; Kit #449430) and can be performed using a Behring Nephelometer Analyzer (Fink et al., J. Clin. Chem. Clin. Biol. Chem., 27:261-276 (1989)).

Antigen capture ELISA can be useful for determining the presence or level of one or more markers in a sample. For example, in an antigen capture ELISA, an antibody directed to a marker of interest is bound to a solid phase and sample is added such that the marker is bound by the antibody. After unbound proteins are removed by washing, the amount of bound marker can be quantitated using, *e.g.,* a radioimmunoassay (*see, e.g.,* Harlow and Lane, Antibodies: A Laboratory Manual, Cold Spring Harbor Laboratory, New York, 1988)). Sandwich ELISA can also be suitable for use in the present invention. For example, in a two-antibody sandwich assay, a first antibody is bound to a solid support, and the marker of interest is allowed to bind to the first antibody. The amount of the marker is quantitated by measuring the amount of a second antibody that binds the marker. The antibodies can be immobilized onto a variety of solid supports, such as magnetic or chromatographic matrix particles, the surface of an assay plate (*e*.*g*., microtiter wells), pieces of a solid substrate material or membrane (*e*.*g*., plastic, nylon, paper), and the like. An assay strip can be prepared by coating the antibody or a plurality of antibodies in an array on a solid support. This strip can then be dipped into the test sample and processed quickly through washes and detection steps to generate a measurable signal, such as a colored spot.

A radioimmunoassay using, for example, an iodine-125 (¹²⁵I) labeled secondary antibody (Harlow and Lane, *supra*) is also suitable for determining the presence or level of one or more markers in a sample. A secondary antibody labeled with a chemiluminescent marker can also be suitable for use in the present invention. A chemiluminescence assay using a chemiluminescent secondary antibody is suitable for sensitive, non-radioactive detection of marker levels. Such secondary antibodies can be obtained commercially from various sources, *e*.*g*., Amersham Lifesciences, Inc. (Arlington Heights, IL).

The immunoassays described herein are particularly useful for determining the presence or level of one or more IBS markers in a sample. As a non-limiting example, an ELISA using a binding molecule for a cytokine of interest such as TNF-α, TWEAK, IL-1β, IL-6, IL-8, IL-10, IL-12 (*e.g.,* IL-12A and/or IL-12B), and/or GRO-α *(e.g.,* antibodies that specifically bind to one of these cytokines and/or extracellular binding proteins including receptors that specifically bind to one of these cytokines or cytokine-binding fragments thereof) is useful for determining whether a sample is positive for the cytokine of interest or for determining protein levels of that particular cytokine in a sample. A fixed neutrophil ELISA is useful for determining whether a sample is positive for ANCA or for determining ANCA levels in a sample. Similarly, an ELISA using yeast cell wall phosphopeptidomannan is useful for determining whether a sample is positive for ASCA-IgA and/or ASCA-IgG, or for determining ASCA-IgA and/or ASCA-IgG levels in a sample. An ELISA using flagellin protein (*e*.*g*., CBir1 flagellin) or a fragment thereof is useful for determining whether a sample is positive for anti-flagellin antibodies (*e*.*g*., anti-CBir1), or for determining anti-flagellin antibody (*e*.*g*., anti-CBir1) levels in a sample. In addition, the immunoassays described above are particularly useful for determining the presence or level of other IBS markers in a sample.

Specific immunological binding of the antibody to the marker of interest can be detected directly or indirectly. Direct labels include fluorescent or luminescent tags, metals, dyes, radionuclides, and the like, attached to the antibody. An antibody labeled with iodine-125 (¹²⁵I) can be used for determining the levels of one or more markers in a sample. A chemiluminescence assay using a chemiluminescent antibody specific for the marker is suitable for sensitive, non-radioactive detection of marker levels. An antibody labeled with fluorochrome is also suitable for determining the levels of one or more markers in a sample. Examples of fluorochromes include, without limitation, DAPI, fluorescein, Hoechst 33258, R-phycocyanin, B-phycoerythrin, R-phycoerythrin, rhodamine, Texas red, and lissamine. Secondary antibodies linked to fluorochromes can be obtained commercially, *e*.*g*., goat F(ab')₂ anti-human IgG-FITC is available from Tago Immunologicals (Burlingame, CA).

Indirect labels include various enzymes well-known in the art, such as horseradish peroxidase (HRP), alkaline phosphatase (AP), β-galactosidase, urease, and the like. A horseradish-peroxidase detection system can be used, for example, with the chromogenic substrate tetramethylbenzidine (TMB), which yields a soluble product in the presence of hydrogen peroxide that is detectable at 450 nm. An alkaline phosphatase detection system can be used with the chromogenic substrate p-nitrophenyl phosphate, for example, which yields a soluble product readily detectable at 405 nm. Similarly, a β-galactosidase detection system can be used with the chromogenic substrate o-nitrophenyl-β-D-galactopyranoside (ONPG), which yields a soluble product detectable at 410 nm. An urease detection system can be used with a substrate such as urea-bromocresol purple (Sigma Immunochemicals; St. Louis, MO). A useful secondary antibody linked to an enzyme can be obtained from a number of commercial sources, *e*.*g*., goat F(ab')₂ anti-human IgG-alkaline phosphatase can be purchased from Jackson ImmunoResearch (West Grove, PA.).

A signal from the direct or indirect label can be analyzed, for example, using a spectrophotometer to detect color from a chromogenic substrate; a radiation counter to detect radiation such as a gamma counter for detection of ¹²⁵I; or a fluorometer to detect fluorescence in the presence of light of a certain wavelength. For detection of enzyme-linked antibodies, a quantitative analysis of the amount of marker levels can be made using a spectrophotometer such as an EMAX Microplate Reader (Molecular Devices; Menlo Park, CA) in accordance with the manufacturer's instructions. If desired, the assays of the present invention can be automated or performed robotically, and the signal from multiple samples can be detected simultaneously.

As a non-limiting example, the immunoassays for the detection of an IBS marker in a sample such as a whole blood or serum sample can comprise: (a) coating a solid phase surface with a first anti-IBS marker capture antibody; (b) contacting the solid phase surface with a sample under conditions suitable to transform the IBS marker present in the sample into a complex comprising the IBS marker and the anti-IBS marker capture antibody; (c) contacting the IBS marker and the anti-IBS maker complex with a second detecting antibody under conditions suitable to form a ternary complex; and (d) contacting the ternary complex with a luminescent or chemiluminescent substrate.

In certain instances, the detecting antibody is conjugated to alkaline phosphatase. In other instances, the detecting antibody is not conjugated to an enzyme and the method further comprises: (i) contacting the ternary complex with a third antibody conjugated to alkaline phosphatase under conditions suitable to form a quaternary complex; and (ii) contacting the quaternary complex with a luminescent or chemiluminescent substrate.

Any suitable antibody pair may be used for the capture and detection of antibodies in a sandwich ELISA. One of skill in the art will know and appreciate how to select an appropriate antibody pair for the assay. Generally, two antibodies are selected that bind to the target of interest, *e*.*g*., the IBS marker, at different epitopes such that the binding of the first (capture) antibody does not interfere with the second (detecting) antibody. In certain embodiments, the detecting antibody will be conjugated to an enzyme, for example, alkaline phophatase, to aid in the detection of the complex. In other embodiments, a secondary antibody conjugated to an enzyme (*e*.*g*., alkaline phophatase) which binds to the detecting antibody may be used in the assay.

Generally, the complex will be detected by the use of a luminescent substrate, for example, a luminescent substrate found in a kit such as Ultra LITE™ (NAG Research Laboratories); SensoLyte® (AnaSpec); SuperSignal ELISA Femto Maximum Sensitivity Substrate (Thermo Scientific); SuperSignal ELISA Pico Chemiluminescent Substrate (Thermo Scientific); or CPSD (disodium 3-(4-methoxyspiro{1,2-dioxetane-3,2'-(5'-chloro)tricyclo[3.3.1.13,7]decan}-4-yl)phenyl phosphate; Tropix, Inc).

In particular embodiments, an assay for detecting the presence or level of an IBS marker comprises a sandwich ELISA that relies on the use of an alkaline phosphatase-conjugated anti-IBS marker antibody as the detecting antibody and a CPSD-containing luminescent substrate to enhance the assay sensitivity. The CPSD substrate can be found in chemiluminescent detection systems, such as, *e.g.,* the ELISA-Light™ System (Applied Biosystems).

In certain instances, the detection limit of the IBS marker present in a sample such as a whole blood or serum sample is less than about 500 pg/ml. In certain embodiments, the detection limit of the IBS marker present in a sample is less than about 500 pg/ml, or less than about 400 pg/ml, 300 pg/ml, 250 pg/ml, 200 pg/ml, 150 pg/ml, 100 pg/ml, 75 pg/ml, 50 pg/ml, 40 pg/ml, 30 pg/ml, 25 pg/ml, 20 pg/ml, 15 pg/ml, or 10 pg/ml.

As another non-limiting example, the immunoassays for the detection of an IBS marker in a sample such as a whole blood or serum sample can comprise: (a) contacting a sample having an IBS marker under conditions suitable to transform the IBS marker into a complex comprising the IBS marker and a capture anti-IBS marker antibody; (b) contacting the complex with an enzyme-labeled indicator antibody to transform the complex into a labeled complex; (c) contacting the labeled complex with a substrate for the enzyme; and (d) detecting the presence or level of the IBS marker in the sample.

In particular embodiments, the immunoassay is an enzyme-linked immunosorbent assay (ELISA). In some instances, detecting the presence or level of the IBS marker in the sample comprises the use of a detection device such as, e.g., a luminescence plate reader or spectrophotometer.

Quantitative western blotting can also be used to detect or determine the presence or level of one or more markers in a sample. Western blots can be quantitated by well-known methods such as scanning densitometry or phosphorimaging. As a non-limiting example, protein samples are electrophoresed on 10% SDS-PAGE Laemmli gels. Primary murine monoclonal antibodies are reacted with the blot, and antibody binding can be confirmed to be linear using a preliminary slot blot experiment. Goat anti-mouse horseradish peroxidase-coupled antibodies (BioRad) are used as the secondary antibody, and signal detection performed using chemiluminescence, for example, with the Renaissance chemiluminescence kit (New England Nuclear; Boston, MA) according to the manufacturer's instructions. Autoradiographs of the blots are analyzed using a scanning densitometer (Molecular Dynamics; Sunnyvale, CA) and normalized to a positive control. Values are reported, for example, as a ratio between the actual value to the positive control (densitometric index). Such methods are well known in the art as described, for example, in Parra et al., J. Vasc. Surg., 28:669-675 (1998).

Alternatively, any of a variety of immunohistochemical assay techniques can be used to determine the presence or level of one or more markers in a sample. The term immunohistochemical assay encompasses techniques that utilize the visual detection of fluorescent dyes or enzymes coupled (*i.e.,* conjugated) to antibodies that react with the marker of interest using fluorescent microscopy or light microscopy and includes, without limitation, direct fluorescent antibody assay, indirect fluorescent antibody (IFA) assay, anticomplement immunofluorescence, avidin-biotin immunofluorescence, and immunoperoxidase assays. An IFA assay, for example, is useful for determining whether a sample is positive for ANCA, the level of ANCA in a sample, whether a sample is positive for pANCA, the level of pANCA in a sample, and/or an ANCA staining pattern (*e*.*g*., cANCA, pANCA, NSNA, and/or SAPPA staining pattern). The concentration of ANCA in a sample can be quantitated, *e*.*g*., through endpoint titration or through measuring the visual intensity of fluorescence compared to a known reference standard.

Alternatively, the presence or level of a marker of interest can be determined by detecting or quantifying the amount of the purified marker. Purification of the marker can be achieved, for example, by high pressure liquid chromatography (HPLC), alone or in combination with mass spectrometry (*e*.*g*., MALDI/MS, MALDI-TOF/MS, SELDI-TOF/MS, tandem MS, *etc.* ). Qualitative or quantitative detection of a marker of interest can also be determined by well-known methods including, without limitation, Bradford assays, Coomassie blue staining, silver staining, assays for radiolabeled protein, and mass spectrometry.

In certain embodiments, the analysis of a plurality of markers may be carried out separately or simultaneously with one test sample. For separate or sequential assay of markers, suitable apparatuses include clinical laboratory analyzers such as the ElecSys (Roche), the AxSym (Abbott), the Access (Beckman), the ADVIA^{®}, the CENTAUR^{®} (Bayer), and the NICHOLS ADVANTAGE^{®} (Nichols Institute) immunoassay systems. Preferred apparatuses or protein chips perform simultaneous assays of a plurality of markers on a single surface. Particularly useful physical formats comprise surfaces having a plurality of discrete, addressable locations for the detection of a plurality of different markers. Such formats include protein microarrays, or "protein chips" *(see, e.g.,* Ng et al., J. Cell Mol. Med., 6:329-340 (2002)) and certain capillary devices *(see, e.g.,* U.S. Pat. No. 6,019,944). In these embodiments, each discrete surface location may comprise antibodies to immobilize one or more markers for detection at each location. Surfaces may alternatively comprise one or more discrete particles (*e*.*g*., microparticles or nanoparticles) immobilized at discrete locations of a surface, where the microparticles comprise antibodies to immobilize one or more markers for detection. Another suitable format for performing simultaneous assays of a plurality of markers is the Luminex MultiAnalyte Profiling (xMAP) technology, previously known as FlowMetrix and LabMAP (Elshal and McCoy, 2006), which is a multiplex bead-based flow cytometric assay that utilizes polystyrene beads that are internally dyed with different intensities of red and infrared fluorophores. The beads can be bound by various capture reagents such as antibodies, oligonucleotides, and peptides, therefore facilitating the quantification of various RNA, mRNA, miRNA, proteins, ligands, and DNA (Fulton *et al,* 1997; Kingsmore, 2006; Nolan and Mandy, 2006, Vignali, 2000; Ray *et al,* 2005).

Several markers of interest may be combined into one test for efficient processing of a multiple of samples. In addition, one skilled in the art would recognize the value of testing multiple samples *(e.g.,* at successive time points, *etc.)* from the same subject. Such testing of serial samples can allow the identification of changes in marker levels over time. Increases or decreases in marker levels, as well as the absence of change in marker levels, can also provide useful information to aid or assist in diagnosing IBS (*e*.*g*., compared with healthy subjects) and/or to aid or assist in discriminating between various subtypes of IBS from each other.

A panel for measuring one or more of the IBS markers described herein may be constructed. Such a panel may be constructed to determine the presence or level of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100 or more individual markers. The analysis of a single marker or subsets of markers can also be carried out by one skilled in the art in various clinical settings. These include, but are not limited to, ambulatory, urgent care, critical care, intensive care, monitoring unit, inpatient, outpatient, physician office, medical clinic, and health screening settings.

The analysis of IBS markers can be carried out in a variety of physical formats as well. For example, the use of microtiter plates or automation can be used to facilitate the processing of large numbers of test samples. Alternatively, single sample formats could be developed to facilitate treatment and diagnosis in a timely fashion.

### VI. Statistical Algorithms

In certain aspects, the present invention provides methods, systems, and codes for aiding or assisting in diagnosing IBS and/or discriminating between various subtypes of IBS from each other using a statistical algorithm to process information obtained from detecting the presence, (concentration) level, and/or gene expression level of one or more IBS markers described herein. In some instances, the statistical algorithms independently comprise one or more learning statistical classifier systems. In particular embodiments, statistical algorithms advantageously provide improved sensitivity, specificity, negative predictive value, positive predictive value, and/or overall accuracy for diagnosing IBS and/or discriminating between various subtypes of IBS from each other.

The term "statistical algorithm" or "statistical process" includes any of a variety of statistical analyses used to determine relationships between variables. The variables can be the presence or level of at least one marker of interest and/or the assessment of at least one psychological measure. Any number of markers and/or psychological measures can be analyzed using a statistical algorithm described herein. For example, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100 or more biomarkers and/or psychological measures can be included in a statistical algorithm. In one embodiment, logistic regression is used. In another embodiment, linear regression is used. In certain instances, the statistical algorithms of the present invention can use a quantile measurement of a particular marker within a given population as a variable. Quantiles are a set of "cut points" that divide a sample of data into groups containing (as far as possible) equal numbers of observations. For example, quartiles are values that divide a sample of data into four groups containing (as far as possible) equal numbers of observations. The lower quartile is the data value a quarter way up through the ordered data set; the upper quartile is the data value a quarter way down through the ordered data set. Quintiles are values that divide a sample of data into five groups containing (as far as possible) equal numbers of observations. The present invention can also include the use of percentile ranges of marker levels *(e.g.,* tertiles, quartile, quintiles, *etc.),* or their cumulative indices *(e.g.,* quartile sums of marker levels, *etc.)* as variables in the algorithms (just as with continuous variables).

In certain embodiments, the statistical algorithms comprise one or more learning statistical classifier systems. As used herein, the term "learning statistical classifier system" includes a machine learning algorithmic technique capable of adapting to complex data sets *(e.g.,* panel of markers of interest and/or psychological measures) and making decisions based upon such data sets. In some embodiments, a single learning statistical classifier system such as a classification tree (*e*.*g*., random forest) is used. In other embodiments, a combination of 2, 3, 4, 5, 6, 7, 8, 9, 10, or more learning statistical classifier systems are used, preferably in tandem. Examples of learning statistical classifier systems include, but are not limited to, those using inductive learning (*e*.*g*., decision/classification trees such as random forests, classification and regression trees (C&RT), boosted trees, *etc.),* Probably Approximately Correct (PAC) learning, connectionist learning (*e*.*g*., neural networks (NN), artificial neural networks (ANN), neuro fuzzy networks (NFN), network structures, perceptrons such as multi-layer perceptrons, multi-layer feed-forward networks, applications of neural networks, Bayesian learning in belief networks, *etc.),* reinforcement learning *(e.g.,* passive learning in a known environment such as naive learning, adaptive dynamic learning, and temporal difference learning, passive learning in an unknown environment, active learning in an unknown environment, learning action-value functions, applications of reinforcement learning, *etc.),* and genetic algorithms and evolutionary programming. Other learning statistical classifier systems include support vector machines (*e*.*g*., Kernel methods), multivariate adaptive regression splines (MARS), Levenberg-Marquardt algorithms, Gauss-Newton algorithms, mixtures of Gaussians, gradient descent algorithms, and learning vector quantization (LVQ).

Random forests are learning statistical classifier systems that are constructed using an algorithm developed by Leo Breiman and Adele Cutler. Random forests use a large number of individual decision trees and decide the class by choosing the mode (*i*.*e*., most frequently occurring) of the classes as determined by the individual trees. Random forest analysis can be performed, *e*.*g*., using the RandomForests software available from Salford Systems (San Diego, CA). *See, e.g.,* Breiman, Machine Learning, 45:5-32 (2001); and http://stat-www.berkeley.edu/users/breiman/RandomForests/cc_home.htm, for a description of random forests.

Classification and regression trees represent a computer intensive alternative to fitting classical regression models and are typically used to determine the best possible model for a categorical or continuous response of interest based upon one or more predictors. Classification and regression tree analysis can be performed, *e*.*g*., using the CART software available from Salford Systems or the Statistical data analysis software available from StatSoft, Inc. (Tulsa, OK). A description of classification and regression trees is found, *e.g.,* in Breiman et al. "Classification and Regression Trees," Chapman and Hall, New York (1984); and Steinberg et al., "CART: Tree-Structured Non-Parametric Data Analysis," Salford Systems, San Diego, (1995).

Neural networks are interconnected groups of artificial neurons that use a mathematical or computational model for information processing based on a connectionist approach to computation. Typically, neural networks are adaptive systems that change their structure based on external or internal information that flows through the network. Specific examples of neural networks include feed-forward neural networks such as perceptrons, single-layer perceptrons, multi-layer perceptrons, backpropagation networks, ADALINE networks, MADALINE networks, Learnmatrix networks, radial basis function (RBF) networks, and self-organizing maps or Kohonen self-organizing networks; recurrent neural networks such as simple recurrent networks and Hopfield networks; stochastic neural networks such as Boltzmann machines; modular neural networks such as committee of machines and associative neural networks; and other types of networks such as instantaneously trained neural networks, spiking neural networks, dynamic neural networks, and cascading neural networks. Neural network analysis can be performed, *e*.*g*., using the Statistical data analysis software available from StatSoft, Inc. *See, e.g.,* Freeman et al., In "Neural Networks: Algorithms, Applications and Programming Techniques," Addison-Wesley Publishing Company (1991); Zadeh, Information and Control, 8:338-353 (1965); Zadeh, "IEEE Trans. on Systems, Man and Cybernetics," 3:28-44 (1973); Gersho et al., In "Vector Quantization and Signal Compression," Kluywer Academic Publishers, Boston, Dordrecht, London (1992); and Hassoun, "Fundamentals of Artificial Neural Networks," MIT Press, Cambridge, Massachusetts, London (1995), for a description of neural networks.

Support vector machines are a set of related supervised learning techniques used for classification and regression and are described, *e.g.,* in Cristianini et al., "An Introduction to Support Vector Machines and Other Kernel-Based Learning Methods," Cambridge University Press (2000). Support vector machine analysis can be performed, *e*.*g*., using the SVM*^{light}* software developed by Thorsten Joachims (Cornell University) or using the LIBSVM software developed by Chih-Chung Chang and Chih-Jen Lin (National Taiwan University).

The statistical algorithms (*e*.*g*., learning statistical classifier systems) described herein can be trained and tested using a cohort of samples (*e*.*g*., serological samples) from healthy individuals, IBS patients, IBD patients, and/or Celiac disease patients. For example, samples from patients diagnosed by a physician, and preferably by a gastroenterologist as having IBD using a biopsy, colonoscopy, or an immunoassay as described in, *e.g.,* U.S. Patent No. 6,218,129, are suitable for use in training and testing the statistical algorithms described herein. Samples from patients diagnosed with IBD can also be stratified into Crohn's disease or ulcerative colitis using an immunoassay as described in, *e.g.,* U.S. Patent Nos. 5,750,355 and 5,830,675. Samples from patients diagnosed with IBS can be stratified into IBS-constipation (IBS-C), IBS-diarrhea (IBS-D), IBS-mixed (IBS-M), IBS-alternating (IBS-A), or post-infectious IBS (IBS-PI). Samples from patients diagnosed with IBS using a published criteria such as the Manning, Rome I, Rome II, or Rome III diagnostic criteria are suitable for use in training and testing the statistical algorithms described herein. Samples from healthy individuals can include those that were not identified as IBD and/or IBS samples. One skilled in the art will know of additional techniques and diagnostic criteria for obtaining a cohort of patient samples that can be used in training and testing the statistical algorithms described herein.

The term "sensitivity" refers to the probability that a method, system, or code of the invention gives a positive result when the sample is positive, *e*.*g*., having IBS or a particular IBS subtype. Sensitivity is calculated as the number of true positive results divided by the sum of the true positives and false negatives. Sensitivity essentially is a measure of how well a method, system, or code of the invention correctly identifies those with IBS or a particular IBS subtype from those without the disease. The statistical algorithms can be selected such that the sensitivity is at least about 60%, and can be, for example, at least about 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

The term "specificity" refers to the probability that a method, system, or code of the invention gives a negative result when the sample is not positive, *e*.*g*., not having IBS or a particular IBS subtype. Specificity is calculated as the number of true negative results divided by the sum of the true negatives and false positives. Specificity essentially is a measure of how well a method, system, or code of the invention excludes those who do not have IBS or a particular IBS subtype from those who have the disease. The statistical algorithms can be selected such that the specificity is at least about 70%, for example, at least about 75%, 80%, 85%, 86%, 87%, 88%, 89%, 90%, 91%, 92%, 93%, 94%, 95%, 96%, 97%, 98%, or 99%.

The term "negative predictive value" or "NPV" refers to the probability that an individual identified as not having IBS or a particular IBS subtype actually does not have the disease. Negative predictive value can be calculated as the number of true negatives divided by the sum of the true negatives and false negatives. Negative predictive value is determined by the characteristics of the method, system, or code as well as the prevalence of the disease in the population analyzed. The statistical algorithms can be selected such that the negative predictive value in a population having a disease prevalence is in the range of about 70% to about 99% and can be, for example, at least about 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

The term "positive predictive value" or "PPV" refers to the probability that an individual identified as having IBS or a particular IBS subtype actually has the disease. Positive predictive value can be calculated as the number of true positives divided by the sum of the true positives and false positives. Positive predictive value is determined by the characteristics of the method, system, or code as well as the prevalence of the disease in the population analyzed. The statistical algorithms can be selected such that the positive predictive value in a population having a disease prevalence is in the range of about 80% to about 99% and can be, for example, at least about 80%, 85%, 86%, 87%, 88%, 89%, 90%, 91%, 92%, 93%, 94%, 95%, 96%, 97%, 98%, or 99%.

Predictive values, including negative and positive predictive values, are influenced by the prevalence of the disease in the population analyzed. In the methods, systems, and code of the invention, the statistical algorithms can be selected to produce a desired clinical parameter for a clinical population with a particular IBS prevalence. For example, statistical algorithms can be selected for an IBS prevalence of up to about 1%, 2%, 3%, 4%, 5%, 6%, 7%, 8%, 9%, 10%, 15%, 20%, 25%, 30%, 35%, 40%, 45%, 50%, 55%, 60%, 65%, or 70%, which can be seen, *e.g.,* in a clinician's office such as a gastroenterologist's office or a general practitioner's office.

The term "overall agreement" or "overall accuracy" refers to the accuracy with which a method, system, or code of the invention classifies a disease state. Overall accuracy is calculated as the sum of the true positives and true negatives divided by the total number of sample results and is affected by the prevalence of the disease in the population analyzed. For example, the statistical algorithms can be selected such that the overall accuracy in a patient population having a disease prevalence is at least about 60%, and can be, for example, at least about 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

### VII. Disease Classification System

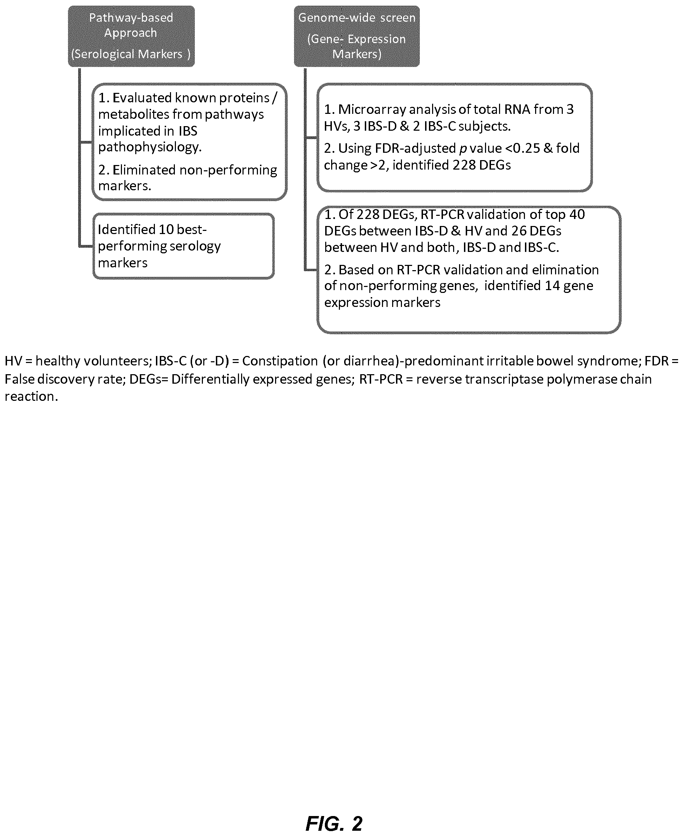

Figure 2 from US Patent Publication No. 2008/0085524, which is incorporated herein by reference in its entirety for all purposes, illustrates a disease classification system (DCS) (**200**) according to one embodiment of the present invention. As shown therein, a DCS includes a DCS intelligence module (**205**), such as a computer, having a processor (**215**) and memory module (**210**). The intelligence module also includes communication modules (not shown) for transmitting and receiving information over one or more direct connections (*e*.*g*., USB, Firewire, or other interface) and one or more network connections *(e.g.,* including a modem or other network interface device). The memory module may include internal memory devices and one or more external memory devices. The intelligence module also includes a display module (**225**), such as a monitor or printer. In one aspect, the intelligence module receives data such as patient test results from a data acquisition module such as a test system (**250**), either through a direct connection or over a network (**240**). For example, the test system may be configured to run multianalyte tests on one or more patient samples (**255**) and automatically provide the test results to the intelligence module. The data may also be provided to the intelligence module via direct input by a user or it may be downloaded from a portable medium such as a compact disk (CD) or a digital versatile disk (DVD). The test system may be integrated with the intelligence module, directly coupled to the intelligence module, or it may be remotely coupled with the intelligence module over the network. The intelligence module may also communicate data to and from one or more client systems (**230**) over the network as is well known. For example, a requesting physician or healthcare provider may obtain and view a report from the intelligence module, which may be resident in a laboratory or hospital, using a client system (**230**).

The network can be a LAN (local area network), WAN (wide area network), wireless network, point-to-point network, star network, token ring network, hub network, or other configuration. As the most common type of network in current use is a TCP/IP (Transfer Control Protocol and Internet Protocol) network such as the global internetwork of networks often referred to as the "Internet" with a capital "I," that will be used in many of the examples herein, but it should be understood that the networks that the present invention might use are not so limited, although TCP/IP is the currently preferred protocol.

Several elements in the system shown in Figure 2 from US Patent Publication No. 2008/0085524 may include conventional, well-known elements that need not be explained in detail here. For example, the intelligence module could be implemented as a desktop personal computer, workstation, mainframe, laptop, *etc.* Each client system could include a desktop personal computer, workstation, laptop, PDA, cell phone, or any WAP-enabled device or any other computing device capable of interfacing directly or indirectly to the Internet or other network connection. A client system typically runs an HTTP client, *e.g.,* a browsing program, such as Microsoft's Internet Explorer browser, Netscape's Navigator browser, Opera's browser, or a WAP-enabled browser in the case of a cell phone, PDA or other wireless device, or the like, allowing a user of the client system to access, process, and view information and pages available to it from the intelligence module over the network. Each client system also typically includes one or more user interface devices, such as a keyboard, a mouse, touch screen, pen or the like, for interacting with a graphical user interface (GUI) provided by the browser on a display (*e*.*g*., monitor screen, LCD display, *etc.)* (**235**) in conjunction with pages, forms, and other information provided by the intelligence module. As discussed above, the present invention is suitable for use with the Internet, which refers to a specific global internetwork of networks. However, it should be understood that other networks can be used instead of the Internet, such as an intranet, an extranet, a virtual private network (VPN), a non-TCP/IP based network, any LAN or WAN, or the like.

According to one embodiment, each client system and all of its components are operator configurable using applications, such as a browser, including computer code run using a central processing unit such as an Intel^{®} Pentium^{®} processor or the like. Similarly, the intelligence module and all of its components might be operator configurable using application(s) including computer code run using a central processing unit (**215**) such as an Intel Pentium processor or the like, or multiple processor units. Computer code for operating and configuring the intelligence module to process data and test results as described herein is preferably downloaded and stored on a hard disk, but the entire program code, or portions thereof, may also be stored in any other volatile or non-volatile memory medium or device as is well known, such as a ROM or RAM, or provided on any other computer readable medium (**260**) capable of storing program code, such as a compact disk (CD) medium, digital versatile disk (DVD) medium, a floppy disk, ROM, RAM, and the like.

The computer code for implementing various aspects and embodiments of the present invention can be implemented in any programming language that can be executed on a computer system such as, for example, in C, C++, C#, HTML, Java, JavaScript, or any other scripting language, such as VBScript. Additionally, the entire program code, or portions thereof, may be embodied as a carrier signal, which may be transmitted and downloaded from a software source (*e*.*g*., server) over the Internet, or over any other conventional network connection as is well known *(e.g.,* extranet, VPN, LAN, *etc.)* using any communication medium and protocols *(e.g.,* TCP/IP, HTTP, HTTPS, Ethernet, *etc.)* as are well known.

According to one embodiment, the intelligence module implements a process *(e.g.,* statistical algorithm) for analyzing marker levels of interest in a sample and/or psychological measures to aid or assist in diagnosing IBS and/or discriminating between various subtypes of IBS from each other. The data may be stored in one or more data tables or other logical data structures in memory (**210**) or in a separate storage or database system coupled with the intelligence module. One or more statistical processes are typically applied to a data set including test data for a particular patient. For example, the test data might include the presence or level of at least one IBS serological and/or genetic marker described herein and an assessment of at least one psychological measure of IBS. In some instances, a statistical process produces a statistically derived decision for aiding or assisting in diagnosing IBS or discriminating between various subtypes of IBS from each other. The statistically derived decision may be displayed on a display device associated with or coupled to the intelligence module, or the decision may be provided to and displayed at a separate system, *e.g.,* a client system (**230**). The displayed results allow a physician such as a gastroenterologist to make a reasoned diagnosis or prognosis.

### VIII. Therapy and Therapeutic Monitoring

Once a subject has been diagnosed with IBS or a particular IBS subtype, the present invention can further comprise administering to the subject a therapeutically effective amount of a drug useful for treating one or more symptoms associated with IBS (*i.e.,* an IBS drug). For therapeutic applications, the IBS drug can be administered alone or co-administered in combination with one or more additional IBS drugs and/or one or more drugs that reduce the side-effects associated with the IBS drug.

IBS drugs can be administered with a suitable pharmaceutical excipient as necessary and can be carried out via any of the accepted modes of administration. Thus, administration can be, *e*.*g*., intravenous, topical, subcutaneous, transcutaneous, transdermal, intramuscular, oral, buccal, sublingual, gingival, palatal, intra-joint, parenteral, intra-arteriole, intradermal, intraventricular, intracranial, intraperitoneal, intralesional, intranasal, rectal, vaginal, or by inhalation. By "co-administer" it is meant that an IBS drug is administered at the same time, just prior to, or just after the administration of a second drug (*e*.*g*., another IBS drug, a drug useful for reducing the side-effects of the IBS drug, *etc.).*

A therapeutically effective amount of an IBS drug may be administered repeatedly, *e.g.,* at least 2, 3, 4, 5, 6, 7, 8, or more times, or the dose may be administered by continuous infusion. The dose may take the form of solid, semi-solid, lyophilized powder, or liquid dosage forms, such as, for example, tablets, pills, pellets, capsules, powders, solutions, suspensions, emulsions, suppositories, retention enemas, creams, ointments, lotions, gels, aerosols, foams, or the like, preferably in unit dosage forms suitable for simple administration of precise dosages.

As used herein, the term "unit dosage form" refers to physically discrete units suitable as unitary dosages for human subjects and other mammals, each unit containing a predetermined quantity of an IBS drug calculated to produce the desired onset, tolerability, and/or therapeutic effects, in association with a suitable pharmaceutical excipient (*e*.*g*., an ampoule). In addition, more concentrated dosage forms may be prepared, from which the more dilute unit dosage forms may then be produced. The more concentrated dosage forms thus will contain substantially more than, *e.g.,* at least 1, 2, 3, 4, 5, 6, 7, 8, 9, 10, or more times the amount of the IBS drug.

Methods for preparing such dosage forms are known to those skilled in the art (*see, e.g.,* REMINGTON'S PHARMACEUTICAL SCIENCES, 18TH ED., Mack Publishing Co., Easton, PA (1990)). The dosage forms typically include a conventional pharmaceutical carrier or excipient and may additionally include other medicinal agents, carriers, adjuvants, diluents, tissue permeation enhancers, solubilizers, and the like. Appropriate excipients can be tailored to the particular dosage form and route of administration by methods well known in the art (*see, e.g., REMINGTON'S PHARMACEUTICAL SCIENCES*, *supra*).

Examples of suitable excipients include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, and polyacrylic acids such as Carbopols, *e.g.,* Carbopol 941, Carbopol 980, Carbopol 981, *etc.* The dosage forms can additionally include lubricating agents such as talc, magnesium stearate, and mineral oil; wetting agents; emulsifying agents; suspending agents; preserving agents such as methyl-, ethyl-, and propyl-hydroxy-benzoates (*i.e.,* the parabens); pH adjusting agents such as inorganic and organic acids and bases; sweetening agents; and flavoring agents. The dosage forms may also comprise biodegradable polymer beads, dextran, and cyclodextrin inclusion complexes.

For oral administration, the therapeutically effective dose can be in the form of tablets, capsules, emulsions, suspensions, solutions, syrups, sprays, lozenges, powders, and sustained-release formulations. Suitable excipients for oral administration include pharmaceutical grades of mannitol, lactose, starch, magnesium stearate, sodium saccharine, talcum, cellulose, glucose, gelatin, sucrose, magnesium carbonate, and the like.

In some embodiments, the therapeutically effective dose takes the form of a pill, tablet, or capsule, and thus, the dosage form can contain, along with an IBS drug, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof. An IBS drug can also be formulated into a suppository disposed, for example, in a polyethylene glycol (PEG) carrier.

Liquid dosage forms can be prepared by dissolving or dispersing an IBS drug and optionally one or more pharmaceutically acceptable adjuvants in a carrier such as, for example, aqueous saline *(e.g.,* 0.9% w/v sodium chloride), aqueous dextrose, glycerol, ethanol, and the like, to form a solution or suspension, *e*.*g*., for oral, topical, or intravenous administration. An IBS drug can also be formulated into a retention enema.

For topical administration, the therapeutically effective dose can be in the form of emulsions, lotions, gels, foams, creams, jellies, solutions, suspensions, ointments, and transdermal patches. For administration by inhalation, an IBS drug can be delivered as a dry powder or in liquid form via a nebulizer. For parenteral administration, the therapeutically effective dose can be in the form of sterile injectable solutions and sterile packaged powders. Preferably, injectable solutions are formulated at a pH of from about 4.5 to about 7.5.

The therapeutically effective dose can also be provided in a lyophilized form. Such dosage forms may include a buffer, *e*.*g*., bicarbonate, for reconstitution prior to administration, or the buffer may be included in the lyophilized dosage form for reconstitution with, *e*.*g*., water. The lyophilized dosage form may further comprise a suitable vasoconstrictor, *e*.*g*., epinephrine. The lyophilized dosage form can be provided in a syringe, optionally packaged in combination with the buffer for reconstitution, such that the reconstituted dosage form can be immediately administered to a subject.

In therapeutic use for the treatment of IBS, an IBS drug can be administered at the initial dosage of from about 0.001 mg/kg to about 1000 mg/kg daily. A daily dose range of from about 0.01 mg/kg to about 500 mg/kg, from about 0.1 mg/kg to about 200 mg/kg, from about 1 mg/kg to about 100 mg/kg, or from about 10 mg/kg to about 50 mg/kg, can be used. The dosages, however, may be varied depending upon the requirements of the subject, the severity of IBS symptoms, and the IBS drug being employed. For example, dosages can be empirically determined considering the severity of IBS symptoms in a subject diagnosed as having IBS or a subtype thereof according to the present methods. The dose administered to a subject, in the context of the present invention, should be sufficient to affect a beneficial therapeutic response in the subject over time. The size of the dose can also be determined by the existence, nature, and extent of any adverse side-effects that accompany the administration of a particular IBS drug in a subject. Determination of the proper dosage for a particular situation is within the skill of the practitioner. Generally, treatment is initiated with smaller dosages which are less than the optimum dose of the IBS drug. Thereafter, the dosage is increased by small increments until the optimum effect under circumstances is reached. For convenience, the total daily dosage may be divided and administered in portions during the day, if desired.

As used herein, the term "IBS drug" includes all pharmaceutically acceptable forms of a drug that is useful for treating one or more symptoms associated with IBS. For example, the IBS drug can be in a racemic or isomeric mixture, a solid complex bound to an ion exchange resin, or the like. In addition, the IBS drug can be in a solvated form. The term "IBS drug" is also intended to include all pharmaceutically acceptable salts, derivatives, and analogs of the IBS drug being described, as well as combinations thereof. For example, the pharmaceutically acceptable salts of an IBS drug include, without limitation, the tartrate, succinate, tartarate, bitartarate, dihydrochloride, salicylate, hemisuccinate, citrate, maleate, hydrochloride, carbamate, sulfate, nitrate, and benzoate salt forms thereof, as well as combinations thereof and the like. Any form of an IBS drug is suitable for use in the methods of the present invention, *e*.*g*., a pharmaceutically acceptable salt of an IBS drug, a free base of an IBS drug, or a mixture thereof.

Suitable drugs that are useful for treating one or more symptoms associated with IBS include, but are not limited to, serotonergic agents, antidepressants, chloride channel activators, chloride channel blockers, guanylate cyclase agonists, antibiotics, opioids, neurokinin antagonists, antispasmodic or anticholinergic agents, belladonna alkaloids, barbiturates, glucagon-like peptide-1 (GLP-1) analogs, corticotropin releasing factor (CRF) antagonists, probiotics, free bases thereof, pharmaceutically acceptable salts thereof, derivatives thereof, analogs thereof, and combinations thereof. Other IBS drugs include bulking agents, dopamine antagonists, carminatives, tranquilizers, dextofisopam, phenytoin, timolol, and diltiazem.

Serotonergic agents are useful for the treatment of IBS symptoms such as constipation, diarrhea, and/or alternating constipation and diarrhea. Non-limiting examples of serotonergic agents are described in Cash et al., Aliment. Pharmacol. Ther., 22:1047-1060 (2005), and include 5-HT₃ receptor agonists (*e.g.,* MKC-733, *etc*.), 5-HT₄ receptor agonists *(e.g.,* tegaserod (Zelnorm), prucalopride, AG1-001, *etc*.), 5-HT₃ receptor antagonists *(e.g.,* alosetron (Lotronex^{®}), cilansetron, ondansetron, granisetron, dolasetron, ramosetron, palonosetron, E-3620, DDP-225, DDP-733, *etc*.), mixed 5-HT₃ receptor antagonists/5-HT₄ receptor agonists *(e.g.,* cisapride, mosapride, renzapride, *etc.),* free bases thereof, pharmaceutically acceptable salts thereof, derivatives thereof, analogs thereof, and combinations thereof. Additionally, amino acids like glutamine and glutamic acid which regulate intestinal permeability by affecting neuronal or glial cell signaling can be administered to treat patients with IBS.

Antidepressants such as selective serotonin reuptake inhibitor (SSRI) or tricyclic antidepressants are particularly useful for the treatment of IBS symptoms such as abdominal pain, constipation, and/or diarrhea. Non-limiting examples of SSRI antidepressants include citalopram, fluvoxamine, paroxetine, fluoxetine, sertraline, free bases thereof, pharmaceutically acceptable salts thereof, derivatives thereof, analogs thereof, and combinations thereof. Examples of tricyclic antidepressants include, but are not limited to, desipramine, nortriptyline, protriptyline, amitriptyline, clomipramine, doxepin, imipramine , trimipramine, maprotiline, amoxapine, clomipramine, free bases thereof, pharmaceutically acceptable salts thereof, derivatives thereof, analogs thereof, and combinations thereof.

Chloride channel activators are useful for the treatment of IBS symptoms such as constipation. A non-limiting example of a chloride channel activator is lubiprostone (Amitiza), a free base thereof, a pharmaceutically acceptable salt thereof, a derivative thereof, or an analog thereof. In addition, chloride channel blockers such as crofelemer are useful for the treatment of IBS symptoms such as diarrhea. Guanylate cyclase agonists such as MD-1100 are useful for the treatment of constipation associated with IBS *(see, e.g.,* Bryant et al., Gastroenterol., 128:A-257 (2005)). Antibiotics such as neomycin can also be suitable for use in treating constipation associated with IBS *(see, e.g.,* Park et al., Gastroenterol., 128:A-258 (2005)). Non-absorbable antibiotics like rifaximin (Xifaxan) are suitable to treat small bowel bacterial overgrowth and/or constipation associated with IBS *(see, e.g.,* Sharara et al., Am. J. Gastroenterol., 101:326-333 (2006)).

Opioids such as kappa opiods (*e*.*g*., asimadoline) may be useful for treating pain and/or constipation associated with IBS. Neurokinin (NK) antagonists such as talnetant, saredutant, and other NK2 and/or NK3 antagonists may be useful for treating IBS symptoms such as oversensitivity of the muscles in the colon, constipation, and/or diarrhea. Antispasmodic or anticholinergic agents such as dicyclomine may be useful for treating IBS symptoms such as spasms in the muscles of the gut and bladder. Other antispasmodic or anticholinergic agents such as belladonna alkaloids (*e*.*g*., atropine, scopolamine, hyoscyamine, *etc.)* can be used in combination with barbiturates such as phenobarbital to reduce bowel spasms associated with IBS. GLP-1 analogs such as GTP-010 may be useful for treating IBS symptoms such as constipation. CRF antagonists such as astressin and probiotics such as VSL#3^{®} may be useful for treating one or more IBS symptoms. One skilled in the art will know of additional IBS drugs currently in use or in development that are suitable for treating one or more symptoms associated with IBS.

A subject can also be monitored at periodic time intervals to assess the efficacy of a certain therapeutic regimen once diagnosed as having IBS or a subtype thereof. For example, the levels of certain markers change based on the therapeutic effect of a treatment such as a drug. The subject is monitored to assess response and understand the effects of certain drugs or treatments in an individualized approach. Additionally, some subjects may not respond to a certain drug, but the markers may change, indicating that these subjects belong to a special population (not responsive) that can be identified by their marker levels. These subjects can be discontinued on their current therapy and alternative treatments prescribed.

### IX. Example

The present invention will be described in greater detail by way of specific example. The following example is offered for illustrative purposes, and is not intended to limit the invention in any manner. Those of skill in the art will readily recognize a variety of noncritical parameters which can be changed or modified to yield essentially the same results.

### Example 1. Diagnostic Models Based on Biomarker Panels and Psychological Morbidity for Irritable Bowel Syndrome and Novel Pathophysiological Leads.

This example illustrates methods of using quantitative biological markers alone or in combination with psychological measures for diagnosing IBS. In particular, the methods can aid in differentiating IBS subjects from healthy subjects and/or IBS subtypes from each other. In certain embodiments, this example describes the identification and validation of panels of 34 or fewer biomarkers that can be used to predict or discriminate IBS and/or IBS subtypes.

The biomarkers of the invention can include serological markers (e.g., histamine, PGE2, tryptase, serotonin, substance P, IL-12, IL-10, IL-6, IL-8, TNF-α, ANCA, ASCA IgA, BDNF, anti-CBir1, GRO-α, IL-1β, NGAL, TIMP-1, TWEAK, and/or tTG), genetic markers (*e*.*g*., CBFA2T2, CCDC147, HSD17B11, LDLR, MAP6D1, MICALL1, RAB7L1, RNF26, RRP7A, SUSD4, SH3BGRL3, VIPR1, WEE1, and/or ZNF326), and combinations thereof. The psychological measures of the invention can include the Patient Health Questionnaire 15 (PHQ-15) such as PHQ (non-GI), the perceived stress scale (PSS), the Hospital Anxiety and Depression scale (HADs), the IBS-Severity Scoring System (IBS-SSS), the Functional Bowel Disease Severity Index (FBDSI), a self-report of overall IBS severity (*e*.*g*., bowel symptom questionnaires such as the Rome III 10-question IBS Module, Bristol Stool Form Scale, and Rome III 93-question GI questionnaire), self-rated pain severity, and combinations thereof.

### Abstract

**Background:** The development of a reliable biomarker for irritable bowel syndrome (IBS) remains one of the major aims of research in functional gastrointestinal disorders (FGIDs). The challenge is formidable in the absence of a perfect or even near-perfect reference standard. Previous efforts based on genetic and immune markers have showed promise, but have not been robust.
**Aims:** To evaluate an extensive panel of gene expression and serology measures against Rome III criteria for IBS.
**Methods:** Of subjects eligible for analysis (N = 244), 168 met criteria for IBS (60 IBS-C, 57 IBS-D, and 51 mixed) while 76 were free of any FGID. A total of 34 markers were selected based on pathways implicated in pathophysiology of IBS or whole human genome screening. Diagnostic models were based on unconditional logistic regression and performance assessed through area under the receiver-operator characteristic curve (AUC), sensitivity, and specificity.
**Results:** The performance of a combination of 34 markers was good with peak performance observed when discriminating IBS-C from IBS-D. Utilizing all 34 markers achieved good overall diagnostic performance with AUCs: 0.81 for IBS v health, 0.92 for IBS-C v IBS-D, 0.85 for IBS-C v IBS-M and 0.86 for IBS-D v IBS-M. Diagnostic model performance was derived largely from a small number of markers. More parsimonious models achieved adequate diagnostic performance: IBS v health AUC = 0.80 from 6 markers, IBS-C v IBS-D AUC = 0.88 from 16 markers, IBS-C v IBS-M AUC = 0.81 from 9 markers and IBS-D v IBS-M AUC = 0.80 from 7 markers. Diagnostic model probabilities showed no correlation with either disease severity or psychological symptom burden.
**Conclusions:** A combination of gene expression and serological biomarkers is particularly useful for diagnosing or differentiating IBS compared with healthy subjects and IBS subtypes from each other *(e.g.,* IBS-C from IBS-D).

### Introduction

Irritable bowel syndrome (IBS) is a highly prevalent functional gastrointestinal disorder affecting 10-15% of the population in the Western countries (1), with a higher prevalence in women than men. Patients with IBS are classified into three major groups according to their predominant bowel symptoms: constipation predominant IBS (IBS-C), diarrhea predominant IBS (IBS-D), and IBS with mixed diarrhea and constipation (IBS-M) (2).

In current clinical practice, guidelines suggest that the diagnosis of IBS should be based on typical symptoms with judicious exclusion of organic gastrointestinal disorders such as celiac disease (3, 4). Symptom-based criteria such as the Rome criteria for diagnosing IBS have been developed by an international committee of gastroenterologists; however, these are not applied consistently in a clinical practice setting by community gastroenterologists or primary care physicians (5). Current clinical practice still leads clinicians to often order a wide variety of tests before making a confident diagnosis of IBS, especially in older patients where the pre-test probability of organic disease (e.g., colon cancer) is much higher (6).

Most of the tests that clinicians may routinely order, including a complete blood count, serum chemistry, liver enzymes, thyroid function tests, and stool sampling, have very low diagnostic values in patients with typical IBS symptoms and no alarm features (such as weight loss, blood in the stool, unexplained iron deficiency anemia, nocturnal diarrhea, or a family history of inflammatory bowel disease, celiac sprue, or colon cancer) (7). Notably, such testing can confuse because false positive results lead to unnecessary diagnostic evaluations, and true negative results are not necessarily reassuring for the doctor or patient. Challenges of diagnosing IBS are further complicated by the fact that IBS patients often present with co-existing functional disorders such as functional dyspepsia, fibromyalgia, chronic pelvic pain, or interstitial cystitis. As a result, patients with IBS visit physicians more often, consume more medications, and undergo more diagnostic tests than non-IBS patients (8, 9).

While the etiology of this disorder remains obscure, there is a body of evidence suggesting dysregulation of several pathophysiological pathways including serotonin biosynthesis and metabolism (10-12), mast cell infiltration and degranulation (13-17), visceral hypersensitivity, an exaggerated stress response, immune activation and bacterial infection (post infectious-IBS) or microbiota alterations (18-22).

Gene expression profiling in tissue samples taken from patients with IBS has been reported using sigmoid colonic mucosal tissue (23). Although certain gene expression biomarkers have been recently reported in the literature, these markers were derived from data mining of a published inflammatory bowel disease study (24). However, it is unknown whether there exists "surrogate" transcriptional biomarkers in the peripheral blood cells of patients with IBS.

IBS is widely considered to be a heterogeneous condition possibly resulting in a common constellation of symptoms from multiple distinct pathologies (25). Apart from the biological pathways discussed already, individuals with IBS are also known to suffer elevated levels of mood disorders (anxiety and depression) compared with healthy individuals (26, 27). Whether mood disorder lies antecedent to the onset of IBS or results from the symptoms of the disease remains an open question (28-30), although the biopsychosocial model (31) would suggest a bidirectional relationship. There is no strong evidence that IBS subtypes have different mood profiles.

### Methods

### Patients

IBS patients and healthy volunteers were recruited from 12 US tertiary referral centers as well as 23 community gastroenterology clinics. All IBS patients had a physician diagnosis of IBS, met Rome III criteria for IBS and did not have any other gastrointestinal disorders; however, dyspepsia or heartburn were not exclusionary. Patients with extraintestinal functional disorders, organic gastrointestinal disorders or major psychiatric comorbidities including severe anxiety and depression (HADs score ≥18 for either scale) were all excluded. Age- and gender-matched healthy volunteers were Rome III-negative for IBS, did not have chronic gastrointestinal symptoms, any active infections, or significant chronic medical conditions. At the time of blood collection, enrolled patients were not taking medications that are known to interfere with serotonin metabolism, mast cell degranulation or other inflammatory pathways that were under investigation. Chronic use of non-steroidal anti-inflammatory drugs (NSAIDs) was exclusionary with the exception of prophylactic use of low dose aspirin (<82 mg). All subjects provided written informed consent for analysis of their blood samples, including separate consents for genetic analyses. The protocol was approved by institutional review boards (IRBs) of the respective academic institutions or by the central IRB, BioMed.

### Definition of IBS and IBS subgroups

IBS subjects in this study were required to meet Rome III criteria (2) and be diagnosed with IBS by experienced gastroenterologists. In addition, subjects were required to experience active IBS symptoms more than twice a week in the month prior to enrolment and be free of comorbidities reported to be highly prevalent in individuals with IBS (33), including major psychiatric disorders as well as other non-gastrointestinal functional disorders such as fibromyalgia, chronic fatigue, and chronic pelvic pain.

Subjects were assigned to the different subgroups of diarrhea-predominant (IBS-D), constipation-predominant (IBS-C) or mixed IBS (IBS-M) based on predominant bowel habit according to the Rome III subtype table and scored by the Bristol Stool Form Scale, which was asked over a three month recall period.

### Assessment of IBS severity

Subjects with any degree of IBS severity were enrolled in the study. However, severity was assessed in all IBS subjects via 4 different measures as there is no consensus definition for categorizing IBS patients based on severity. These measures included 2 validated instruments, the IBS-Severity Scoring System (IBS-SSS) (34) and the Functional Bowel Disease Severity Index (FBDSI) (35) as well as 2 self report scales: a self-report of overall IBS severity (not at all, somewhat, moderately, very, or extremely severe in response to, "rate how severe your IBS is?"); and self-rated pain severity using a 5 point Likert scale (0 = none, 1 = mild, 2 = moderate, 3 = intense and 4 = severe).

### Psychological measures

In addition to excluding subjects with a diagnosis of one of the excluded comorbidities, all subjects were administered the Hospital Anxiety and Depression scale or HADs (36) to identify and exclude subjects with severe anxiety or depression at screening (anxiety or depression score >18). Other psychological measures assessed somatisation status using the Patient Health Questionnaire 15 (PHQ-15) and stress status using the perceived stress scale or PSS (37). While no subjects were excluded based on total score on these two scales, the scoring was intended to allow stratification of patients during analyses.

The PHQ-15 assesses the extent to which individuals are bothered by a range of somatic symptoms. Several of these symptoms are gastrointestinal and have been excluded from consideration in this analysis since they may induce a logical and statistical circularity. Specifically, for this analysis, we omitted items "a: stomach pain", "d: menstrual cramps", "1: constipation, loose bowels, diarrhea" and "m: nausea, gas or indigestion" from the PHQ-15. A total PHQ score was calculated using the remaining items and is referred to as the PHQ-non GI.

### Selection of markers

The panel of ten biomarkers reported by Lembo *et al.* (32) was identified using a seven-step procedure that initially considered more than 60,000 potential biomarkers identified via literature searching then filtered down to 10 candidate biomarkers through pragmatic considerations around measurement and demonstrated efficacy in differentiating IBS patients from controls.

### 1. Blood sample collection and RNA isolation

The process used to identify the additional 24 markers is summarized in Figure 2. Blood samples were collected from eight subjects. In this case, ∼2.4 ml of whole blood was collected from each subject. The blood sample was divided into two aliquots, and one was processed according to the PAXgene RNA preparation protocol. Degradation of multiple hemoglobin mRNA species in the samples was accomplished using RNase H and specifically designed primers for nine common hemoglobin genes on four donor samples. Briefly, 5 µg of total cellular RNA was incubated in 10mMTris·HCl, pH 7.6, 20 mM KCl with 10 µM of oligonucleotide primers at 70°C for 5 min. The samples were cooled to 4°C, and 2 U of RNase H (New England Biolabs), along with 20 U of SUPERase Inhibitor (Ambion), was added. The buffer conditions were adjusted to 55 mM Tris·HCl, 85 mM KCl, 3 mM MgCl₂,and 10 mM dithiothreitol, and the samples were incubated at37°C for 15 min. Immediately following the incubation, the samples were again cooled to 4°C and 1 µl of 0.5 M EDTA was added to stop the RNase H digestion. The samples were then repurified using the RNeasy Mini Protocol for RNA Cleanup(Qiagen), according to the manufacturer's specifications and including the optional DNase treatment.

### 2. Gene chip human array

The samples were grouped by class and the group were blinded prior the screening; group 1 contained three IBS-D, group 2 contained two IBS-C, and group 3 contained three healthy subjects. The screening was performed with Affymetrix Human Gene 1.0 ST arrays (Affymetrix, Santa Clara, CA), an oligonucleotide-probe based gene array chip containing ∼35,000 transcripts, which provides a comprehensive coverage of the whole human genome. Eight micrograms of total RNA was used to synthesize cDNA. T7 promoter introduced during the first strand synthesis was then used to direct cRNA synthesis, which was labeled with biotinylated deoxynucleotide triphosphate, following the manufacturer's protocol (Affymetrix, San Diego, California). After fragmentation, the biotinylated cRNA was hybridized to the gene chip array at 45°C for 16 h. The chip was washed, stained with phycoerytherin-streptavidin, and scanned with the Gene Chip Scanner 3000. After background correction, preliminary data analysis was done in the Microarray Suite 5.0 software (MAS 5.0, Stratagene, La Jolla, CA). For primary analysis we used PLIER as recommended in the work flow of software Gene Spring GX10.0 (Agilent Technologies, Santa Clara, CA).

### 3. Gene array data analysis

Fluorescence intensities were uploaded to the Array Assist 6.5 and Gene Spring GX10.0 (Agilent Technologies, Santa Clara, CA) software. Data was normalized by quantitative normalization, and then transferred logarithmically for further analysis to determine changes in a particular gene.

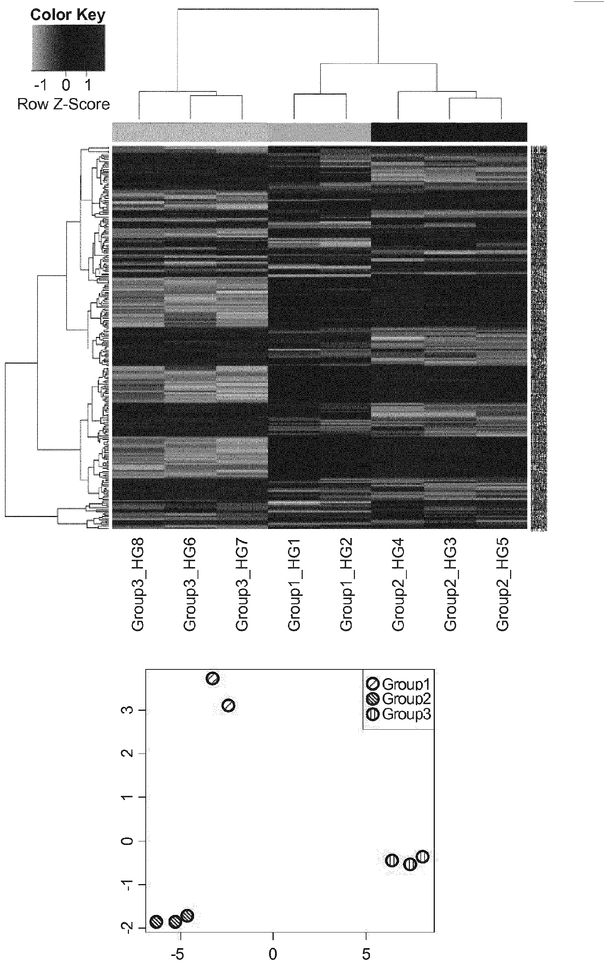

In order to compare the changes in gene expression, the data was further normalized by using the 50 RFU fluorescence value as threshold, and statistical significance was determined (*p* ≤ 0.05). Hierarchical clustering analysis was performed to explore whether the expression profiles of the differentially expressed genes (DEGs) can separate the three groups of samples into distinct classes. A heat map with two dimension hierarchical clustering results was generated in the micro array analysis to demonstrate the sample and gene clustering structure based on gene expression profiles. To ensure of the robustness of the profile among the group, we then performed multidimensional scaling testing (MDS) to explore similarities or dissimilarities in data from different groups. We used an MDS algorithm that starts with a matrix of item-item similarities, then assigned a location of each item in a low-dimensional space, suitable for 2D visualization. After the group status was unveiled, we analyzed the raw gene expression data using analysis of variance (ANOVA) to compare the means of hybridization signals in all three groups. The test is designed to detect DEGs between any pair group. Using a threshold of false discovery rate adjusted p-value < 0.25 and a fold change > 2, we found 228 differentially expressed genes cumulatively. We then performed a hierarchical clustering analysis to explore whether the gene expression profiles of the DEGs can separate samples into distinct classes. We used all unmasked probe sets in this analysis. Figures 3a/3b show the clustering results. Three groups are completely separated by the gene expression profiles of the DEGs, which are indicated by the panels at the top of the heatmap (Figure 3a). The separation among samples was further visualized based on the gene expression profiles of all unmasked probe sets using a multidimensional scaling plot (Figure 3b).

In order to select among the 228 differentially expressed genes, a pair-wise t-test was performed between each pair of groups. Fold change, p value and FDR-adjusted p-value (38) were computed for each probe set on the array in each comparison. Differentially expressed genes (DEGs) were defined as those genes that have an FDR-adjusted p-value <0.25 and a fold change > 2. 40 DEGs between IBS-D and healthy volunteers were ordered by fold change. In order to identify genes which can be used for both IBS-C and IBS-D subgroup diagnosis, we further selected 26 genes which were up-regulated in both groups based on>2 fold changes and P values.

### 4. Real time quantitative PCR validation of selected DEGs

We further validated the 66 selected genes out of 228 by qRT-PCR using samples from 27 healthy volunteers, 19 IBS-C, 22 IBS-D, and 17 IBS-M patients. Total RNA was reverse transcribed into cDNA in a 20 µl reaction using a high capacity cDNA reverse transcription kit (Applied Biosystems, Bedford, MA). cDNA was then diluted to 200 ng/µl per reaction. Real time quantitative reverse transcript-polymerase chain reaction (qRT-PCR) was performed in duplicates using two sequence-specific PCR primers and a TaqMan assay-FAM dye labeled MGB probe to validate the microarray data. Assays were run using 2 x Taqman gene expression master mixes with RNase inhibitor on ABI 7900 Fast thermocycler (Applied Biosystems, Bedford, MA). FAM-dye labeled β-actin is used as an endogenous control for normalization and Ct values were obtained for both reference and target gene by auto baseline and auto threshold settings. ΔΔCt method is used to calculate the % expression.

A panel of 14 genes was subsequently selected based on the microarray/TaqMan results confirmation with reference to fold change levels and tested again for confirmation on samples from 97 healthy volunteers, 72 IBS-C, 82 IBS-D, and 71 IBS-M patients.

### Statistical methods

### 1. Identification of Biomarkers

As described above, biomarker selection therefore comprised multiple approaches:
(1) Pathway-focused approach targeting pathways implicated in IBS pathophysiology, which resulted in identification of 10 serological markers from pathways involved in pain, serotonin metabolism, mast cell activation and inflammation.
(2) Analysis of differentially expressed genes in IBS and healthy volunteers, which resulted in identification of 14 differentially expressed genes.

The 24 new markers identified using these approaches were combined with the 10 markers from Lembo *et al.* (32), resulting in a set of 34 markers that were used for further statistical analyses as described below and in Table 1.

**Table 1. The 34 biomarker panel for IBS identified and tested.**

| **Original Biomarker Panel (from Lembo *et al.* [32])** | **Description** |
|---|---|
| Interleukin-1β (IL-1β) | A proinflammatory cytokine that plays a central role in inflammatory diseases such as IBD and is known to be downregulated by glucocorticoids released during stress. |
| Growth-related oncogene-α (GRO-α) | A chemokine associated with chemotactic migration and activation of neutrophils, which may be involved in tissue injury in IBD patients. |
| Brain-derived neurotrophic factor (BDNF) | A nerve growth factor thought to be a regulator of neuronal transmission, which may play an important stimulant role in long-term regulation of gastrointestinal motility. |
| Anti-*Saccharomyces cerevisiae* antibody (ASCA IgA) | An antibody that may reflect a generalized loss of immunotolerance. High levels of ASCA IgA are frequently found in Chron's disease patients. |
| Antibody against CBir1 (Anti-CBirl) | An antibody against bacterial flagellin. Bacterial flagellin is recognized by cells of the gut mucosa, which may then activate innate immunity. |
| Anti-human tissue transglutaminase (tTG) | tTG is a tissue-repair enzyme and the major autoantigen in celiac disease. Anti-tTG testing can aid in the diagnosis of celiac disease. |
| Tumor necrosis factor (TNF) - like weak inducer of apoptosis (TWEAK) | A cyctokine that controls cellular activities such as proliferation, migration, differentiation, apoptosis, and angiogenesis. TWEAK levels are downregulated in autoimmune pathologies. |
| Anti-neutrophil cytoplasmic antibody (ANCA) | Autoantibodies that target antigens present in neutrophils which have been identified in the serum of 50% to 80% of ulcerative colitis patients. |
| Tissue inhibitor of metalloproteinase-1 (TIMP-1) | An inhibitor of metalloproteinase (MMPs) that breaks down extracellular matrix proteins involved in wound healing, angiogenesis, and tumor-cell metastasis. In the gut, altered TIMP activity can result in tissue destruction, intestinal barrier function impairment, bacteria influx, and excessive immune response. |
| Neutrophil gelatinase-associated lipocalin (NGAL) | Belongs to the lipocalin family of proteins. In the viscera, NGAL is involved in a range of functions including molecular transport and GI mucosal regeneration. |

| **New Biomarkers (N = 24) (serologic markers and gene expression markers)** | |
|---|---|
| Serologic Markers (N = 10): | |
| Histamine | Released by mast cells and involved in allergic reactions. Histamine can cause inflammation and increased permeability of blood vessels. Histamine causes constriction of smooth muscle. |
| PGE2 | Prostaglandin E2 (PGE2) is involved in neuronal function, female reproduction, vascular hypertension, tumorigenesis, kidney function and inflammation. |
| Tryptase | Can be used as an indicator of mast cell activation. Tryptases are mediators of asthma and other allergic reactions, and are also involved in several inflammatory disorders. |
| Serotonin | Serotonin is a neurotransmitter, derived from tryptophan, involved in brain function. Serotonin is primarily found in the gut. Serotonin regulates physiological function such as well being, appetite, sleep, and pain sensitivity and gut motility. |
| Substance P | A sensory neuropeptide involved in pain perception. Substance P is also associated with mood disorders, anxiety and stress. |
| IL-12 | IL-12 is a heteromeric cytokine involved in naive T cells development. It stimulates production of INF-y and TNF-α by T cells. IL-12 also has anti-angiogenic activity. |
| IL-10 | IL-10 is an anti-inflammatory cytokine mainly produced by monocytes, and can inhibit the synthesis of IFN-γ, IL-2, TNF-α, and GM-CSF. |
| IL-6 | IL-6 is a pro-inflammatory cytokine. It is an acute phase response cytokine secreted by T cells and macrophages. |
| IL-8 | IL-8 is a chemokine and one of the major mediators of the inflammatory response. IL-8 is produced by several cell types and by macrophages. IL-8 is chemoattractant, and is also a potent angiogenic factor. |
| TNF-α | TNF-α is mainly produced by macrophages and is found in acute and chronic inflammatory conditions. |
| Gene Expression Markers (N = 14): | |
| CBFA2T2 | Core-binding factor, runt domain, alpha subunit 2; translocated to, 2. Biological role unknown. May function as a complex with the chimeric protein RUNX1/ AML1-CBFA2T1/MTG8 which is produced in acute myeloid leukemia with the chromosomal translocation t(8;21) potentially repressing AML1-dependent transcription of G-CSF/CSF-3-dependent cell growth. |
| CCDC147 | Coiled-coil domain containing 147. Biological role unknown. |
| HSD17B11 | Hydroxysteroid (17-beta) dehydrogenase 11. May participate in androgen metabolism during steroidogenesis. |
| LDLR | The low density lipoprotein receptor (LDLR) gene family consists of cell surface proteins involved in receptor-mediated endocytosis of specific ligands. |
| MAP6D1 | Encodes a protein highly similar to the mouse MAP6 domain containing 1 protein. May function as a calmodulin-regulated neuronal protein that binds and stabilizes microtubules. |
| MICALL1 | MICAL-like 1, Cytoskeletal regulator, binds to Rab 13. Participates in the assembly and activity of tight junctions. |
| RAB7L1 | Member RAS oncogene family-like 1. Biological role unknown. |
| RNF26 | Ring finger protein 26, contains a C3HC5 type of RING finger, a motif known to be involved in protein-DNA and protein-protein interactions. |
| RRP7A | Ribosomal RNA processing 7 homolog A (*S. cerevisiae*). Biological role unknown. |
| SUSD4 | Sushi domain containing 4. Biological role unknown. |
| SH3BGRL3 | Belongs to the SH3BGR family, binds to SH3 domain and has SH3/SH2 adaptor activity. |
| VIPR1 | Vasoactive Intestinal Peptide Receptor 1, a gut hormone. |
| WEE1 | Biological role unknown. May act as negative regulator of entry into mitosis. Activity of WEE1 increases during s and G2 phases and decreases during M Phase |
| ZNF326 | Zinc finger protein 326, Probable transcriptional activator which may play a role in neuronal differentiation. |

### 2. Validation of Biomarkers

Diagnostic models have been developed to differentiate IBS from healthy volunteers and to distinguish between IBS subtypes, specifically: (i) IBS from health; (ii) IBS-C from IBS-D; (iii) IBS-C from IBS-M; and (iv) IBS-D from IBS-M. All models are based on unconditional logistic regression estimating the probability of a specific disease state (i - iv above) based on a panel of 34 biological markers (biomarkers), all of which are measured on a quantitative scale as described above. For each disease comparison, the diagnostic performance of three models is reported: (a) the full model incorporating all 34 potential biomarkers regardless of statistical significance; (b) four psychological measures *(e.g.,* PHQ (omitting GI items), HAD anxiety and depression and the perceived stress score) in addition to the 34 biomarkers; (c) backward elimination selection of markers with statistical significance at p<0.05; and (d) backward elimination selection of markers and the four psychological measures with statistical significance at p<0.05. We regard models (a) and (b) to be the primary analyses and models (c) and (d) to provide an indication of many individual markers and psychological factors are driving the panel's diagnostic performance.

The performance of the panel of ten markers originally reported by Lembo *et al.* (32) was also considered and the results reported in Table 2.

**Table 2. Performance of the original panel of 10 markers from Lembo et al. (32).**

| **Groups discriminated** | **AUC (95% CI)** | **¹Sensitivity (95% CI)** | **¹Specificity (95% CI)** |
|---|---|---|---|
| IBS v health | 0.74 (0.68, 0.81) | 0.70 (0.62, 0.76) | 0.67 (0.55, 0.77) |
| IBS-C v IBS-D | 0.70 (0.61, 0.80) | 0.72 (0.59, 0.83) | 0.75 (0.62, 0.86) |
| IBS-C v IBS-M | 0.65 (0.54, 0.75) | 0.55 (0.42, 0.68) | 0.69 (0.54, 0.81) |
| IBS-D v IBS-M | 0.71 (0.61, 0.81) | 0.67 (0.53, 0.79) | 0.65 (0.50, 0.78) |

| | | | |
|---|---|---|---|
| ¹Diagnostic probabilities categorized as positive if greater than the probability at which the separate sensitivity and specificity curves cross. | | | |

Model performance is reported in terms of overall performance through the AUC with 95% confidence interval and through sensitivity and specificity assessed at a threshold probability identified as the point at which the separate sensitivity and specificity curves cross when both are plotted against diagnostic probability.

We performed a logistic regression analysis using all 34 markers as predictor variables, and disease vs. healthy control as the response variable. No marker interactions were investigated. For this analysis IBS-C, -D and -M were considered to be a single disease state ("IBS"). These data comprised n = 246 subjects. Predictions from the model were applied to the same data set and Receiver Operator Characteristic (ROC) analysis was performed to find the ROC Area Under the Curve (AUC).

### Results

The validation sample consisted of n=294 individuals of whom n=90 were healthy volunteers (HV) free of functional gastrointestinal disease while the remaining n=204 met Rome III criteria for irritable bowel syndrome (IBS). A subset of n=244 individuals have data on all 34 biomarkers and this group has been utilized in all statistical analyses reported while 25 individuals have values recorded for 28 markers and a further 25 individuals have values recorded for only 24 markers. There was no difference in the missing value pattern across IBS and health with 82% of IBS subjects having complete data compared with 84% of the healthy volunteers.

Among the n=244 subjects utilized in this study, the IBS group were divided into 60 IBS-C, 57 IBS-D and 51 mixed IBS (IBS-M) and there were n=76 health volunteers. Study groups did not vary substantially with age or gender (Table 3) except that the IBS-D group was made up of proportionately fewer females than IBS-C, IBS-M and healthy volunteers. IBS subgroups did not differ substantively with respect to any psychological variable (Table 3). IBS subgroups were also not markedly different in average scores on disease severity scales (Table 3). IBS subjects were however elevated compared with healthy volunteers in anxiety, depression, somatic symptom reporting and measures of functional bowel symptoms (Table 3).

**Table 3. Demographic psychological characteristics of the cohort.**

| **Characteristic** | **IBS-C (n=60)** | **IBS-D (n=57)** | **IBS-M (n=51)** | **Healthy (n=76)** | **p¹** | **p²** |
|---|---|---|---|---|---|---|
| Age (Mean, SD) | 38.8 (12.6) | 41.1 (13.6) | 37.5 (13.3) | 38.8 (12.4) | 0.9 | 0.6 |
| Gender (% Female) | 86 | 65 | 85 | 79 | 0.9 | 0.01 |
| Anxiety Score (mean, SD) | 6.52 (3.89) | 6.09 (3.42) | 6.22 (3.50) | 4.12 (2.67) | <0.0001 | 0.8 |
| Depression Score (mean, SD) | 3.30 (3.98) | 3.07 (3.20) | 2.41 (2.52) | 1.47 (1.85) | 0.0002 | 0.7 |
| Non-GI PHQ Score Score (mean, SD) | 5.70 (3.61) | 5.81 (3.67) | 6.16 (3.32) | 1.99 (1.63) | <0.0001 | 0.5 |
| PSS Score Score (mean, SD) | 15.12 (7.80) | 12.81 (6.36) | 15.00 (7.08) | 9.01 (5.80) | <0.0001 | 0.3 |
| Total IBS-SSS score (mean) | 267.20 (91.64) | 250.14 (74.13) | 266.24 (78.70) | - | - | 0.1 |
| Total FBDSI score (mean) | 53.37 (51.12) | 51.84 (40.76) | 67.61 (50.71) | - | - | 0.3 |

| | | | | | | |
|---|---|---|---|---|---|---|
| ¹Comparing IBS as one group with health ²Comparing IBS subtypes | | | | | | |

### Performance of the original panel

The original panel of ten markers from Lembo *et al.* (32) is reported in Table 2 above. Lembo *et al.* reported an AUC of 0.76 for the discrimination of IBS from health and the performance of their panel in the current sample was consistent with that with an AUC of 0.74 (95% CI 0.68, 0.81). Performance of this panel in discriminating between subgroups was a little lower than for IBS from health (Table 2).

### Simple comparisons of IBS and healthy volunteers

Inspection of Table 4 indicates that a small number of biomarkers individually noticeably differentiate the four study groups.

**Table 4. Values obtained for the 34 biomarker panel in IBS subtypes and healthy volunteers.**

| | **Study group** | | | | | | | | | | | | |
|---|---|---|---|---|---|---|---|---|---|---|---|---|---|
| | **HV** | | | **IBS-C** | | | **IBS-D** | | | **IBS-M** | | | |
| | **Mean** | **SD** | **N** | **Mean** | **SD** | **N** | **Mean** | **SD** | **N** | **Mean** | **SD** | **N** | **¹p** |
| Histamine | 181.04 | 125.57 | 6 | 135.33 | 74 | | 176.60 | 11 | 7 | 126.35 | 59.91 | 51 | 0.02 |
| PGE2 | 423.35 | 320.76 | 76 | 413.42 | 452.32 | 60 | 507.95 | 409.98 | 57 | 324.62 | 215.46 | 51 | 0.1 |
| Tryptase | 9.00 | 17.08 | 76 | 9.72 | 20.54 | 60 | 7.41 | 6.98 | 57 | 7.15 | 11.98 | 51 | 0.6 |
| Serotonin | 239.60 | 105.95 | 76 | 247.22 | 142.80 | 60 | 202.12 | 100.94 | 57 | 202.22 | 97.75 | 51 | 0.1 |
| Substance P | 515.08 | 221.24 | 76 | 569.01 | 219.28 | 60 | 549.70 | 201.28 | 57 | 573.59 | 187.46 | 51 | 0.1 |
| IL12 | 8.54 | 51.94 | 76 | 3.35 | 11.71 | 60 | 5.38 | 23.20 | 57 | 0.54 | 1.51 | 51 | 0.3 |
| IL10 | 5.69 | 24.11 | 76 | 2.25 | 5.81 | 60 | 3.99 | 15.07 | 57 | 1.04 | 2.50 | 51 | 0.5 |
| IL6 | 0.39 | 0.33 | 76 | 0.36 | 0.33 | 60 | 0.42 | 0.25 | 57 | 0.80 | 1.98 | 51 | 0.02 |
| IL8 | 5.67 | 5.33 | 76 | 5.95 | 11.41 | 60 | 8.38 | 11.23 | 57 | 5.81 | 6.63 | 51 | 0.3 |
| TNF-α | 1.78 | 1.11 | 76 | 1.95 | 1.37 | 60 | 1.82 | 0.54 | 57 | 2.58 | 4.34 | 51 | 0.1 |
| CBFA2T2 | 0.55 | 0.34 | 76 | 0.58 | 0.28 | 60 | 0.67 | 0.44 | 57 | 0.57 | 0.35 | 51 | 0.5 |
| CCDC147 | 0.02 | 0.01 | 76 | 0.03 | 0.02 | 60 | 0.03 | 0.02 | 57 | 0.03 | 0.02 | 51 | 0.6 |
| HSD17B11 | 2.38 | 1.28 | 76 | 2.62 | 1.35 | 60 | 2.66 | 1.45 | 57 | 2.45 | 1.48 | 51 | 0.4 |
| LDLR | 0.06 | 0.03 | 76 | 0.06 | 0.03 | 60 | 0.06 | 0.02 | 57 | 0.06 | 0.04 | 51 | 0.8 |
| MAP6D1 | 0.01 | 0.00 | 76 | 0.01 | 0.00 | 60 | 0.01 | 0.00 | 57 | 0.01 | 0.00 | 51 | 0.5 |
| MICALL1 | 0.27 | 0.12 | 76 | 0.29 | 0.10 | 60 | 0.25 | 0.10 | 57 | 0.27 | 0.12 | 51 | 0.3 |
| RAB7L1 | 0.36 | 0.26 | 76 | 0.42 | 0.20 | 60 | 0.36 | 0.19 | 57 | 0.36 | 0.21 | 51 | 0.06 |
| RNF26 | 0.34 | 0.17 | 76 | 0.37 | 0.14 | 60 | 0.38 | 0.17 | 57 | 0.35 | 0.14 | 51 | 0.1 |
| RNF26 RRP7A | 0.57 | 0.41 | 76 | 0.73 | 0.60 | 60 | 0.61 | 0.40 | 57 | 0.58 | 0.28 | 51 | 0.2 |
| SUSD4 | 0.09 | 0.09 | 76 | 0.12 | 0.12 | 60 | 0.09 | 0.07 | 57 | 0.11 | 0.10 | 51 | 0.04 |
| SH3BGRL3 | 22.09 | 8.84 | 76 | 23.18 | 8.07 | 60 | 21.99 | 6.59 | 57 | 22.78 | 7.73 | 51 | 0.6 |
| VIPR1 | 0.36 | 0.30 | 76 | 0.35 | 0.17 | 60 | 0.31 | 0.14 | 57 | 0.36 | 0.21 | 51 | 0.5 |
| WEE1 | 0.07 | 0.04 | 76 | 0.08 | 0.05 | 60 | 0.06 | 0.03 | 57 | 0.07 | 0.04 | 51 | 0.3 |
| ZNF326 | 0.31 | 0.26 | 76 | 0.31 | 0.16 | 60 | 0.27 | 0.12 | 57 | 0.28 | 0.15 | 51 | 0.6 |
| ANCA | 6.83 | 5.70 | 76 | 8.45 | 6.49 | 60 | 10.26 | 13.50 | 57 | 8.49 | 7.24 | 51 | 0.3 |
| ASCA-IgA | 7.52 | 8.59 | 76 | 8.58 | 10.88 | 60 | 8.10 | 12.76 | 57 | 7.48 | 7.72 | 51 | 0.6 |
| BDNF | 16644.68 | 4983.28 | 76 | 16835.42 | 5846.83 | 60 | 17552.89 | 5925.02 | 57 | 17560.20 | 5558.95 | 51 | 0.9 |
| Anti-CBir1 | 16.79 | 20.52 | 76 | 13.22 | 8.75 | 60 | 14.35 | 16.25 | 57 | 13.90 | 11.31 | 51 | >0.9 |
| GRO-α | 239.01 | 208.91 | 76 | 413.38 | 583.09 | 60 | 251.01 | 202.93 | 57 | 327.30 | 288.07 | 51 | 0.2 |
| IL1Beta | 158.93 | 194.28 | 76 | 171.01 | 269.50 | 60 | 109.88 | 110.16 | 57 | 132.31 | 135.16 | 51 | 0.7 |
| NGAL | 139.78 | 64.82 | 76 | 136.61 | 47.68 | 60 | 157.13 | 46.14 | 57 | 151.53 | 54.93 | 51 | 0.01 |
| TMP-1 | 240.63 | 45.60 | 76 | 238.03 | 51.55 | 60 | 249.45 | 50.59 | 57 | 253.90 | 69.54 | 51 | 0.4 |
| TWEAK | 1080.76 | 443.86 | 76 | 1112.27 | 371.58 | 60 | 1221.61 | 375.27 | 57 | 1057.49 | 401.11 | 51 | 0.04 |
| | 0.96 | 4.68 | 76 | 0.30 | 0.27 | 60 | 0.29 | 0.29 | 57 | 0.23 | 0.18 | 51 | 0.0002 |

| | | | | | | | | | | | | | |
|---|---|---|---|---|---|---|---|---|---|---|---|---|---|
| ¹p = p-value from Kruskal-Wallis test | | | | | | | | | | | | | |

### Panel performance in differentiating IBS from health

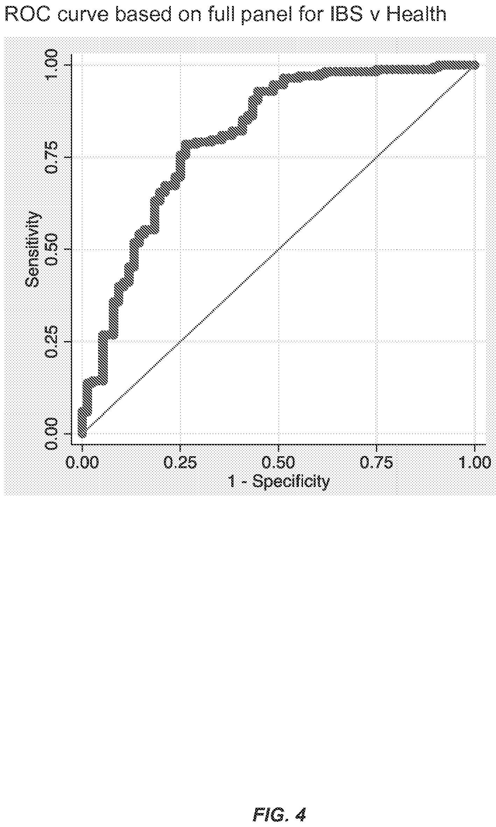

A diagnostic model including all biomarkers provides credible differentiation of IBS from health with an AUC of 0.81 (Table 5, Figure 4) and at a threshold probability of 0.60, sensitivity is 0.81 (95% CI: 0.75, 0.87) and specificity is 0.64 (95% CI: 0.54, 0.75). Model selection indicates that a small subset of markers is responsible for the bulk of this performance with a sub-panel of 4 markers yielding an AUC of 0.71 (Table 5).

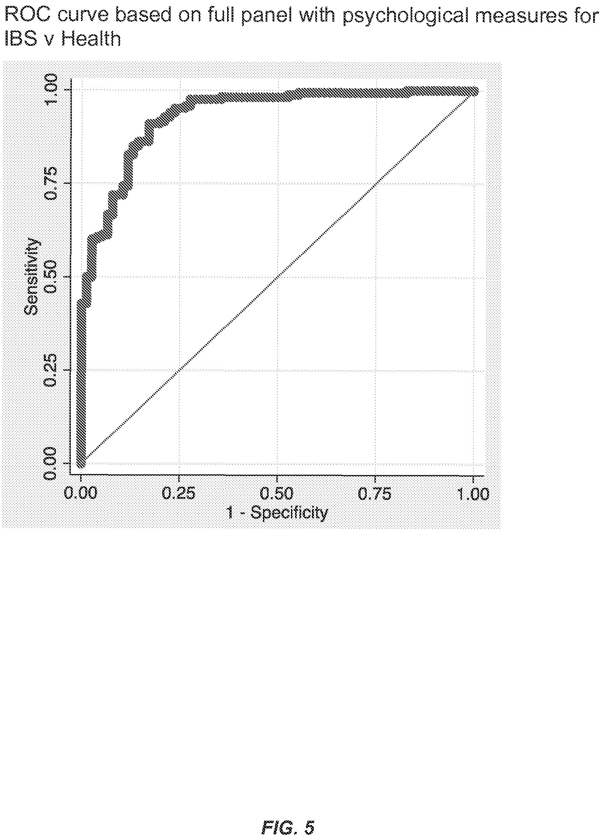

The addition of four psychological measures to the full panel provided substantial incremental value with an AUC of 0.93 and sensitivity and specificity ≥0.80 at a probability threshold of 0.70 (Table 5) and this is reflected in the shape of the ROC curve (Figure 5). Of the four psychological measures, the non-GI PHQ (excluding GI items) OR=2.41 (95% CI 1.77, 3.27; p<0.0005) and perceived stress OR=1.12 (95% CI 1.01, 1.23; p=0.03) were most important. The addition of the psychological measures to the sub-panel also improved performance substantially with an AUC of 0.91 and reasonable sensitivity and specificity, although only the PHQ reached statistical significance. Neither age nor gender added to the discriminatory performance of the model once genetic and psychological factors are taken into account.

**Table 5. Diagnostic model performance.**

| **Panel** | **IBS v health** | **IBS-C v IBS-D** | **IBS-C v IBS-M** | **IBS-D v IBS-M** |
|---|---|---|---|---|
| All markers | 0.81 (0.75, 0.87) | 0.92 (0.87, 0.97) | 0.85 (0.78, 0.92) | 0.86 (0.79, 0.93) |
| | [34] | [34] | [34] | [34] |
| | Sₑ=0.81, Sₚ=0.64 | Sₑ=0.83, Sₚ=0.86 | Sₑ=0.82, Sₚ=0.69 | Sₑ=0.84, Sₚ=0.67 |
| Minimum set | 0.71 (0.64, 0.78) | 0.75 (0.67, 0.84) | 0.70 (0.60, 0.79) | 0.77 (0.68, 0.86) |
| | [4] | [4] | [4] | [5] |
| | Sₑ=0.80, Sₚ=0.47 | Sₑ=0.75, Sₚ=0.65 | Sₑ=0.88, Sₚ=0.45 | Sₑ=0.81, Sₚ=0.55 |
| | Histamine, znf326, rnf26, Ttg | Histamine, NGALn, micall1, rnf26 | Ttg, rab711, IL6, vipr1 | Histamine, vipr1, rnf26, ttg, TWEAKn |
| All markers and psych² | 0.93 (0.90, 0.97) | 0.94 (0.90, 0.98) | 0.88 (0.82, 0.94) | 0.91 (0.86, 0.96) |
| | [38] | [38] | [38] | [38] |
| | Sₑ=0.85, Sₚ=0.88 | Sₑ=0.87, Sₚ=0.84 | Sₑ=0.77, Sₚ=0.75 | Sₑ=0.79, Sₚ=0.84 |
| Minimum set with psych² | 0.91 (0.87,0.95) | 0.81 (0.73, 0.88) | 0.75 (0.66, 0.84) | 0.80 (0.72, 0.88) |
| | [8] | [8] | [6] | [7] |
| | Sₑ=0.82, Sₚ=0.83 | Sₑ=0.77, Sₚ=0.70 | Sₑ=0.72, Sₚ=0.65 | Sₑ=0.79, Sₚ=0.67 |
| | Ttg, vipr1, znf326, hsd17bl1, wee1, TNFa, PSS, PHQ (non-GI) | Histamine, rnf26, rrp7a, substance P, NGALn, rab711, PHQ (non-GI), PSS | IL6, MAP6dl, vipr1, rab711, PHQ (non-GI), HAD depression | GROAn, PGE2, TWEAKn, RNF26, VIPr1, HAD anxiety, HAD depression |

| | | | | |
|---|---|---|---|---|
| Note: Entries are area under ROC curve (AUC) followed by 95% confidence interval in parentheses and number of markers included in the model in square parentheses. Sₑ = sensitivity, Sₚ = specificity. | | | | |

### Panel performance in differentiating IBS subtypes

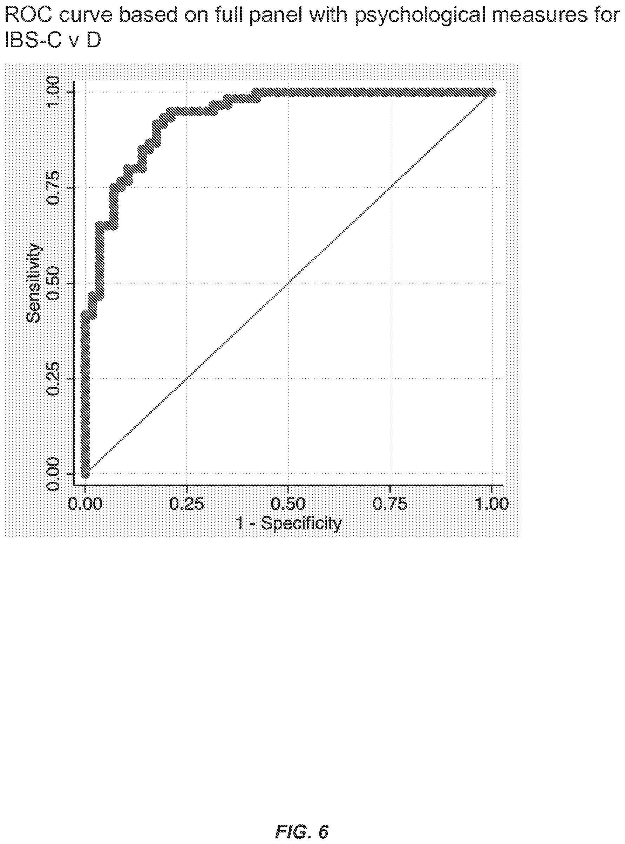

A diagnostic model including all biomarkers provides good differentiation of IBS-C from IBS-D with an AUC of 0.92 (Table 5) and at a threshold probability of 0.50 achieves sensitivity of 0.83 (95% CI: 0.74, 0.93) and specificity of 0.86 (95% CI: 0.77, 0.95). Model selection again indicates that a small subset of markers is responsible for the bulk of this performance, with a sub-panel of 4 markers yielding an AUC of 0.75 (Table 5). The addition of four psychological measures provided little incremental value to the diagnostic performance, raising the AUC to 0.94 (Figure 6), although only one of the measures met the conventional criterion of statistical significance (perceived stress; OR=1.19, 95% CI 1.01, 1.41; p=0.04).

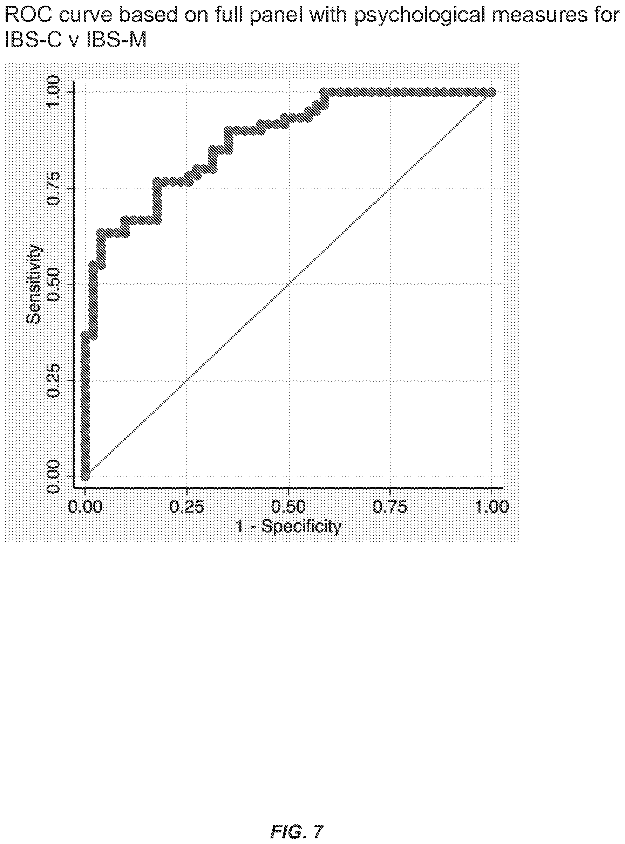

Adequate differentiation of IBS-C from IBS-M was achieved using all 34 markers (AUC=0.85, Table 5) and again a sub-panel of four markers accounts for a large proportion of the overall panel's diagnostic performance (Table 5). The additional of psychological measures provided little incremental differentiation (Figure 7).

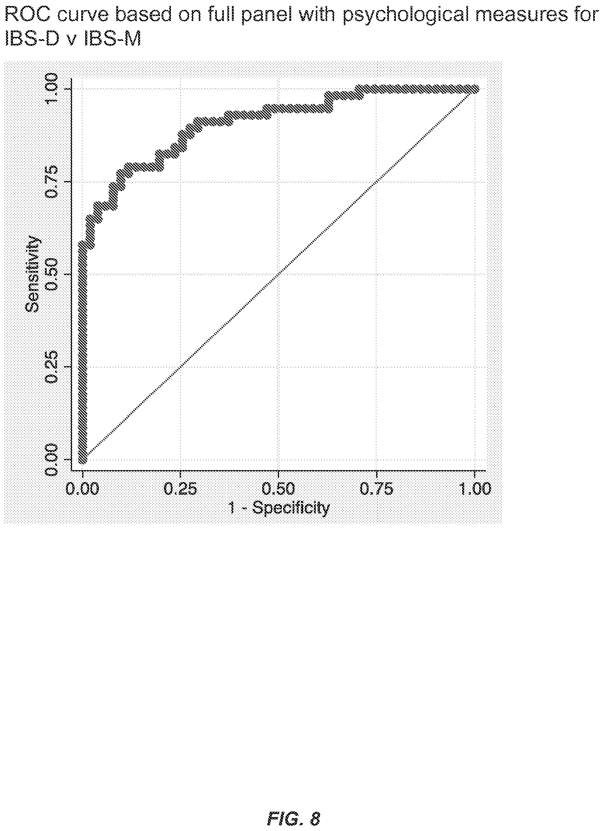

Adequate differentiation of IBS-D from IBS-M was also achieved using all 34 markers (AUC=0.86, Table 5) and a sub-panel of five markers accounts for a large proportion of the overall panel's diagnostic performance (Table 5). The addition of psychological measures provided little incremental differentiation (Figure 8).

Neither age nor gender add to the discriminatory performance of the model with respect to differentiating any pair of subtypes once genetic and psychological factors are taken into account.

### Additional IBS panel performance data

Tables 6 and 7 below provide additional analysis and comparison of different panels of the diagnostic biomarkers of the present invention. In particular, to develop a small subset of biomarkers that provides sufficient diagnostic performance (termed Parsimonious panel or model), all 34 quantitative markers were considered as potential predictive variables for four outcome variables (*e*.*g*., IBS vs. health; IBS-C vs. IBS-D; IBS-C vs. IBS-M, IBS-D vs. IBS-M). The data was modeled via unconditional logistic regression. Backward elimination of markers at o<0.1 was performed to achieve an AUC≥0.8. Model performance was assessed for the Parsimonious model similarly to that for the Full model. Table 6 shows the diagnostic biomarkers of the Parsimonious model for the four outcome variables. Performance analysis was performed (AUC = 0.7412). Table 7 shows the performance data *(e.g.,* AUC, sensitivity, and specificity) of the diagnostic methods of the invention, including the Full panel of 34 quantitative biomarkers, the Parsimonious panel, and the Minimal panel for each of the four diagnostic outcomes analyzed.

As described above, the performance of markers that met conventional statistical criteria were calculated and a Minimal panel or model of biomarkers ("Minimum set") was developed that can be used to diagnose or discriminate IBS and/or IBS subtypes. To create the diagnostic model, all 34 quantitative markers were considered as potential predictive variables for four outcome variables ((*e*.*g*., IBS vs. health; IBS-C vs. IBS-D; IBS-C vs. IBS-M, IBS-D vs. IBS-M). The Minimal model was created using unconditional logistic regression. Backward elimination of markers at p<0.05 to achieve conventionally statistically independent discriminators was performed. The diagnostic performance of the model was assessed from calculations of the area under the receiver-operator characteristic curve (AUC) and both sensitivity and specificity with the Rome III criteria as the reference standard. Sensitivity and specificity were estimated at the threshold where curves cross on the predicted probability scale. Table 6 shows the diagnostic biomarkers of the Minimal model for the four outcome variables. The performance of the method was calculated (AUC = 0.7099). Table 7 shows the performance data *(e.g.,* AUC, sensitivity, and specificity) of the diagnostic methods of the invention, including the Full panel of 34 quantitative biomarkers, the Parsimonious panel, and the Minimal panel for each of the four diagnostic outcomes analyzed.

**Table 6. Diagnostic biomarkers to discriminate IBS subjects from healthy subjects and IBS subtypes from each other.**

| **Panel (Model)** | **IBS v. Healthy** | **IBS-C v. IBS-D** | **IBS-C v. IBS-M** | **IBS-D v. IBS-M** |
|---|---|---|---|---|
| **Full panel (model)** | Entire Panel | Entire Panel | Entire Panel | Entire Panel |
| **Parsimonious panel (model)** | histamine, NGALn, znf326, substance P, rnf26, TTG | histamine, TTG, vipr1, substance P, IL12, IL10, IL6, IL1Betan, TNFa, rrp7a, ccdc147, ASCA IgA, NGALn, map6d1, GROα | map6d1, rab711, NGALn, serotonin, vipr1, IL1Betan, IL10, IL6, rrp7a | histamine, pge2, GROAN, TTG, TWEAKn, rnf26, vipr1 |
| **Minimal panel (model)** | histamine, znf326, rnf26, TTG | histamine, NGALn, micall1, rnf26 | TTG, rab711, IL6, vipr1 | histamine, vipr1, rnf26, TTG, TWEAKn |

**Table 7. Diagnostic tests of IBS and IBS subtypes.**

| **Panel (Model)** | **Discrimination** | **# Markers** | **AUC (95% CI)** | **Sensitivity** | **Specificity** |
|---|---|---|---|---|---|
| **Full panel (model)** | IBS v. Health | 34 | 0.81 (0.75,0.87) | 0.81 | 0.64 |
| | IBS-C v. IBS-D | 34 | 0.92 (0.87, 0.97) | 0.83 | 0.86 |
| | IBS-C v. IBS-M | 34 | 0.85 (0.78, 0.92) | 0.82 | 0.69 |
| | IBS-D v. IBS-M | 34 | 0.86 (0.79, 0.93) | 0.84 | 0.67 |
| **Parsimonious panel (model)** | IBS v. Health | 6 | 0.74 (0.67, 0.81) | 0.83 | 0.55 |
| | IBS-C v. IBS-D | 16 | 0.88 (0.82, 0.95) | 0.85 | 0.84 |
| | IBS-C v. IBS-M | 9 | 0.81 (0.73, 0.89) | 0.83 | 0.61 |
| | IBS-D v. IBS-M | 7 | 0.80 (0.72, 0.88) | 0.79 | 0.61 |
| **Minimal panel (model)** | IBS v. Health | 4 | 0.71 (0.64, 0.78) | 0.80 | 0.47 |
| | IBS-C v. IBS-D | 4 | 0.75 (0.67, 0.84) | 0.75 | 0.65 |
| | IBS-C v. IBS-M | 4 | 0.70 (0.60, 0.79) | 0.88 | 0.45 |
| | IBS-D v. IBS-M | 5 | 0.77 (0.68, 0.86) | 0.81 | 0.55 |

### Discussion

This study set out to assess the performance of a set of 34 biological markers of irritable bowel syndrome (IBS) both in terms of differentiating IBS-qualifying individuals from healthy volunteers and in terms of differentiating IBS subtypes from each other. The identification of an array of biological markers that would achieve these differentiations with high sensitivity and specificity would transform IBS from a symptom-based diagnosis of exclusion in clinical practice into a regular medical disease and provide avenues of investigation into possible new pathophysiological mechanisms.

The full set of 34 biomarkers was found to provide encouraging differentiation of IBS from healthy volunteers and with acceptable sensitivity and specificity (Table 5). Further, the addition of four psychological measures covering mood (anxiety and depression), stress and non-GI somatic symptoms yielded excellent overall performance (AUC=0.93) with both sensitivity and specificity ≥0.90. In the model that included both biological and psychological markers, a small subset of both accounts for the bulk of the model performance. This study indicates a clinically useful role for psychological factors in the identification of IBS.

The set of biomarkers described in this study also differentiated IBS subtypes. The data for differentiation of IBS-C from IBS-D was particularly strong with an AUC based on the full panel of biomarkers of 0.92 (Table 5). Performance of this set of biomarkers in differentiating IBS-C from IBS-M (AUC 0.85) and IBS-D from IBS-M (AUC 0.86) were also encouraging. The incremental value of psychological measures in differentiating subtypes appears to be minimal, with modest increases in AUC when psychological measures were included (Table 5). This indicates the influence of psychological factors is limited to differentiating IBS from health and that, conditional on having IBS, psychological factors play little if any role in subtype differentiation.

The subset of markers that were found to provide statistically independent differentiation of IBS subtypes varied considerably between subtype comparisons (Table 5). This provides encouraging although indirect evidence that distinct mechanisms are being identified through the markers selected. For example, four biomarkers provided discrimination of IBS-C from IBS-D: histamine, NGALn, micall1, and rnf26 (*see,* Table 5). NGALn belongs to the lipocalin family of proteins; in the viscera, NGAL is involved in a range of functions including molecular transport and mucosal regeneration. Similarly, MICAL-like 1 cytoskeletal regulator binds to Rab 13, and participates in the assembly and activity of tight junctions. Ring finger protein 26 is known to be involved in protein-DNA and protein-protein interactions, which may also impact on intestinal barrier function. Other data indicate that a leaky mucosal barrier may be a key abnormality in IBS (39). Histamine may reflect mast cell dysfunction, also known to be a key pathophysiolgical marker in IBS (40, 41). The highly novel data presented here in turn indicate that IBS subtypes represent entities that are to some extent biologically distinct.

IBS is likely a heterogenous disorder making identification of unique biomarkers potentially extremely challenging. In order to maximize the signal to noise ratio and allow the possible identification of unique biomarkers, the patients enrolled in this study comprised a relatively homogenous IBS population. Specifically, they were diagnosed by experienced gastroenterologists, met established symptom-based criteria (Rome III) for IBS, were experiencing typical IBS symptoms at the time of study enrolment, and were free of comorbidities reported to be highly prevalent in IBS patients to avoid identification of confounding markers. These comorbidities included psychiatric disorders such as major depression, anxiety or somatoform disorders, as well as other non-gastrointestinal functional disorders such as fibromyalgia, chronic fatigue, and chronic pelvic pain. Healthy volunteers enrolled as the control group were adults without any illness, active infection or significant medical condition.

This study adds to the mounting evidence that IBS has an underlying set of biological cause(s) (42). Strengths of the study include well-characterized cases and controls, and the novel application of a biomarker panel. The set of biomarkers described in this study could distinguish IBS from health. A study of unselected patients presenting for care can also be performed (STARD guidelines) (43).

In conclusion, we have identified a novel panel of biomarkers in IBS. Strikingly, a panel of biomarkers alone can discriminate IBS-C from IBS-D, and psychological measures added little additional information, providing strong novel evidence these may be distinct and measurable disease states that can be objectively identified.

### References

1. Cremonini F, Talley N. Irritable bowel syndrome: epidemiology, natural history, health-care seeking and emerging risk factors. Gastroenterol Clin North Am. 2005(34):189-204.
2. Thompson W, Longstreth G, Drossman D, Heaton K, Irvine E, Muller-Lissner S. Functional bowel disorders and functional abdominal pain. Gut. 1999;Sep;45(Suppl 2:II43-7).
3. Brandt L, Chey W, Foxx-Orenstein A, Schiller L, Schoenfeld P, Spiegel B, et al. An evidence-based position statement on the management of irritable bowel syndrome. Am J Gastroenterol. 2009;Jan;104(Suppl 1:S1-35).
4. Chey W, Nojkov B, Rubenstein J, Dobhan R, Greenson J, Cash B. The yield of colonoscopy in patients with non-constipated irritable bowel syndrome: results from a prospective, controlled US trial. Am J Gastroenterol. 2010;Apr;105(4):859-65.
5. Spiegel B, Farid M, Esrailian E, Talley J, Chang L. Is irritable bowel syndrome a diagnosis of exclusion?: a survey of primary care providers, gastroenterologists, and IBS experts. Am J Gastroenterol. 2010;Apr;105(4):848-58.
6. Lacy B, Rosemore J, Robertson D, Corbin D, Grau M, Crowell M. Physicians' attitudes and practices in the evaluation and treatment of irritable bowel syndrome. Scand J Gastroenterol. 2006;Aug;41(8):892-902.
7. Cash B, Schoenfeld P, Chey W. The utility of diagnostic tests in irritable bowel syndrome patients: a systematic review. Am J Gastroenterol. 2002;97(11):2812-9.
8. Schmulson M, Chang L. Diagnostic approach to the patient with irritable bowel syndrome. Am J Med. 1999;Nov 8;107(5A):20S-6S.
9. Tillisch K, L LC. Diagnosis and treatment of irritable bowel syndrome: state of the art. Curr Gastroenterol Rep. 2005;Aug;7(4):249-56.
10. Gershon M. Nerves, reflexes, and the enteric nervous system: pathogenesis of the irritable bowel syndrome. J Clin Gastroenterol. 2005;May-Jun;39(5, Suppl 3):5184-93.
11. Coates M, Mahoney C, Linden D, Sampson J, Chen J, Blaszyk H, et al. Molecular defects in mucosal serotonin content and decreased serotonin reuptake transporter in ulcerative colitis and irritable bowel syndrome. Gastroenterol. 2004;Jun;126(7):1657-64.
12. Mawe G, Coates M, Moses P. Review article: intestinal serotonin signalling in irritable bowel syndrome. Aliment Pharmacol Ther. 2006;Apr 23(8):1067-76.
13. Róka R, Rosztóczy A, Leveque M, Izbéki F, Nagy F, Molnár T, et al. A pilot study of fecal serine-protease activity: a pathophysiologic factor in diarrhea-predominant irritable bowel syndrome. Clin Gastroenterol Hepatol. 2007;May;5(5):550-5.
14. Barbara G, Wang B, Stanghellini V, Giorgio Rd, Cremon C, Nardo GD, et al. Mast cell-dependent excitation of visceral-nociceptive sensory neurons in irritable bowel syndrome. Gastroenterol. 2007;Jan;132(1):26-37.
15. Guilarte M, Santos J, Torres Id, Alonso C, Vicario M, Ramos L, et al. Diarrhoea-predominant IBS patients show mast cell activation and hyperplasia in the jejunum. Gut. 2007;Feb;56(2):203-9.
16. Barbara G, Stanghellini V, Giorgio Rd, Cremon C, Cottrell G, Santini D, et al. Activated mast cells in proximity to colonic nerves correlate with abdominal pain in irritable bowel syndrome. Gastroenterol. 2004;Mar;126(3):693-702.
17. O'Sullivan M, Clayton N, Breslin N, Harman I, Bountra C, McLaren A, et al. Increased mast cells in the irritable bowel syndrome. Neurogastroenterol Motil. 2000;Oct;12(5):449-57.
18. Feng B, La JH, Schwartz ES, Gebhart GF. Neural and neuro-immune mechanisms of visceral hypersensitivity in irritable bowel syndrome. American Journal of Physiology - Gastrointestinal and Liver Physiology. 2012 March 8, 2012.
19. Spiller R, Garsed K. Postinfectious irritable bowel syndrome. Gastroenterol. 2009;May;136(6):1979-88.
20. Videlock EJ, Adeyemo M, Licudine A, Hirano M, Ohning G, Mayer M, et al. Childhood trauma is associated with hypothalamic-pituitary-adrenal axis responsiveness in irritable bowel syndrome. Gastroenterol. 2009;Dec;137(6):1954-62.
21. Liebregts T, Adam B, Bredack C, Röth A, Heinzel S, Lester S, et al. Immune activation in patients with irritable bowel syndrome. Gastroenterol. 2007;Mar;132(3):913-20.
22. Talley NJ, Fodor AA. Bugs, stool, and the irritable bowel syndrome: too much is as bad as too little? Gastroenterol. 2011;Nov;141(5):1555-9.
23. Aerssens J, Camilleri M, Talloen W, Thielemans L, Göhlmann H, Wyngaert IVd, et al. Alterations in mucosal immunity identified in the Colon of patients with irritable bowel syndrome. Clin Gastroenterol Hepatol. 2008;Feb;6(2):194-205.
24. Harris C, Ma T, Leighton J, Tang L, Doherty P, Zhou F, et al. Novel Genomic Biomarkers That Differentiate Between Irritable Bowel Syndrome and Normal Patients Using Peripheral Blood Specimens. Am J Gastroenterol. 2008;103(1):S468.
25. Talley N, Spiller R. Irritable bowel syndrome: a little understood organic bowel disease? Lancet. 2002;360:555-64.
26. Walker E, Roy-Byrne P, Katon W. Irritable bowel syndrome and psychiatric illness. Am J Psychiatry. 1990;147:565-72.
27. Gros D, Antony M, McCabe R, Swinson R. Frequency and severity of the symptoms of irritable bowel syndrome across the anxiety disorders and depression. J Anxiety Dis. 2009;23:290-6.
28. Drossman D. Do psychosocial factors define symptom severity and patient status in irritable bowel syndrome? Am J Med. 1999;107(suppl):41-50.
29. Ditto B, Miller S, Barr R. A one-hour active coping stressor reduces small bowel transit time in healthy young adults. Psychosom Med. 1998;60:7-10.
30. Fossey M, Lydiard R. Anxiety and the gastrointestinal system. Psychiatr Med. 1990;8:175-86.
31. Drossman D. Gastrointestinal illness and the biopsychosocial model. Psychosom Med. 1998;60:258-67.
32. Lembo A, Neri B, Tolley J, Barken D, Carroll S, Pan H. Use of serum biomarkers in a diagnostic test for irritable bowel syndrome. Aliment Pharmacol Ther. 2009;Apr;29(8):834-42.
33. Whitehead W, Palsson O, Levy R, Feld A, Turner M, Korff MV. Comorbidity in Irritable Bowel Syndrome. Am J Gastroenterol. 2007;102:2767-76.
34. Francis CY, Morris J, Whorwell PJ. The irritable bowel severity scoring system: a simple method of monitoring irritable bowel syndrome and its progress. Alimentary Pharmacology & Therapeutics. 1997;Apr;11(2):395-402.
35. Drossman DA, Li Z, Toner BB, Diamant NE, Creed FH, Thompson D, et al. Functional bowel disorders: a multicenter comparison of health status and development of illness severity index. Digestive Diseases and Sciences. 1995;May;40(5):986-95.
36. Zigmond A, Snaith R. The hospital anxiety and depression scale. Acta Psychiatr Scand. 1983;67(6):361-70.
37. Kroenke K, Spitzer R, Williams J. The PHQ-15: validity of a new measure for evaluating the severity of somatic symptoms. Psychosom Med. 2002;64(2):258-66.
38. Benjamini Y, Hochberg Y. Controlling the false discovery rate: a practical and powerful approach to multiple testing. J Roy Stat Soc B. 1995;57(1):289-300.
39. Vivinus-Nebot M, Dainese R, Anty R, Saint-Paul M, Nano J, Gonthier N, et al. Combination of allergic factors can worsen diarrheic irritable bowel syndrome: role of barrier defects and mast cells. Am J Gastroenterol. 2012;Jan;107(1):75-81.
40. Walker M, Warwick A, Ung C, Talley N. The role of eosinophils and mast cells in intestinal functional disease. Curr Gastroenterol Rep. 2011;Aug;13(4):323-30.
41. Ford A, Talley N. Mucosal inflammation as a potential etiological factor in irritable bowel syndrome: a systematic review. J Gastroenterol. 2011;Apr;46(4):421-31.
42. Ford A, Talley N. IBS in 2010: advances in pathophysiology, diagnosis and treatment. Nat Rev Gastroenterol Hepatol. 2011;Feb;8(2):76-8.
43. Bossuyt P, Reitsma J, Bruns D, Gatsonis C, Glasziou P, Irwig L, et al. The STARD statement for reporting studies of diagnostic accuracy: explanation and elaboration. Ann Intern Med. 2003;Jan 7;138(1):W1-12.

## Claims

1. A method for aiding in the differentiation of IBS-constipation (IBS-C) from IBS-diarrhea (IBS-D) in a subject, the method comprising:
(a) contacting a first sample from the subject with a binding moiety under conditions suitable to transform an IBS serological marker present in the first sample into a complex comprising the IBS serological marker and the binding moiety,
wherein the IBS serological marker comprises a combination of histamine and neutrophil gelatinase-associated lipocalin (NGAL);
(b) contacting isolated and/or amplified RNA obtained from a second sample from the subject with a detection reagent under conditions suitable to transform an IBS genetic marker present in the second sample into a complex comprising the IBS genetic marker and the detection reagent,
wherein the IBS genetic marker comprises a combination of MICALL1 and RNF26;
(c) determining the level of the complex in step (a), thereby determining the level of the IBS serological marker present in the first sample; and
(d) determining the level of the complex in step (b), thereby determining the level of the IBS genetic marker present in the second sample.

2. The method of claim 1, wherein the IBS serological marker further comprises PGE2, tryptase, serotonin, substance P, IL-12, IL-10, IL-6, IL-8, TNF-α, IL-1β, GRO-α, BDNF, ASCA IgA, anti-CBir1 antibody, tTG, TWEAK, ANCA, TIMP-1, or combinations thereof.

3. The method of claim 2, wherein the level of at least 2, 3, 4, 5, 6, 7, 8, 9, 10, 11, 12, 13, 14, 15, 16, 17, or 18 of the IBS serological markers are determined.

4. The method of any one of claims 1 to 3, wherein the IBS genetic marker further comprises CBFA2T2, CCDC147, HSD17B11, LDLR, MAP6D1, RAB7L1, RRP7A, SUSD4, SH3BGRL3, VIPR1, WEE1, ZNF326, or combinations thereof.

5. The method of claim 4, wherein the level of at least 2, 3, 4, 5, 6, 7, 8, 9, 10, 11, or 12 of the IBS genetic markers are determined.

6. The method of any one of claims 1 to 5, wherein the IBS serological marker comprises a combination of histamine, NGAL, PGE2, tryptase, serotonin, substance P, IL-12, IL-10, IL-6, IL-8, TNF-α, IL-1β, GRO-α, BDNF, ASCA IgA, anti-CBir1 antibody, tTG, TWEAK, ANCA, and TIMP-1; and the IBS genetic marker comprises a combination of RNF26, CBFA2T2, CCDC147, HSD17B11, LDLR, MAP6D1, RAB7L1, RRP7A, SUSD4, SH3BGRL3, VIPR1, WEE1, MICALL1, and ZNF326.

7. The method of any one of claims 1 to 6, wherein the method further comprises determining a psychological measure of the subject.

8. The method of claim 7, wherein the psychological measure is selected from the group consisting of a Patient Health Questionnaire 15 (PHQ-15), a PHQ-15 wherein gastrointestinal symptoms have been excluded from consideration (PHQ-non GI), a perceived stress scale (PSS), a Hospital Anxiety and/or Depression scale (HADs), and combinations thereof.

9. The method of any one of claims 1 to 8, wherein the method further comprises applying an algorithm to the level of the IBS serological marker, the level of the IBS genetic marker, and/or the psychological measure of the subject.

10. The method of any one of claims 1 to 9, wherein the first sample and the second sample are independently selected from the group consisting of whole blood, serum, plasma, and stool.

11. The method of any one of claims 1 to 10, wherein the subject has previously been diagnosed with IBS.

## Patentansprüche

1. Verfahren zum Helfen bei der Unterscheidung zwischen IBS-Verstopfung (IBS-V) und IBS-Durchfall (IBS-D) bei einer Versuchsperson, wobei das Verfahren umfasst:
(a) In-Kontakt-Bringen einer ersten Probe der Versuchsperson mit einem bindenden Anteil unter Bedingungen, die zum Transformieren eines in der ersten Probe vorliegenden IBS-serologischen Markers in einen den IBS-serologischen Marker und den bindenden Anteil umfassenden Komplex geeignet sind,
wobei der IBS-serologische Marker eine Kombination von Histamin und Neutrophilen-Gelatinase-assoziiertem Lipocalin (NGAL) umfasst;
(b) In-Kontakt-Bringen aus einer zweiten Probe der Versuchsperson erhaltener isolierter und/oder amplifizierter RNA mit einem Nachweisreagenz unter Bedingungen, die zum Transformieren eines in der zweiten Probe vorliegenden IBSserologischen Markers in einen den IBS-serologischen Marker und das Nachweisreagenz umfassenden Komplex geeignet sind,
wobei der IBS-genetische Marker eine Kombination von MICALLI und RNF26 umfasst;
(c) Bestimmen des Niveaus des Komplexes in Schritt (a), dadurch Bestimmen des Niveaus des in der ersten Probe vorliegenden IBS-serologischen Markers und
(d) Bestimmen des Niveaus des Komplexes in Schritt (b), dadurch Bestimmen des Niveaus des in der zweiten Probe vorliegenden IBS-serologischen Markers.

2. Verfahren nach Anspruch 1, wobei der IBS-serologische Marker ferner PGE2, Tryptase, Serotonin, Substanz P, IL-12, IL-10, IL-6, IL-8, TNF-α, IL-1 β, GRO-α, BDNF, ASCA IgA, anti-CBirl-Antikörper, tTG, TWEAK, ANCA, TIMP-1 oder Kombinationen davon umfasst.

3. Verfahren nach Anspruch 2, wobei das Niveau von wenigstens 2, 3, 4, 5, 6, 7, 8, 9, 10, 11, 12, 13, 14, 15, 16, 17 oder 18 der IBS-serologischen Marker bestimmt wird.

4. Verfahren nach einem der Ansprüche 1 bis 3, wobei der IBS-genetische Marker ferner CBFA2T2, CCDC147, HSD17B11, LDLR, MAP6D1, RAB7L1, RRP7A, SUSD4, SH3BGRL3, VIPR1, WEE I, ZNF326 oder Kombinationen davon umfasst.

5. Verfahren nach Anspruch 4, wobei das Niveau von wenigstens 2, 3, 4, 5, 6, 7, 8, 9, 10, 11 oder 12 der IBS-genetischen Marker bestimmt wird.

6. Verfahren nach einem der Ansprüche 1 bis 5, wobei der IBS-serologische Marker eine Kombination aus Histamin, NGAL, PGE2, Tryptase, Serotonin, Substanz P, IL-12, IL-10, IL-6, IL-8, TNF-α, IL-1 β, GRO-α, BDNF, ASCA IgA, anti-CBirl-Antikörper, tTG, TWEAK, ANCA und TIMP-1 umfasst und der IBSgenetische Marker eine Kombination aus RNF26, CBFA2T2, CCDC147, HSD17B11, LDLR, MAP6D1, RAB7L1, RRP7A, SUSD4, SH3BGRL3, VIPR1, WEEI, MICALLI und ZNF326 umfasst.

7. Verfahren nach einem der Ansprüche 1 bis 6, wobei das Verfahren ferner das Bestimmen einer psychologischen Maßnahme der Versuchsperson umfasst.

8. Verfahren nach Anspruch 7, wobei die psychologische Maßnahme ausgewählt ist aus der Gruppe bestehend aus einem Gesundheitsfragebogen für Patienten 15 (PHQ-15), einem PHQ-15, worin gastrointestinale Symptome von der Betrachtung ausgeschlossen wurden (PHQ-kein Gl), einer Skala für wahrgenommenen Stress (PSS), einem Fragebogen zur Selbstbeurteilung von depressiven Symptomen und Angstsymptomen (Hospital Anxiety and/or Depression scale, HADs) und Kombinationen davon.

9. Verfahren nach einem der Ansprüche 1 bis 8, wobei das Verfahren ferner die Anwendung eines Algorithmus auf das Niveau des IBS-serologischen Markers, das Niveau des IBS-genetischen Markers und/oder der psychologischen Maßnahmen der Versuchsperson umfasst.

10. Verfahren nach einem der Ansprüche 1 bis 9, wobei die erste Probe und die zweite Probe unabhängig ausgewählt werden aus der Gruppe bestehend aus Vollblut, Serum, Plasma und Stuhl.

11. Verfahren nach einem der Ansprüche 1 bis 10, wobei bei der Versuchsperson zuvor IBS diagnostiziert wurde.

## Revendications

1. Procédé d'assistance à la différenciation d'une constipation SCI (SCI-C) par rapport à une diarrhée SCI (SCI-D) chez un sujet, le procédé comprenant :
(a) la mise en contact d'un premier échantillon du sujet avec une fraction de liaison dans des conditions appropriées pour transformer un marqueur sérologique du SCI présent dans le premier échantillon en un complexe comprenant le marqueur sérologique du SCI et la fraction de liaison,
dans lequel le marqueur sérologique du SCI comprend une combinaison d'histamine et de lipocaline associée à la gélatinase des neutrophiles (NGAL) ;
(b) la mise en contact d'un ARN isolé et/ou amplifié obtenu à partir d'un second échantillon du sujet avec un réactif de détection dans des conditions appropriées pour transformer un marqueur génétique du SCI présent dans le second échantillon en un complexe comprenant le marqueur génétique du SCI et le réactif de détection,
dans lequel le marqueur génétique du SCI comprend une combinaison de MICALLI et RNF26 ;
(c) la détermination du taux du complexe dans l'étape (a), afin de déterminer ainsi le taux du marqueur sérologique du SCI présent dans le premier échantillon ; et
(d) la détermination du taux du complexe dans l'étape (b), afin de déterminer ainsi le taux du marqueur génétique du SCI présent dans le second échantillon.

2. Procédé selon la revendication 1, dans lequel le marqueur sérologique du SCI comprend en outre PGE2, tryptase, sérotonine, substance P, IL-12, IL-10, IL-6, IL-8, TNF-α, IL-1β, GRO-α, BDNF, ASCA IgA, anticorps anti-CBirl, tTG, TWEAK, ANCA, TIMP-1 ou leurs combinaisons.

3. Procédé selon la revendication 2, dans lequel le taux d'au moins 2, 3, 4, 5, 6, 7, 8, 9, 10, 11, 12, 13, 14, 15, 16, 17 ou 18 des marqueurs sérologiques du SCI est déterminé.

4. Procédé selon l'une quelconque des revendications 1 à 3, dans lequel le marqueur génétique du SCI comprend en outre CBFA2T2, CCDC147, HSD17B11, LDLR, MAP6D1, RAB7L1, RRP7A, SUSD4, SH3BGRL3, VIPR1, WEE 1, ZNF326 ou leurs combinaisons.

5. Procédé selon la revendication 4, dans lequel le taux d'au moins 2, 3, 4, 5, 6, 7, 8, 9, 10, 11 ou 12 des marqueurs génétiques du SCI est déterminé.

6. Procédé selon l'une quelconque des revendications 1 à 5, dans lequel le marqueur sérologique du SCI comprend une combinaison d'histamine, NGAL, PGE2, tryptase, sérotonine, substance P, IL-12, IL-10, IL-6, IL-8, TNF-α, IL-1β, GRO-α, BDNF, ASCA IgA, anticorps anti-CBirl, tTG, TWEAK, ANCA et TIMP-1 ; et le marqueur génétique du SCI comprend une combinaison de RNF26, CBFA2T2, CCDC147, HSD17B11, LDLR, MAP6D1, RAB7L1, RRP7A, SUSD4, SH3BGRL3, VIPR1, WEE1, MICALL1 et ZNF326.

7. Procédé selon l'une quelconque des revendications 1 à 6, où le procédé comprend en outre la détermination d'une mesure psychologique du sujet.

8. Procédé selon la revendication 7, dans lequel la mesure psychologique est choisie dans le groupe constitué d'un questionnaire de santé du patient 15 (PHQ-15), un PHQ-15 dans lequel les symptômes gastro-intestinaux ont été exclus de la prise en charge (PHQ-non Gl), une échelle de stress ressenti (PSS), une échelle hospitalière de meure de l'anxiété et/ou de la dépression (HADs) et leurs combinaisons.

9. Procédé selon l'une quelconque des revendications 1 à 8, dans lequel le procédé comprend en outre l'application d'un algorithme au taux du marqueur sérologique du SCI, au taux du marqueur génétique du SCI et/ou à la mesure psychologique du sujet.

10. Procédé selon l'une quelconque des revendications 1 à 9, dans lequel le premier échantillon et le second échantillon sont indépendamment choisis dans le groupe constitué de sang total, sérum, plasma et selles.

11. Procédé selon l'une quelconque des revendications 1 à 10, dans lequel le sujet a précédemment été diagnostiqué comme souffrant du SCI.
